# EUROPEAN PATENT APPLICATION

(11) **EP 4 851 301 A1**
(43) Date of publication of application: **09.12.2026**
(21) Application number: 25748823.9
(22) Date of filing: 30.01.2025
(51) Int. Cl.: C12Q 1/04, A61K 35/545, A61L 27/38, A61P 27/02, C12N 5/079, C12Q 1/6851, G01N 33/48, G01N 33/483, G06T 7/00, G06T 7/12, G06T 7/62, G06V 10/44, G06V 20/69

(54) **METHOD FOR DETERMINING NORMAL RETINAL TISSUE REGION IN CELL AGGREGATE, AND METHOD FOR PRODUCING RETINAL TISSUE IMPLANTABLE IN ANIMALS**

(30) Priority: 31.01.2024 JP 2024012802
(71) Applicant: RACTHERA Co., Ltd., Tokyo 103-6012 (JP)
(72) Inventor: UEYAMA, Kazuki, Suita-shi, Osaka 564-0053 (JP); KUWAHARA, Atsushi, Kobe-shi, Hyogo 650-0047 (JP); WATARI, Kenji, Kobe-shi, Hyogo 650-0047 (JP); DAIBA, Akito, Tokyo 103-6012 (JP)
(74) Representative: Zimmermann & Partner Patentanwälte mbB
(86) International application number: PCT/JP2025/003041
(87) International publication number: WO 2025/164733

(57) **Abstract**

Provided is a method for determining the quality of a cell aggregate by obtaining an evaluation score on an image of a cell aggregate according to a determination criterion from an appropriate viewpoint, and performing quick, objective, and appropriate quality evaluation. In an image region of a cell aggregate of each of the inverted observation image and the upright observation image of the cell aggregate that may contain a retinal tissue derived from pluripotent stem cells, a region determined to satisfy a candidate region determination criterion regarding color corresponding to each of the inverted observation image and the upright observation image is identified as a candidate region included in a region to be a candidate of normal retinal tissue region based on the candidate region determination criterion regarding color corresponding to each of the inverted observation image and the upright observation image, and in each of the candidate regions of the inverted observation image and the upright observation image, a candidate region is determined to satisfy a normal region determination criterion regarding brightness and/or morphology corresponding to each of the inverted observation image and the upright observation image as a normal retinal tissue region.

## Description

### Technical Field

The present disclosure relates to a method for determining, in a cell aggregate, a normal retinal tissue region that is transplantable into an animal, and to a method for producing retinal tissue that is transplantable into an animal. The present disclosure also relates to a method for determining a tissue region other than retinal tissue of animals.

### Background Art

Various studies have been conducted for the purpose of treatment for transplanting neural retinal tissue in humans. Through the studies, a method for producing a cell aggregate including neural retinal tissue (three-dimensional tissue) has also been improved, and it has become possible to produce high-quality neural retinal tissue with high efficiency. On the other hand, the neural retinal tissue is a very complicated tissue, and due to the property of artificially differentiating cells in vitro, off-target cells may be partially induced. Thus, removing cell aggregates containing off-target cells or cutting out a normal region of cell aggregates not containing off-target cells for transplantation is a very important step in terms of quality control.

In recent years, methods for evaluating the quality of neural retina for transplantation have been developed. For example, as a method for evaluating the quality of neural retina for transplantation, there is a method of extracting a part or all of a cell aggregate including a neural retina having an epithelial structure derived from pluripotent stem cells as a sample for quality evaluation, detecting expression of a neural retina-related cell-associated gene and a non-neural retina-related cell-associated gene in the sample for quality evaluation, and determining that a neural retina (neural retina for transplantation) in the same cell aggregate as the cell aggregate containing the sample for quality evaluation, or in a cell aggregate of the same lot as that cell aggregate, can be used as a neural retina for transplantation when expression of the neural retina-related cell-associated gene is observed and expression of the non-neural retina-related cell-associated gene including one or more genes selected from the group consisting of a cerebrospinal tissue marker gene and an eyeball-associated tissue marker gene is not observed (Patent Document 1). According to this method, the quality of the neural retina for transplantation can be evaluated by measuring the gene expression level. However, this method requires the use of a PCR apparatus, and is not suitable for determining the quality of a large number of cell aggregates. Thus, there has been a demand for a method capable of appropriately determining the quality of a large number of cell aggregates using a microscope or the like.

### Citation List

### Patent Document

Patent Document 1: WO 2020/184720

### Summary of Invention

### Technical Problem

In general, the shape of a three-dimensional tissue (organoid) contained in a cell aggregate is irregular. Thus, an appropriate method is necessary when the observation is performed with a microscope. Usually, a plurality of small epithelial tissues are present in one organoid tissue. In each organoid tissue, there are several numbers of epithelial tissues, and the size of each epithelial tissue and the angle at which the epithelial tissue is attached are completely different for each organoid tissue.

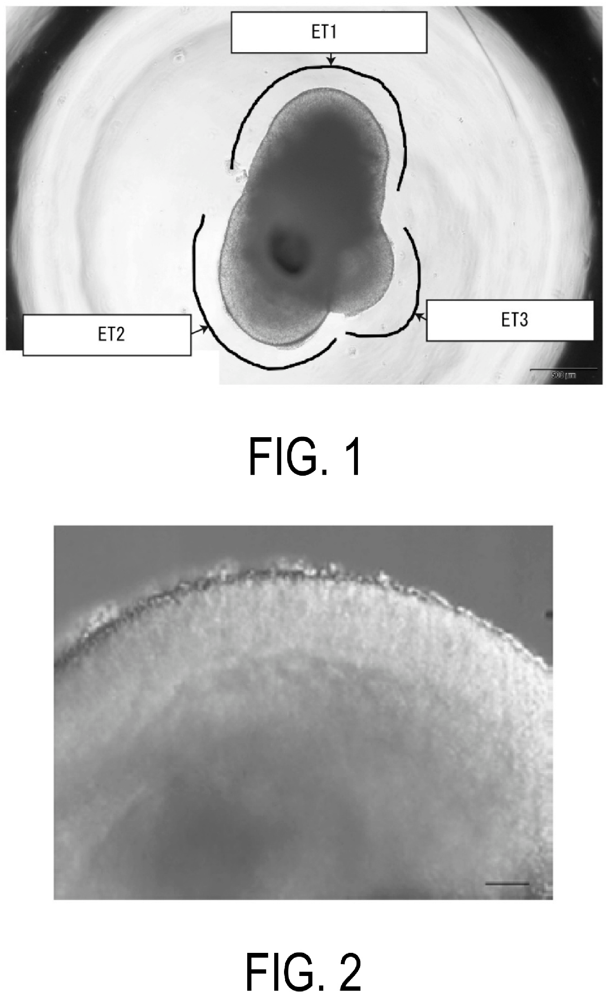

The organoid tissue contained in a cell aggregate has a different shape for each individual. Normally, neurons including neural retina form a neuroepithelial structure in which cells are tightly joined to each other, aligned, and layered. The neuroepithelium is composed of closely related cells. This continuous mass of neuroepithelium is referred to as "epithelial tissue" or "lobe". A cell aggregate often has a plurality of such lobes. FIG. 1 is an image of a cell aggregate containing three lobes, taken by upright observation. For example, in the cell aggregate shown in FIG. 1, there are three epithelial tissues, that is, three lobes, but their sizes differ from one another. That is, in FIG. 1, three lobes having different sizes are shown as epithelial tissue ET1, epithelial tissue ET2, and epithelial tissue ET3. Gravity is applied to the cell aggregate, and thus, the cell aggregate as a whole has a substantially elliptical shape when viewed from a vertical direction, and has a convex lens shape with a thick central portion when viewed from a horizontal direction, and each lobe basically expands in the horizontal direction. Each lobe has a different thickness, and thus, a focused position of the microscope in each lobe is also different.

However, epithelial tissues have undesirable properties for microscopic observation. First, when a three-dimensional retinal organoid is created from iPS cells, off-target cells or off-target tissues may also be produced in addition to retinal tissue. In the case of commercial production, it is usual to simultaneously produce, for example, about 30 to 30000 organoid tissues in one differentiation culture. Then, the size and state of the epithelial tissue are spatially and three-dimensionally different for each of the produced organoid tissues. Thus, even when iPS cells are differentiated under the same differentiation conditions to produce a three-dimensional retinal organoid, a slight difference in quality occurs for each differentiation lot (batch).

As a method for observing a three-dimensional retinal organoid or a method for evaluating the quality of a three-dimensional retinal organoid, an examination using an optical microscope suitable for mass observation is often performed. Here, as a method of microscopic examination, there are an upright observation using an upright microscope and an inverted observation using an inverted microscope. The observation of three-dimensional retinal organoid is often performed only through inverted observation, but when the observation is performed through either upright observation or inverted observation, there may be an off-target cell that cannot be detected because it is present on the back side of the three-dimensional retinal organoid. In this case, there is a possibility that the off-target cell is overlooked, and erroneous quality evaluation is performed. As described above, quality evaluation using only either an upright observation image acquired by upright observation using an upright microscope or an inverted observation image acquired by inverted observation using an inverted microscope involves a problem.

In addition, the quality evaluation is based on observation using an optical microscope, and the cell aggregate to be observed has a three-dimensional shape. Thus, when a certain portion is focused, other portions are not focused. In addition, when there is an image such as a subtle shadow, whether the image represents an off-target cell or a shadow caused by illumination conditions cannot be clearly determined in some cases.

To reliably observe cell aggregates, measures against the undesirable properties as described above are required, but there are technical limitations to the measures. Here, for example, based on common sense, measures of the following methods can be conceived, but it is extremely difficult to actually perform any of them because of technical limitations.

First, a method of taking out cell aggregates from a medium and observing and evaluating the cell aggregates in air is conceived. According to this method, the observation is performed in the air, and thus, it is considered that observation can be performed from a wider variety of directions. However, when the cell aggregates are taken out from a medium, the cell aggregates are dried and damaged, which adversely affects the quality, and thus such a method cannot be adopted.

A method of performing analysis by moving cell aggregates in a culture solution under a microscope and spatially observing the cell aggregates is also conceived. However, cell aggregates are soft and susceptible to physical damage, and thus they may be damaged when moved. In addition, a very long time is required for the operation for observation, and therefore this method is also not realistic.

Further, to observe cell aggregates from a more flexible direction, a method of observing and evaluating cell aggregates by rotating the cell aggregates together with the vessel is conceived. However, in practice, even when the entire vessel is rotated, proper observation cannot be performed. This is because the culture solution leaks when the vessel is rotated because the normal culture plate is not sealed. In addition, a multi-well plate allows observation from above and below, but does not allow observation from a lateral side because other wells obstruct the observation.

Further, to observe cell aggregates from the back side, it is conceivable to rotate the cell aggregates. However, the cell aggregates have elasticity, and there is a deviation of the center of gravity due to individual differences. Thus, it is difficult to physically rotate the cell aggregates even when the vessel is rotated. Here, it is conceivable to grip and rotate the cell aggregates using tweezers or the like, but this is not realistic because there is a possibility of damaging cells and tissues. In particular, the surface of the three-dimensional retinal organoid is easily damaged, and thus, it is substantially impossible to rotate the three-dimensional retinal organoid using tweezers or the like.

As described above, according to the methods commonly conceived as described above, it is not possible to sufficiently observe organoid tissue with a microscope or to efficiently observe the quality of a large number of organoid tissues, and thus it has been difficult to achieve automation and increased speed. In fact, no research institution has yet established a method for performing quality assessment of neural retinal tissue at a clinical level. Further, there is no technology for evaluating a large amount of neural retinal tissue using both an upright microscope and an inverted microscope.

Further, there is no known technology for performing quality evaluation by determining images of cell aggregates photographed with an upright microscope and an inverted microscope through information processing. In such a case, there is no method of objectively determining the quality by obtaining an evaluation score based on a determination criterion from an appropriate viewpoint from the image. There is also no method of setting a determination criterion through machine learning and objectively determining the quality by integrating various features on the image.

The present invention has been made in view of the above-described issues, and provides a method for determining the quality of a cell aggregate by obtaining an evaluation score on an image of a cell aggregate photographed with an upright microscope and an inverted microscope according to a determination criterion from an appropriate viewpoint, and performing quick, objective, and appropriate quality evaluation.

### Solution to Problem

The present invention has been made in view of the above-described issues, and solves the issues by adopting the following characteristic configurations. That is, the present invention is a method for determining a normal retinal tissue region that can be transplanted into an animal in a cell aggregate such as continuous epithelial tissue that may contain retinal tissue derived from pluripotent stem cells, the method including:
an image data acquisition stage of acquiring image data of an inverted observation image and an upright observation image of the cell aggregate by photographing the cell aggregate using an inverted microscope and an upright microscope;
a candidate region identification stage of identifying, in the image region of the cell aggregate of each of the inverted observation image and the upright observation image, a region determined to satisfy a candidate region determination criterion regarding color corresponding to each of the inverted observation image and the upright observation image as a candidate region included in a region to be a candidate of the normal retinal tissue region based on the candidate region determination criterion regarding color corresponding to each of the inverted observation image and the upright observation image; and
a normal region determination stage of determining, in the candidate region of each of the inverted observation image and the upright observation image, a candidate region determined to satisfy a normal region determination criterion regarding brightness and/or morphology corresponding to each of the inverted observation image and the upright observation image as the normal retinal tissue region based on the normal region determination criterion regarding brightness and/or morphology corresponding to each of the inverted observation image and the upright observation image.

In the present invention, the inverted observation image and the upright observation image may be position-associated in a manner that positions in respective images of the inverted observation image and the upright observation image correspond to an identical position of the cell aggregate viewed from a vertical direction, and
in the normal region determination stage, in the candidate region of each of the inverted observation image and the upright observation image, the candidate region determined to simultaneously satisfy a normal region determination criterion regarding brightness and morphology corresponding to each of the inverted observation image and the upright observation image may be determined as the normal retinal tissue region based on the normal region determination criterion regarding brightness and morphology corresponding to each of the inverted observation image and the upright observation image.

In the present invention, the normal region determination stage may include:
a region portion evaluation stage of dividing the candidate region into a plurality of ranges in which region portions are present, along a curve representing a direction in which the candidate region extends, and obtaining an evaluation score of each of the region portions, for the candidate region identified in the image region of the cell aggregate with which the position of each of the inverted observation image and the upright observation image is associated, based on an evaluation criterion regarding color and/or morphology corresponding to each of the inverted observation image and the upright observation image; and
a continuous length-based normal region determination stage of determining the region portion as the normal retinal tissue region when it is determined that the region portion of the candidate region in which both the evaluation scores of the inverted observation image and the upright observation image have a value equal to or more than a corresponding normal region determination reference value is continuously present along a curve representing an extending direction of the candidate region by a length equal to or more than a predetermined normal region determination reference length.

In the present invention, the candidate region determination criterion in the candidate region identification stage may further include a requirement that brightness of the region to be identified is higher than a maximum value or an average value of brightness of a central region of an image region of the cell aggregate by a predetermined degree or more.

In the present invention, the candidate region determination criterion in the candidate region identification stage may further include a requirement that, in the region to be identified, an outermost contour viewed from a central portion of an image region of the cell aggregate in a region portion included in the region to be identified substantially matches a contour of the image region of the cell aggregate.

In the present invention, the candidate region determination criterion in the candidate region identification stage may further include a requirement that a change in an angle in a tangential direction of the outermost contour of the region to be identified is continuous.

In the present invention, the candidate region determination criterion in the candidate region identification stage may further include a requirement that a width between an outermost portion and an innermost portion of the region to be identified as viewed from a central portion of an image region of the cell aggregate is equal to or more than a predetermined reference width.

In the present invention, the candidate region identification stage may further include an off-target cell identification stage of identifying whether an off-target cell is present based on a predetermined off-target cell determination criterion based on color and morphology in the image region of the cell aggregate of each of the inverted observation image and the upright observation image, and the candidate region determination criterion further includes a requirement that the off-target cell is not present in the region to be identified.

In the present invention, the region portion evaluation stage may divide the candidate region identified in the image region of the cell aggregate to which each position of the inverted observation image and the upright observation image is associated into a plurality of ranges in which region portions are present at every predetermined angle as viewed from a central portion of the image region of the cell aggregate, and obtain an evaluation score of each of the region portions based on an evaluation criterion regarding morphology corresponding to each of the inverted observation image and the upright observation image with respect to the candidate region, and the continuous length-based normal region determination stage may determine that the cell aggregate in which the region portion in which both the evaluation scores of the inverted observation image and the upright observation image are determined to have values equal to or more than the corresponding normal region determination reference value is continuously present at a predetermined normal region determination reference angle or more has the normal retinal tissue region.

In the present invention, the predetermined reference width in the candidate region identification stage may be 0.03 mm for the inverted observation image and 0.15 mm for the upright observation image.

In the present invention, the predetermined normal region determination reference length in the continuous length-based normal region determination stage may be 0.4 mm.

In the present invention, the predetermined normal region determination reference angle in the continuous length-based normal region determination stage may be 45 degrees.

In the present invention, the image data of the inverted observation image and the upright observation image of the cell aggregate that may include the retinal tissue may be obtained by photographing the cell aggregate suspended in a liquid.

In the present invention, the image data of the inverted observation image and the upright observation image of the cell aggregate that may include the retinal tissue may be obtained by photographing the cell aggregate in a plastic vessel.

In the present invention, the plastic vessel may be a vessel having one or more isolated culture regions.

In the present invention, the plastic vessel may be a spherical or conical vessel having a downward convex shape or a recess.

The present invention may be a method for producing a retinal tissue that can be transplanted into an animal, the method including cutting out the normal retinal tissue region determined by any one of the methods described above.

The present invention may be a method for determining a normal retinal tissue region that can be transplanted into an animal, the method including, with respect to the normal retinal tissue region determined by any one of the methods described above, further:
detecting expression of a neural retina-related cell-associated gene and a non-neural retina-related cell-associated gene in the normal retinal tissue region; and
determining that the normal retinal tissue region can be transplanted into an animal when expression of the neural retina-related cell-associated gene is observed and expression of the non-neural retina-related cell-associated gene is not observed.

The present invention is a method for producing a retinal tissue that can be transplanted into an animal by executing, with respect to the normal retinal tissue region which is a region portion of a candidate region determined as satisfying the normal region determination criterion by any one of the methods described above, further:
extracting a part or all of the normal retinal tissue region as a sample for quality evaluation;
detecting expression of a neural retina-related cell-associated gene and a non-neural retina-related cell-associated gene in the sample for quality evaluation;
when expression of the neural retina-related cell-associated gene is observed and expression of the non-neural retina-related cell-associated gene is not observed, determining that the following (1), (2), or (3) is usable as a neural retina for transplantation:
   (1) the neural retina (neural retina for transplantation) in the same cell aggregate as the cell aggregate containing the sample for quality evaluation in a case where the sample is a part of the normal retinal tissue region,
   (2) the neural retina (neural retina for transplantation) in a cell aggregate of the same lot as that of the cell aggregate containing the sample for quality evaluation in a case where the sample is a part of the normal retinal tissue region,
   (3) the neural retina (neural retina for transplantation) in a cell aggregate of the same lot as that of the cell aggregate of the sample for quality evaluation in a case where the sample is all of the normal retinal tissue region; and
cutting out the normal retinal tissue region determined to be usable as the neural retina for transplantation.

### Advantageous Effects of Invention

In the present invention, identification is performed on a region determined to satisfy a candidate region determination criterion corresponding to each of an inverted observation image and an upright observation image of a cell aggregate that may contain a retinal tissue derived from pluripotent stem cells regarding color as a candidate region included in a region to be a candidate of the normal retinal tissue region based on the candidate region determination criterion corresponding to each of the inverted observation image and the upright observation image in the image region of the cell aggregate of each of the inverted observation image and the upright observation image, and
determination is performed on, as an aggregate having the normal retinal tissue region, the cell aggregate having the candidate region of each of the inverted observation image and the upright observation image, the candidate region determined to satisfy the normal region determination criterion corresponding to each of the inverted observation image and the upright observation image based on a normal region determination criterion regarding brightness and/or morphology corresponding to each of the inverted observation image and the upright observation image, whereby an organoid in a culture solution can be analyzed with three-dimensionally and spatially high precision using an image data obtained by observing the cell aggregate from upper and lower two directions using an upright microscope and an inverted microscope, and further, by obtaining an evaluation score of a good product from an appropriate view point, detecting a region that satisfies the evaluation score in the case of observing from upper and lower two directions, and make determination by integrating these, an effect of automatically extracting a cell aggregate containing a three-dimensionally favorable normal region can be obtained.

### Brief Description of Drawings

FIG. 1 is an image of a cell aggregate containing retinal tissue observed through upright observation.
FIG. 2 is an image obtained by photographing a region of one lobe of a cell aggregate through inverted observation.
FIG. 3 is an image diagram of inverted observation and upright observation.
FIG. 4 includes an upright observation image and an inverted observation image of a cell aggregate of sample SPL1.
FIG. 5 includes an upright observation image and an inverted observation image of a cell aggregate of sample SPL2.
FIG. 6 includes an upright observation image and an inverted observation image of a cell aggregate of sample SPL3.
FIG. 7 includes an upright observation image and an inverted observation image of a cell aggregate of sample SPL4.
FIG. 8 includes an upright observation image and an inverted observation image of a cell aggregate of sample SPL5.
FIG. 9 includes an upright observation image and an inverted observation image of a cell aggregate of sample SPL6.
FIG. 10 includes an upright observation image and an inverted observation image of a cell aggregate of sample SPL7.
FIG. 11 includes an upright observation image and an inverted observation image of a cell aggregate of sample SPL8 held in a 96-well plate (U bottom).
FIG. 12 includes an upright observation image and an inverted observation image of a cell aggregate of sample SPL8 held in a 96-well plate (V bottom).
FIG. 13 includes an upright observation image and an inverted observation image of a cell aggregate of sample SPL8 held in a 24-well plate.
FIG. 14 includes an upright observation image and an inverted observation image of a cell aggregate of sample SPL8 held in a 6-well plate.
FIG. 15 includes an upright observation image and an inverted observation image of a cell aggregate of sample SPL8 held in a 60 mm dish.
FIG. 16 includes an upright observation image and an inverted observation image of a cell aggregate of sample SPL8 held in a T25 flask.
FIG. 17 includes an upright observation image and an inverted observation image of a retinal tissue.
FIG. 18 includes an upright observation image and an inverted observation image of a retinal pigment epithelium (RPE).
FIG. 19 includes an upright observation image and an inverted observation image of a ciliary body.
FIG. 20 includes an upright observation image and an inverted observation image of an optic stalk.
FIG. 21 includes an upright observation image and an inverted observation image of a telencephalic tissue.
FIG. 22 includes an upright observation image and an inverted observation image of a spinal cord tissue.
FIG. 23 includes an upright observation image and an inverted observation image of a non-neural retinal tissue of sample SPL15.
FIG. 24 includes an upright observation image and an inverted observation image of a non-neural retinal tissue of sample SPL16.
FIG. 25 includes an upright observation image and an inverted observation image of a non-neural retinal tissue of sample SPL17.
FIG. 26 is an upright observation image of a cell aggregate of sample SPL18 at a magnification of 12 times.
FIG. 27 is an upright observation image of the cell aggregate of sample SPL18 at a magnification of 20 times.
FIG. 28 is an upright observation image of the cell aggregate of sample SPL18 at a magnification of 32 times.
FIG. 29 is an upright observation image of the cell aggregate of sample SPL18 at a magnification of 50 times.
FIG. 30 is an upright observation image of the cell aggregate of sample SPL18 at a magnification of 80 times.
FIG. 31 is an upright observation image of the cell aggregate of sample SPL18 at a magnification of 100 times.
FIG. 32 is an inverted observation image of the cell aggregate of sample SPL18 with a phase contrast at a magnification of 4 times.
FIG. 33 is an inverted observation image of the cell aggregate of sample SPL18 with a phase contrast at a magnification of 10 times.
FIG. 34 is an inverted observation image of the cell aggregate of sample SPL18 with Hoffman modulation contrast at a magnification of 10 times.
FIG. 35 is an upright observation image of the cell aggregate of sample SPL19 at a magnification of 25 times.
FIG. 36 is an upright observation image of the cell aggregate of sample SPL19 at a magnification of 50 times.
FIG. 37 is an upright observation image of the cell aggregate of sample SPL19 at a magnification of 80 times.
FIG. 38 is an inverted observation image of the cell aggregate of sample SPL19 with a phase contrast at a magnification of 4 times.
FIG. 39 is an inverted observation image of the cell aggregate of sample SPL19 with a phase contrast at a magnification of 10 times.
FIG. 40 is an inverted observation image of the cell aggregate of sample SPL19 with Hoffman modulation contrast at a magnification of 10 times.
FIG. 41 is a schematic configuration diagram illustrating a configuration of a normal cell aggregate determination system 100.
FIG. 42 is a block diagram of a normal cell aggregate determination server 110.
FIG. 43 is an overall operation flowchart of Example 1.
FIG. 44 is a detailed operation flowchart relating to normal region determination of Example 1.
FIG. 45 is an overall operation flowchart of Example 2.
FIG. 46 is an operation flowchart of a preparation procedure of Example 2.
FIG. 47 is a detailed operation flowchart relating to normal region determination of Example 2.
FIG. 48 is an overall operation flowchart of Example 3.
FIG. 49 is an overall operation flowchart of Example 4.
FIG. 50 is a detailed operation flowchart relating to normal region determination of Example 4.
FIG. 51 includes inverted observation images (before and after marking) of a cell aggregate to be determined in Example 1.
FIG. 52 includes upright observation images (before marking and after learning marking) of a cell aggregate for learning in Example 2.
FIG. 53 includes upright observation images (before and after marking) of a cell aggregate to be determined in Example 2.
FIG. 54 includes an inverted observation image and an upright observation image of a cell aggregate to be determined in Example 4 after marking.
FIG. 55 is a conceptual diagram for measuring the width of a candidate region for a cell aggregate.
FIG. 56 is a diagram illustrating a width, an evaluation score, and a determination result of a region portion of a candidate region of the inverted observation image of Example 1.
FIG. 57 is a diagram illustrating a width, an evaluation score, and a determination result of a region portion of a candidate region of the upright observation image of Example 2.
FIG. 58 is a diagram illustrating a width, an evaluation score, and a determination result of a region portion of candidate regions of the upright observation image and the inverted observation image of Example 3.
FIG. 59 is a diagram illustrating widths, evaluation scores, and determination results of region portions of candidate regions of the upright observation image and the inverted observation image of Example 4.

### Description of Embodiments

### Description of Terms

The term "stem cell" means an undifferentiated cell having differentiation potential and proliferation capacity (in particular, self-renewal competence). The stem cells include sub-populations such as pluripotent stem cells, multipotent stem cells, and unipotent stem cells, depending on the differentiation potential. The pluripotent stem cell refers to a stem cell that can be cultured in vitro and has an ability to differentiate into all cell lineages belonging to three germ layers (ectoderm, mesoderm, endoderm) and/or extraembryonic tissue (pluripotency). The multipotent stem cell means a stem cell having an ability to differentiate into a plurality of types of tissues or cells but not all types of tissues or cells. The unipotent stem cell means a stem cell having an ability to differentiate into a specific tissue or cell.

The "pluripotent stem cell" can be induced from a fertilized egg, a cloned embryo, a germline stem cell, a tissue stem cell, a somatic cell, or the like. Examples of the pluripotent stem cell include an embryonic stem cell (ES cell), an embryonic germ cell (EG cell), and an induced pluripotent stem cell (iPS cell). a Muse cell (multi-lineage differentiating stress enduring cell) obtained from a mesenchymal stem cell (MSC) and a GS cell produced from a germ cell (for example, testis) are also included in pluripotent stem cells.

The "retinal tissue" means a tissue in which one or more types of retina-related cells constituting each retinal layer in a living retina are present according to a certain order, and the "neural retina" means a retinal tissue including an inner neural retina layer not including a retinal pigment epithelium layer among retinal layers described later.

In the present specification, a region of the retinal tissue that does not contain off-target cells and has a certain size suitable for transplantation is referred to as a "normal retinal tissue region".

The "retina-related cell" means a cell constituting each retinal layer or a precursor cell thereof in the living retina. Examples of the retina-related cell include, but are not limited to, photoreceptor cells (rod photoreceptor cells, cone photoreceptor cells), horizontal cells, amacrine cells, interneurons, retinal ganglion cells (ganglion cells), bipolar cells (rod bipolar cells, cone bipolar cells), Müller glial cells, retinal pigment epithelium (RPE) cells, ciliary bodies, precursor cells thereof (e.g., photoreceptor precursors, bipolar precursors), and retinal precursor cells. Among retina-related cells, specific examples of cells constituting the neural retina layer (also referred to as neural retina cells or neural retina-related cells) include photoreceptor cells (rod photoreceptor cells, cone photoreceptor cells), horizontal cells, amacrine cells, interneurons, retinal ganglion cells (ganglion cells), bipolar cells (rod bipolar cells, cone bipolar cells), Müller glial cells, and precursor cells thereof (e.g., photoreceptor precursors, bipolar precursors). That is, the neural retina-related cells do not include RPE cells or ciliary body cells.

The term "photoreceptor precursor" refers to a precursor cell that is determined to differentiate into a photoreceptor cell.

The "retinal precursor cell" refers to a precursor cell that can differentiate into any immature retina-related cells such as photoreceptor precursors, horizontal precursors, bipolar precursors, amacrine precursors, retinal ganglion precursors, Müller glial cells, and RPE precursors, and that can finally differentiate into any mature retina-related cells such as photoreceptor cells, rod photoreceptor cells, cone photoreceptor cells, horizontal cells, bipolar cells, amacrine cells, retinal ganglion cells, and RPE cells.

The "neural retinal precursor cell" refers to a precursor cell that can differentiate into any immature neural retina-related cells such as photoreceptor precursors, horizontal precursors, bipolar precursors, amacrine precursors, retinal ganglion precursors, and Müller glial cells, and that can ultimately differentiate into any mature neural retina-related cells such as photoreceptor cells, rod photoreceptor cells, cone photoreceptor cells, horizontal cells, bipolar cells, amacrine cells, and retinal ganglion cells. In the present specification, the term "retinal precursor cell" means a cell of a higher concept including "neural retinal precursor cell" unless otherwise specified.

The "photoreceptor cell" is present in the photoreceptor cell layer of the retina in a living body, and has a role of absorbing a light stimulus and converting the light stimulus into an electric signal. There are two types of photoreceptor cells, cones that function in bright light and rods that function in dark light (referred to as cone photoreceptor cell and rod photoreceptor cell, respectively). Examples of the cone photoreceptor cell include an S cone photoreceptor cell that expresses S-opsin and receives blue light, an L cone photoreceptor cell that expresses L-opsin and receives red light, and an M cone photoreceptor cell that expresses M-opsin and receives green light. Photoreceptor cells differentiate and mature from photoreceptor precursors.

The "retinal layer" means each layer constituting the retina, and specific examples thereof include a retinal pigment epithelium layer, a photoreceptor cell layer, an external limiting membrane, an outer nuclear layer, an outer plexiform layer, an inner nuclear layer, an inner plexiform layer, a ganglion cell layer, a nerve fiber layer, and an internal limiting membrane.

The "neural retina layer" means each layer constituting the neural retina, and specific examples thereof include a photoreceptor cell layer, an external limiting membrane, an outer nuclear layer, an outer plexiform layer, an inner nuclear layer, an inner plexiform layer, a ganglion cell layer, a nerve fiber layer, and an internal limiting membrane. The "photoreceptor cell layer" means a retinal layer formed on the outermost side of the neural retina and containing many photoreceptor cells (rod photoreceptor cells, cone photoreceptor cells), photoreceptor precursors, and retinal precursor cells. In view of the continuity of the formation of the layer structure, the photoreceptor cell layer may include a retinal precursor cell layer in addition to the photoreceptor cell layer. Each layer other than the photoreceptor cell layer is referred to as an inner layer. Which retinal layer is formed from each cell can be confirmed by a known method, for example, the presence or absence of expression of a cell marker, the degree of expression, or the like.

The outer neuroblastic layer (ONbL) is a layer including a photoreceptor precursor layer and a neural retinal precursor cell layer. Most of the photoreceptor precursor cells contained in the photoreceptor precursor layer differentiate into photoreceptor cells (rod photoreceptor cells, cone photoreceptor cells) to constitute a part of the photoreceptor cell layer. Photoreceptor precursors remain as a layer of spindle-shaped nuclei arranged in a dense manner, and differentiate into cone cells and rod cells. The neural retinal precursor cell layer differentiates into neural retina cells and constitutes a part of the neural retina layer. Photoreceptor precursors remain as a layer of spindle-shaped nuclei arranged in a dense manner, and differentiate into cone cells and rod cells.

The inner neuroblastic layer (INbL) is a layer including a retinal neuron layer. This corresponds to the retinal neurons of the inner layer.

The "ciliary body" includes a "ciliary body" and a "ciliary marginal zone (CMZ)" in the development stage and in an adult. Examples of the "ciliary marginal zone" include a region which is a tissue present in a boundary region between the neural retina and the RPE in the living retina and contains tissue stem cells of the retina (retinal stem cells). The ciliary marginal zone is also called ciliary margin or retinal margin, and the ciliary marginal zone, ciliary margin, and retinal margin are equivalent tissues. It is known that the ciliary marginal zone plays an important role in supplying retinal precursor cells and differentiated cells to retinal tissues, maintaining retinal tissue structure, and the like. The "ciliary marginal zone-like structure" is a structure similar to the ciliary marginal zone. The ciliary body is an off-target cell in relation to the retinal tissue.

The "cell aggregate" is a three-dimensional structure formed through adhesion of a plurality of cells. The structure is not particularly limited, and examples thereof include a mass formed through aggregation of cells dispersed in a medium such as a culture medium, a mass of cells formed through cell division, and the like. The cell aggregate includes a case where a specific tissue is formed. In the present specification, a cell aggregate forming a specific tissue may be referred to as a "three-dimensional tissue", and an organoid tissue in the case of forming a retinal tissue may be referred to as a "three-dimensional retinal organoid".

The term "epithelial tissue" (epithelium) refers to a tissue formed by covering the surface of a body surface, a lumen (such as the digestive tract), a body cavity (such as the pericardial cavity), or the like with cells without gaps. Cells forming epithelial tissue are referred to as epithelial cells. Epithelial cells have an apical-to-basal polarity. Epithelial cells can form a layer of cells by forming a strong joint between epithelial cells through an adherence junction and/or a tight junction. A tissue formed by one to more than ten layers of the cell layers is epithelial tissue. Tissues that can form epithelial tissue also include fetal and/or adult retinal tissue, cerebrospinal tissue, ocular tissue, neural tissue, and the like. The neural retina in the present specification is also epithelial tissue. The "epithelial structure" means a structure that epithelial tissue characteristically has, such as an apical surface or a basement membrane. "Epithelial tissue" may also be referred to as "lobe".

The term "continuous epithelial tissue" refers to a tissue having a continuous epithelial structure. The continuous epithelial structure is a structure in which epithelial tissue is continuous. The state in which epithelial tissue is continuous is, for example, a state in which from 10 cells to 10⁷ cells, preferably from 30 cells to 10⁷ cells, more preferably from 10² cells to 10⁷ cells are arranged in a tangential direction with respect to the epithelial tissue.

For example, in the continuous epithelial structure formed in the retinal tissue, the retinal tissue has an apical surface unique to the epithelial tissue, and the apical surface is formed substantially parallel to and continuously with at least the photoreceptor cell layer (outer nuclear layer) and the like among the layers forming the neural retina layer on the surface of the retinal tissue. For example, in the case of a cell aggregate containing a retinal tissue produced from pluripotent stem cells, an apical surface is formed on the surface of the aggregate, and 10 or more, preferably 30 or more, more preferably 100 or more, still more preferably 400 or more photoreceptor cells or photoreceptor precursors are regularly and continuously arrayed in a tangential direction with respect to the surface.

The term "pseudostratified epithelium" refers to one of epithelial tissues composed of superficial epithelial cells, intercalated epithelial cells, and basal epithelial cells, and is an epithelial tissue that looks like a stratified epithelium in which two or more cells are arranged in the apical to basal direction, but in which the bottom of all cells is in contact with the basement membrane, and is actually a monolayer.

The term "optic stalk" refers to a tissue that forms at the base of the optic cup, connecting the eye and the forebrain, and later becomes the optic nerve. The optic stalk is an off-target cell in relation to the retinal tissue.

The term "retinal pigment epithelium" (RPE) refers to a sheet-like monolayer cell layer located outside the retina, and is a cell that plays a role of supporting the photoreceptor cells of the retina. The RPE is an off-target cell in relation to the retinal tissue.

The term " telencephalic tissue" refers to the most anterior tissue among the brain vesicles formed on the most cranial side of the neural tube, and is a tissue that finally becomes the left and right cerebral hemispheres in the process of brain development. The telencephalic tissue is an off-target cell in relation to the retinal tissue.

The term "spinal cord tissue" refers to the tissue of the spinal cord, a columnar extension of the central nervous system that continues from the medulla oblongata, within the spinal canal. The spinal cord tissue is an off-target cell in relation to the retinal tissue.

### Morphology and Color Features of Retinal Tissue

The cell aggregate can be characterized by its morphology and color. Hereinafter, the morphology and color features of the retinal tissue for determining the region of the normal retinal tissue included in an image of the cell aggregate from the image of the cell aggregate will be described. The features of the morphology of the retinal tissue for determining the region of the normal retinal tissue are features of the morphology that can be visually recognized, and may vary depending on the observation method. Such features of the morphology of the retinal tissue are features that have been first found by confirming an image of a cell aggregate that may include a large amount of retinal tissue using an observation method described later in the process leading to the present invention. Color means a color stimulus that causes color perception, having three attributes of brightness, hue, and saturation. In some cases, an attribute of a color when a hue is particularly focused on and is determined sensuously by adding brightness and saturation to the hue is expressed as a color tone. The color and the color tone include a case where only achromatic colors are determination targets. In this case, the difference in color and color tone is only brightness, and an image having only that difference is a gray scale. FIG. 2 is an image obtained by photographing a region of one lobe of a cell aggregate through inverted observation, and typical features of the morphology of retinal tissue can be confirmed.

The retinal tissue has the following features when the entire cell aggregate is first observed macroscopically. That is, the surface of the cell aggregate in the region containing the retinal tissue is smooth, and the retinal tissue has a convex arc shape in which the direction from the center toward the outer periphery is upward, and the curvature of the arc is also gentle. In FIG. 2, it has an arc shape protruding upward. In addition, black substances (collagen and Pax6-positive cells) may be contained inside the cell aggregate.

Although a favorable retinal tissue has a continuous epithelial structure, the continuous epithelial structure of the retinal tissue is observed in a morphology clearly separated into two regions in a manner that ONbL, which is a bright ring-shaped or arc-shaped region having a certain width, is present outside, and INbL, which is a dark region, is present inside. Here, the outer ONbL region is an ONbL region composed of retinal precursor cells and photoreceptor precursors, and the inner region is an INbL region composed of inner retinal neurons.

Here, the ONbL region has a bright color tone, and the cells contained in the ONbL region have a feature that the degree of grainy texture, which is a degree to which each cell can be clearly distinguished, is higher than that of other tissues. In the normal retinal tissue, the ONbL region has a width equal to or more than a certain value.

In a continuous epithelial structure having an ONbL region, epithelial cells of the same type form an epithelial structure and are continuously present in a tangential direction. Thus, in the ONbL region, the cells of the tissue are arranged continuously in the tangential direction with regularity. The outer periphery of the ONbL region is smooth, and the angle of the tangent does not change abruptly.

The retinal tissue has a region of INbL having a dark color tone inside the region of the ONbL, and this region is a region corresponding to retinal neurons in the inner layer. The color tone is dark brown, and the cells contained therein have a low degree of grainy texture (difficult to clearly distinguish individual cells). The INbL region does not reach the outer periphery of the outer ONbL region, and the outer ONbL region and the inner INbL region usually have widths equal to or larger than a certain value. Conversely, a portion of the cell aggregate having such a feature can be determined as normal retinal tissue. Normally, when the outer ONbL region has a width equal to or more than a certain value, the inner INbL region also has a width equal to or more than a certain value. Thus, when it is confirmed that the outer ONbL region has a width equal to or more than a certain value, it is determined that the ONbL region and the inner INbL region adjacent thereto constitute normal retinal tissue.

There is also a clear boundary between the outer ONbL region and the inner INbL region. First, the arrangement of cells is different between the ONbL region and the INbL region. In the ONbL region, cells are densely arranged, and somas are in close contact with each other. In the ONbL region, the orientations of the somas are aligned. That is, the ONbL region has a structure in which cells having grainy texture are continuously arranged with regularity in a tangential direction of the contour of the tissue. On the other hand, in the inner INbL region, cells are sparsely arranged, and somas are not in close contact with each other. In the INbL region, the orientation of each soma is slightly disordered. The orientation of somas of the off-target cell is greatly disordered and is not in order.

Here, the grainy texture refers to the ease of seeing the structural features of individual cells, and the degree of the grainy texture is particularly and significantly observed when the focus position is changed. By shifting the focus position at the time of observing the cell aggregate, the degree of grainy texture at the focused position can be confirmed. In FIG. 2, the cell aggregate is shown in an arcuate shape, but the ONbL region has a high grainy texture of cells. In this manner, the ONbL region has a structure in which cells having grainy texture are continuously arranged with regularity in the tangential direction of the contour of the tissue.

The grainy texture of the retinal tissue has the following differences when compared with, for example, the optic stalk. The optic stalk has an epithelial structure similar to that of retinal tissue and has a bright color tone, but the entire tissue appears to have vertical streaks, and individual cells are unlikely to appear as independent grains. This is thought to be due to the fact that the cells of the optic stalk are more vertically long than the cells present in the retinal tissue. On the other hand, cells present in retinal tissue are nerve cells and are therefore somewhat vertically long, but the degree of vertical length is lower than in other tissues such as the optic stalk, and individual cells tend to appear as independent grains. That is, the degree of grainy texture is high in the retinal tissue. In fact, the cells in the region of ONbL (retinal precursor cells and photoreceptor precursor) have a low degree of longitudinal elongation and an elliptical nucleus. This is a structure called pseudostratified epithelium. The nucleus of mesenchymal cells and the nucleus of retina-related cells in the inner layer have a shape close to a perfect circle.

### Description of Observation Method

To identify a high-quality retinal tissue in a cell aggregate, it is necessary to accurately discriminate a difference between the retinal tissue and an off-target tissue that is not the retinal tissue. As described below, it has been confirmed that the retinal tissue can be determined with high accuracy by using images acquired using an upright microscope and an inverted microscope, respectively. The observation with both an upright microscope and an inverted microscope has the following advantages.

First, the observation with both an upright microscope and an inverted microscope has an advantage that the off-target cells on the back side of the three-dimensional retinal organoid as well as the front side thereof can be detected. That is, in the observation from only one side, the observation target can be confirmed only from one side, and the focus cannot be sufficiently adjusted over the entire region of the observation target, and the quality cannot be accurately determined in some cases. This is particularly noticeable in the case of inverted observation. Thus, for example, there is a possibility that a quality problem such as a collapse of the structure of the retina or the presence of dead cells cannot be detected. However, the possibility of such a problem occurring is reduced by performing observation from both sides.

In addition, images according to the features of the respective microscopes of the upright microscope and the inverted microscope can be obtained, and thus, there is an advantage that the quality can be determined more accurately through determination by combining the images observed from both sides of an upright microscope and an inverted microscope.

The inverted microscope has higher resolution than the upright microscope, and thus, according to inverted observation, the structure in the tissue can be observed with high accuracy, and it is easy to confirm whether the tissue is normal retinal tissue from the features of the structure. Thus, inverted observation has a feature that it is possible to clearly identify off-target tissues having an epithelial structure similar to that of the retina, such as an optic stalk and telencephalic tissue, which are off-target cells. Here, the structure in the tissue is typically a structure in which cells having a grainy texture are continuously arranged with regularity in a tangential direction of the contour of the tissue. FIG. 2 shows an image of a cell aggregate containing retinal tissue observed through inverted observation. In FIG. 2, a partial region of the cell aggregate is shown in an arc shape, but a structure in which grains appear to be aggregated is observed in a bright ring-shaped region close to the circumference. The aggregate of the grains is a part of the region of the ONbL included in the retinal tissue having grainy texture, and such a feature can be clearly observed through inverted observation. In this manner, in inverted observation, it is possible to clearly distinguish the retinal tissue and the off-target tissue in the cell aggregate.

On the other hand, the upright microscope has a wider range of focus than the inverted microscope, and thus, according to upright observation, an observation image in which the entire cell aggregate is in focus is obtained, and the features of the entire cell aggregate can be grasped and determined, but the resolution is slightly inferior to that of inverted observation. Even in the case of using an inverted microscope, information on a portion out of focus can be obtained by acquiring a plurality of observation images, but this is not realistic in the case of observing a large amount of cell aggregate. In the case of performing observation with oblique illumination, the upright microscope has a feature that it is easier to detect cells having a low light transmittance, that is, cells having a blackish color tone such as RPE and dead cells, and shadows of the shape of cell aggregate because the exposure time can be set long. FIG. 1 is an image of a cell aggregate containing retinal tissue observed through upright observation. In FIG. 1, the entire cell aggregate is shown, but a region having a blackish color tone is observed slightly below the center of the cell aggregate. The upright observation has a feature that the off-target cell can be easily detected like this.

By combining the two observation methods having the respective features as described above, it is possible to compensate for the shortcomings of the respective observation methods and to perform synergistically more effective observation. That is, by both the overall analysis and the detailed structural analysis of the cell aggregate, it is possible to more reliably detect a favorable retinal tissue in a shorter time, and it is possible to more reliably detect off-target cells that are easily detected by the respective observation methods. The feature of the synergistic analysis using upright observation and inverted observation will be described below.

In general, inverted observation is suitable for extracting detailed information of a certain section of organoid tissue because the depth of focus is shallow and the range in which the image is in focus is narrow, while the resolution is high, but it is difficult to obtain information of a portion out of focus. On the other hand, it is possible to evaluate the structure such as the arrangement of cells in detail for the section within the range of the narrow depth of focus. In this manner, the "sectional structure information" can be favorably acquired through inverted observation.

In upright observation, generally, the depth of focus is deep, and the range in which the image is in focus is wide, but the resolution is slightly low, and thus, upright observation is suitable for favorably extracting the entire texture information of the surface of the organoid tissue mainly within the range of the wide depth of focus. In upright observation, a large amount of information on the overall texture of the surface can be acquired, but on the other hand, it is difficult to acquire more detailed information than in inverted observation regarding information on the structure such as the arrangement of cells in the tissue. In this manner, "the entire texture information of the surface" can be favorably acquired through upright observation.

When a cell aggregate is observed to determine whether a portion of a certain region is a retinal tissue, the presence of an epithelial structure is usually used as an index, but there are also off-target cells having an epithelial structure like the retina among the off-target cells. Thus, although the presence of the epithelial structure can be detected from the texture information of the surface obtained in the upright observation image, it is difficult to know the arrangement and detailed structure of the cells inside the epithelial structure. In this manner, it is difficult to distinguish the retinal tissue having the epithelial structure from the off-target cell having the epithelial structure only through upright observation. On the other hand, in the structural information of the section obtained from inverted observation image, the retinal tissue and the off-target cell can be distinguished by observing the details of the arrangement of the cells and the epithelial structure. In addition, there are off-target cells (mainly RPE cells) whose color tone is close to black among the off-target cells, and in some cases, it is not possible to determine whether the off-target cells are black, because only the structural information of the section obtained from the inverted observation image has a narrow focused range, the inside of the cell aggregate is difficult to transmit light, and the inverted observation image becomes dark as a whole. Even in such a case, in the upright observation image, texture information of the surface is obtained from a wide range in which the image is in focus, and thus, it is possible to easily detect the black off-target cell. Thus, it is possible to detect the normal retinal tissue with high accuracy by complementarily comparing the observation result of inverted observation with the observation result of upright observation.

In both upright observation and inverted observation, an objective determination result can be obtained at high speed by discriminating an image photographed with a microscope through information processing. As the information processing, criteria in various viewpoints are provided, and a score of an evaluation parameter of an image of an epithelial tissue photographed with a microscope is obtained based on the criteria, whereby such determination can be performed based on the score. The criteria may be determined based on features such as color and morphology, in the image of the cell aggregate including retinal tissue. The criteria can also be set by setting a threshold or the like through information processing using an image of a cell aggregate including retinal tissue and a determination result as training data. As another information processing, a machine learning model is trained through machine learning such as deep learning using a combination of an image of a cell aggregate including retinal tissue photographed with a microscope and a label of a determination result as training data, and an image of a determination target is input to the machine learning model based on the training, and an estimated value of the determination result can be output.

Hereinafter, two examples in which off-target cells can be detected with high accuracy by combining the observation result of an inverted observation image and the observation result of an upright observation image will be described. In all of these examples, when the three-dimensional retinal organoid is observed through inverted observation, a small shadow is observed, and therefore it is necessary to check whether the shadow is an off-target cell. In general, when organoid tissue is cultured, dead cells may accumulate at the bottom of the well, and it is common that the dead cells are observed at a certain frequency in inverted observation in which the cells are seen from below. In addition, the off-target cell (RPE cell) having a black color tone has a feature that it is difficult to appropriately determine the size of the off-target cell when the size is small because the color tone (blackness) of the off-target cell varies depending on the maturity of the cell and the imaging conditions (exposure conditions and the amount of medium). In this case, by observing the shadow observed in inverted observation in combination with upright observation in which the unevenness of the surface is easily grasped, it is possible to easily determine whether the shadow is a dead cell or an off-target cell.

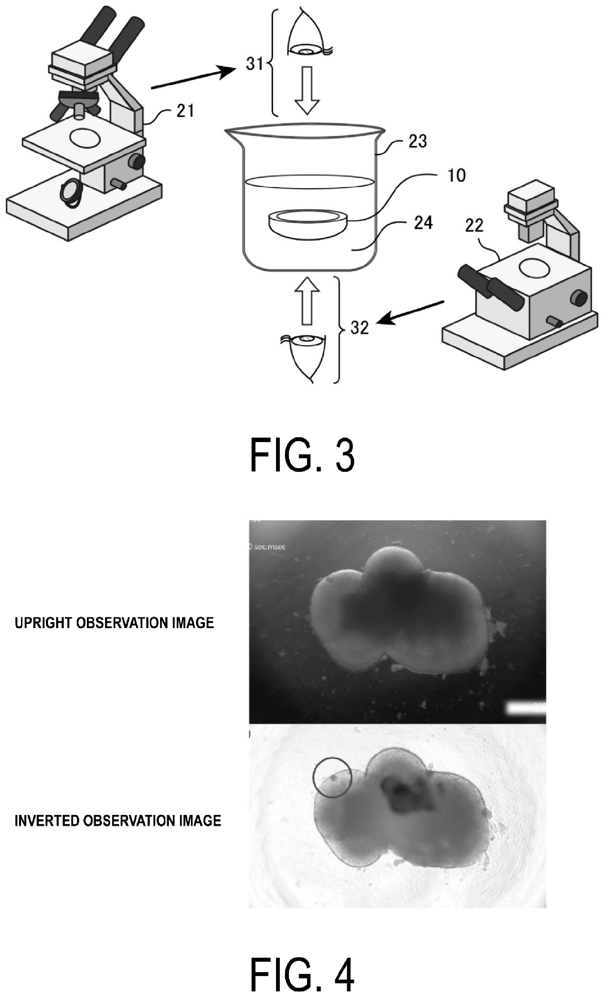

FIG. 3 shows an image diagram of inverted observation and upright observation. A cell aggregate 10 is retained in a culture solution 24 in a vessel 23. The case of observing the cell aggregate 10 through the liquid surface of the culture solution 24 from above with an upright microscope 21 (In FIG. 3, a mode of observation represented by a downward eye and an arrow symbol) is an upright observation 31, and the case of observing the cell aggregate 10 through the bottom surface of the vessel from below with an inverted microscope 22 (In FIG. 3, a mode of observation represented by an upward eye and an arrow symbol) is an inverted observation 32. The upright microscope 21 and the inverted microscope 22 can output an upright observation image and an inverted observation image, which are observation images obtained through the observation, as image data, respectively.

The upper image in FIG. 4 is an upright observation image of sample SPL1 of the cell aggregate, and the lower image in FIG. 4 is an inverted observation image of sample SPL1. In the upright observation image and the inverted observation image of FIG. 4, by mirror-inverting one of the images, the upright observation image and the inverted observation image of the cell aggregate have the same contour, and the positions in the respective images correspond to an identical position of the cell aggregate viewed from a vertical direction. Hereinafter, the same applies to the figures for comparing the upright observation image and the inverted observation image of the same cell aggregate. In the inverted observation image of FIG. 4, a small shadow is observed in a portion surrounded by a circle, but the inverted observation image is an image having only information of a region that is more planar than the actual region, and thus it is difficult to determine whether the shadow is a dead cell or an off-target cell. However, according to the upright observation image of FIG. 4, the continuity of the smooth light and dark color tone, which is a feature of the continuous epithelial structure, is broken, and it can be confirmed that there is a bulge of cells on the surface, and it can be confirmed that there are cells having different textures, and therefore, it can be determined that this shadow is a three-dimensional structure. Further, returning to the inverted observation image of FIG. 4, it can be observed that the shade of the portion surrounded by the circle has a color tone (blackness) similar to that of the black off-target cell on the right side thereof. In view of these, it can be estimated that the shadow is a similar RPE as an off-target cell.

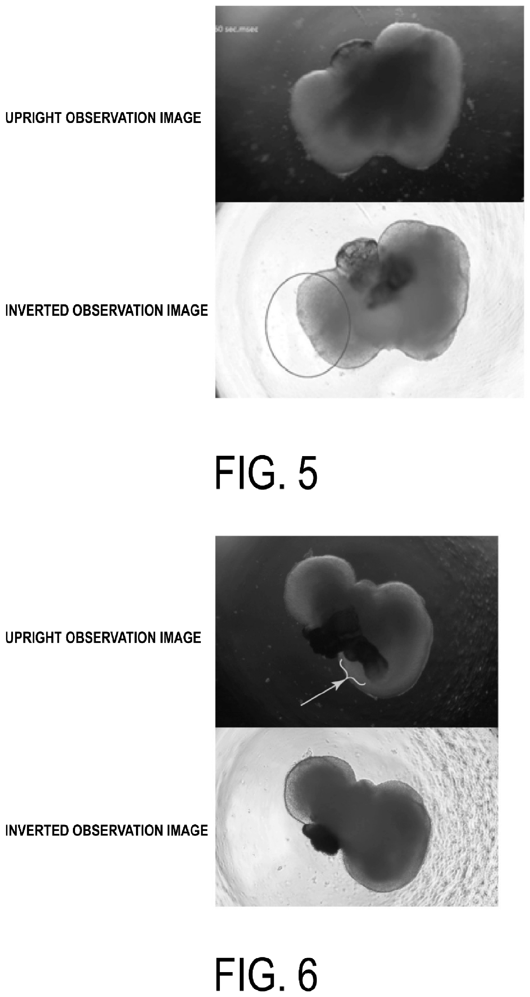

The upper image in FIG. 5 is an upright observation image of sample SPL2 of the cell aggregate, and the lower image in FIG. 5 is an inverted observation image of sample SPL2. In the inverted observation image of FIG. 5, small shadows are observed in the portions surrounded by a circle. In the upright observation image of FIG. 5, when the position corresponding to the portion surrounded by the circle in the inverted observation image is checked, it can be confirmed that the continuity of the smooth light and dark color tones is observed and the unevenness of the surface is not disturbed. This is a feature of a smooth continuous epithelial structure, and it can be determined that the shadow is not a three-dimensional structure. Further returning to the inverted observation image of FIG. 5, the shadows have the same color tone as the dead cells visible around the cell aggregate. In view of these, it can be estimated that the shadows are dead cells accumulated at the bottom of the well. Thus, the dead cells confirmed as the shadows in the portion surrounded by the circle are not adhered to the cell aggregate, and the cell aggregate can be determined to be an epithelial tissue not containing the off-target cells.

As described above, it is understood that whether the cell aggregate includes normal cells can be more accurately determined by combining the inverted observation image and the upright observation image, and the features of various observation methods will be studied in more detail. The following table lists the observation methods that have been studied, along with the samples used and the corresponding figures.

| Observation method | Contents of observation method | Sample | FIG. |
|---|---|---|---|
| 1 | Observation of cell aggregate containing neural retina with upright microscope and inverted microscope | SPL3-4 | 6-7 |
| 2 | Observation of neural retinal sheet with upright microscope and inverted microscope | SPL5-7 | 8-10 |
| 3 | Observation vessel for upright microscope observation and inverted microscope observation | SPL8 | 11-16 |
| 4 | Observation of various types of organoid tissue with upright microscope and inverted microscope | SPL9-14 | 17-22 |
| 5 | Observation of non-neural retinal tissue with upright microscope and inverted microscope | SPL15-17 | 23-25 |
| 6 | Study of observation method for inverted observation image and upright observation image | SPL18-19 | 26-39 |

### <Observation Method 1-Observation of Cell Aggregate Containing Neural Retina with Upright Microscope and Inverted Microscope>

As a new observation method for detecting off-target cells contained in organoid tissue, the effectiveness of upright microscope observation and inverted microscope observation was studied through the following procedure. First, it was studied whether the off-target cells on the cell aggregate can be reliably detected by combining upright microscope observation and inverted microscope observation.

Human iPS cells (DSP-SQ line, established by Sumitomo Pharma Co., Ltd.) were established using a commercially available Sendai virus vector (four factors of Oct3/4, Sox2, KLF4, and c-Myc, Cyto Tune kit manufactured by ID Pharma Co., Ltd.), a StemFit medium (AK03; manufactured by AJINOMOTO CO., INC.), and Laminin511-E8 (manufactured by Nippi Inc.) based on the methods described in a published protocol of Thermo Fisher Scientific (iPS2.0 Sendai Reprogramming Kit, Publication Number MAN0009378, Revision 1.0) and a published protocol of Kyoto University (feeder-free establishment and maintenance culture of human iPS cells, CiRA_Ff-iPSC_protocol_JP_v140310, http://www.CiRA.kyoto-u.ac.jp/j/research/protocol.html).

The human iPS cells (DSP-SQ strain) were subjected to feeder-free culture according to the method described in Scientific Reports, 9, 18936 (2019). StemFit medium (AK03N, manufactured by AJINOMOTO CO., INC.) was used as a feeder-free medium, and Laminin511-E8 (manufactured by Nippi Inc.) was used as a feeder-free scaffold.

As a specific maintenance culture operation, first, subconfluent human iPS cells (DSP-SQ strain) were washed with PBS, and then dispersed into single cells using TrypLE Select (manufactured by Life Technologies). Thereafter, the human iPS cells dispersed into single cells were seeded on a plastic culture dish coated with Laminin511-E8, and feeder-free cultured in StemFit medium under the presence of Y27632 (ROCK inhibitor, 10 µM). When a 6-well plate (manufactured by IWAKI CO., LTD., for culturing cells, culture area: 9.4 cm²) was used as the plastic culture dish, the number of seeded cells of the human iPS cells dispersed into single cells was 1.0 × 10⁴. One day after the seeding, the medium was exchanged with StemFit medium containing no Y27632. Thereafter, the medium was exchanged with StemFit medium containing no Y27632 once every 1 to 2 days. Thereafter, the cells were cultured until 5 days after the seeding.

As the differentiation induction operation, human iPS cells (DSP-SQ strain) were feeder-free cultured using StemFit medium until 2 days before subconfluence (to the extent that 30% of the culture area was covered with cells). The human iPS cells 2 days before subconfluence were feeder-free cultured for 2 days under the presence of SAG (300 nM) (precondition treatment).

The preconditioned human iPS cells were treated with a cell dispersion using TrypLE Select (manufactured by Life Technologies), and further dispersed into single cells by pipetting. Thereafter, the human iPS cells dispersed into single cells were suspended in 100 µL of a serum-free medium so as to be 1 × 10⁴ cells per well in a non-cell adhesive 96-well culture plate (PrimeSurface 96V bottom plate, manufactured by Sumitomo Bakelite Co., Ltd.), and subjected to suspension culture at 37 °C and 5%CO₂. As the serum-free medium (gfCDM + KSR), a serum-free medium obtained by adding 10% KSR, 450 µM 1-monothioglycerol, and 1 × chemically defined lipid concentrate to a 1 : 1 mixture solution of F-12 medium and IMDM medium was used. At the start of suspension culture (day 0 after the start of suspension culture), Y27632 (final concentration: 20 µM) and SAG (final concentration: 10 nM) were added to the serum-free medium. On day 3 after the start of suspension culture, 50 µL of the fresh above-described serum-free medium was added to a medium containing no Y27632 or SAG but containing human recombinant BMP4 (manufactured by R&D) so that the final concentration of exogenous human recombinant BMP4 became 1.5 nM (55 ng/mL).

Three days after that (6 days after the start of suspension culture), the medium was exchanged with the above-described serum-free medium containing no Y27632, no SAG, and no human recombinant BMP4. As the operation of medium exchange, an operation of discarding 60 µL of the medium in the culture vessel and adding 90 µL of the new serum-free medium was performed so that the total amount of the medium was 180 µL. Thereafter, a half amount of the medium was exchanged with the above-described serum-free medium containing no Y27632, no SAG, and no human recombinant BMP4 once every 2 to 4 days. In the half-medium exchange operation, a half volume of the medium in the culture vessel, that is, 90 µL, was discarded, and 90 µL of the above-described fresh serum-free medium was added so that the total amount of the medium was 180 µL.

The cell aggregates on day 13 after the start of suspension culture thus obtained were cultured in a serum-free medium containing CHIR99021 (3 µM) and SU5402 (5 µM) (a medium obtained by adding 1%N ₂ supplement to DMEM/F12 medium) for 3 days, that is, until day 16 after the start of suspension culture.

The obtained cell aggregate on day 16 after the start of suspension culture was cultured under 5%CO₂ conditions until day 75 after the start of suspension culture using the serum-containing media shown in [1], [2], and [3] below.
[1] From day 16 to day 40 after the start of suspension culture: a medium prepared by adding 10% fetal calf serum, 1% N₂ supplement, and 100 µM taurine to DMEM/F12 medium (hereinafter referred to as medium A).
[2] From day 40 to day 60 after the start of suspension culture: medium A and a medium prepared by adding 10% fetal calf serum, 2% B27 supplement, 2 mM glutamine, 60 nM T3 and 100 µM taurine to Neurobasal medium (hereinafter referred to as medium B), and mixing the medium A and the medium B at a ratio of 1 : 3.
[3] On and after day 60 from the start of suspension culture: medium B.

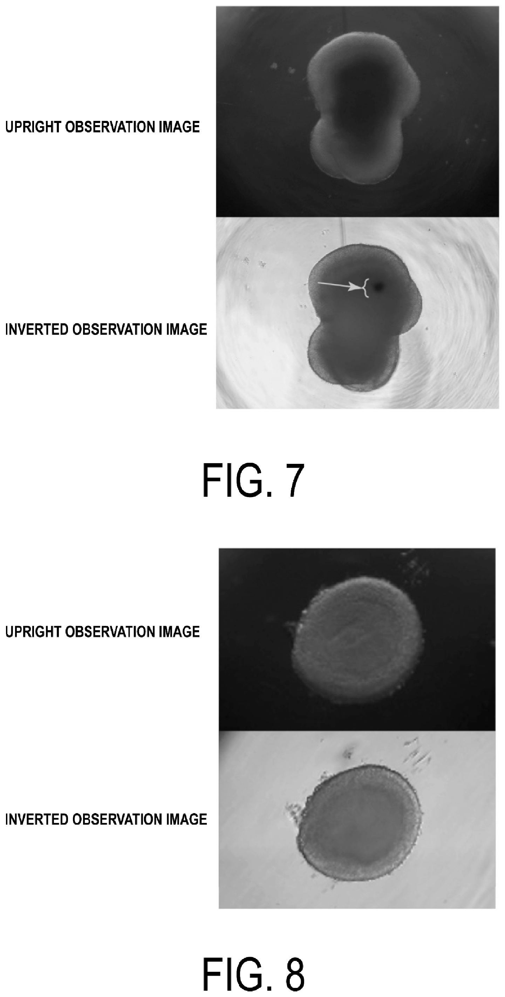

The cell aggregates on day 76 after the start of suspension culture were transferred to a 96-well slit well plate. Two cell aggregates were selected from the obtained cell aggregates, and were used as sample SPL3 and sample SPL4. A bright-field image (phase-contrast image, objective lens: 4 times) of sample SPL3 and sample SPL4 was observed with an inverted microscope (IX-83, manufactured by Olympus Corporation) to acquire inverted observation images. The same cell aggregates were observed with an upright microscope (AxioZoom, V16, manufactured by Carl Zeiss AG) at a zoom magnification of 33.3 times to acquire upright observation images. The acquired observation images of sample SPL3 and sample SPL4 are shown in FIGS. 6 and 7, respectively. In each of the figures, the upper image is an upright observation image, and the lower image is an inverted observation image.

A general procedure of photographing an observation image will be described. First, the position of the object to be photographed is aligned in the XY-axis direction of the microscope. In the case of an upright microscope, the cell aggregate is placed below the objective lens at the time of photographing an image. In the case of an inverted microscope, the cell aggregate is placed above the objective lens at the time of photographing an image. It is preferable that the type of the culture vessel and the position of the cell aggregate are stored in the control software of the microscope, and the stage of the microscope on which the culture vessel is placed is moved to control each cell aggregate to come in front of the objective lens. When a special multi-well plate such as a U-bottom or V-bottom plate is used as the culture vessel, the XY coordinates of the cell aggregate are uniquely determined as the position of the well. It is also possible to store the coordinates of each well in the control software of the microscope and control each well to move in front of the objective lens.

Next, the microscope is focused in the Z direction on the object to be photographed. In the case of upright microscope observation (stereoscopic microscope and upright zoom microscope), the center of the cell aggregate in a vertical direction is focused. In the case of inverted microscope observation, the central portion of a large epithelial tissue contained in the cell aggregate is focused. In the case of more detailed analysis, it is preferable to use a Z-stack image obtained by reconstructing images photographed while shifting the focal plane little by little.

Next, the microscope images are photographed with an upright microscope and an inverted microscope, respectively. Here, the order of photographing in inverted observation and inverted observation may be reversed, but it is preferable to perform inverted observation after upright observation because upright observation becomes difficult when the vapor adheres to the lid of the well plate. After observation images are photographed, the image data is output from the microscope, and the image data is acquired by an image analysis system such as a normal cell aggregate determination system 100 described later by a data transfer means (for example, a LAN, a wireless LAN, Bluetooth (registered trademark), a USB, a USB memory, an external SSD, an external hard disk, or the like).

In sample SPL3 of the cell aggregate, the presence of RPE having black pigment, which is an off-target cell, can be confirmed in the portion indicated by the arrow in the upright observation image of FIG. 6. However, in the inverted observation image of FIG. 6, only a part of the same RPE was confirmed. In the inverted observation image of FIG. 7, the presence of RPE, which is an off-target cell, can be confirmed in the portion indicated by the arrow in sample SPL4 of the cell aggregate. However, in the upright observation image of FIG. 7, the RPE was not confirmed. From these results, it was confirmed that, when organoid tissue having a spatially complex structure is observed, there is a tissue that cannot be confirmed through observation from one direction, and the entire tissue can be observed through observation from both upper and lower directions in the vertical direction.

### <Observation Method 2: Observation of Neural Retinal Sheet with Upright Microscope and Inverted Microscope>

Whether the observation from both directions with an upright observation image and an inverted observation image is effective for the neural retinal sheet was studied by the following procedure. Some of the cell aggregates produced by the method described in Observation Method 1 were observed, and an upright observation image and an inverted observation image were acquired. Based on the acquired upright observation image and inverted observation image, a site having a continuous epithelial structure and appearing to be divided into two layers of the ONbL (including the photoreceptor cell layer and the neural retinal precursor cell layer) and the INbL when viewed from the vertical direction was specified in the cell aggregate, and the site was determined as the neural retina. Then, a cell aggregate determined to contain neural retina was selected from the plurality of observed cell aggregates. Thereafter, while the selected cell aggregate was observed with a stereoscopic microscope, the neural retina was cut out from the cell aggregate using tapered tweezers and scissors under observation with a stereoscopic microscope (manufactured by Carl Zeiss, STEREO) to produce a neural retinal sheet consisting of a single lobe. Three neural retinal sheets were selected from them and were studied as sample SPL5 to sample SPL7.

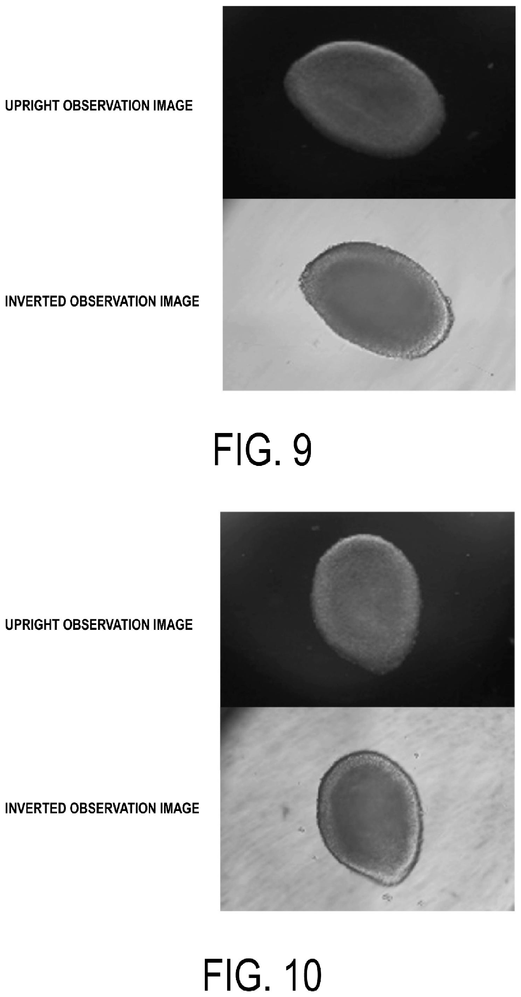

The produced neural retinal sheets were transferred to a 96-well U-bottom plate. The neural retinal sheet was observed as a bright-field image (phase-contrast image, objective lens: 4 times) using an inverted microscope (manufactured by Olympus Corporation, IX-83). The same neural retinal sheet was observed at a zoom magnification of 50 times using an upright microscope (manufactured by Carl Zeiss, zoom microscope AxioZoom). The obtained observation images of sample SPL5 to sample SPL7 are shown in FIGS. 8 to 10, respectively. In each of the figures, the upper image is an upright observation image, and the lower image is an inverted observation image.

In FIGS. 8 to 10, in both the upper upright observation image and the lower inverted observation image, the features of the neural retina (the presence of a continuous epithelial structure and a two-layer structure of the ONbL and the INbL) were observed over the entire circumference, but the features of the non-neural retina were not observed at any position of the samples. In this manner, it was confirmed that a region having a high purity of the target cell can be selected by observing the cell aggregate from both directions using an upright observation image and an inverted observation image. Thus, it was confirmed that the observation from both directions with an upright observation image and an inverted observation image is an effective method for the determination of the neural retinal sheet.

### <Observation Method 3: Observation Vessel for Upright and Inverted Microscope Observation>

An observation vessel suitable for upright microscope observation and inverted microscope observation was studied. It was confirmed that a flask, a petri dish, or a multi-well plate can be used as the shape of the culture vessel, but a multi-well plate is preferable, and a multi-well plate having a V-bottom or U-bottom concave surface is more preferable. This is because the use of the multi-well plate having such a shape enables the cell aggregate to be stationary even in a liquid.

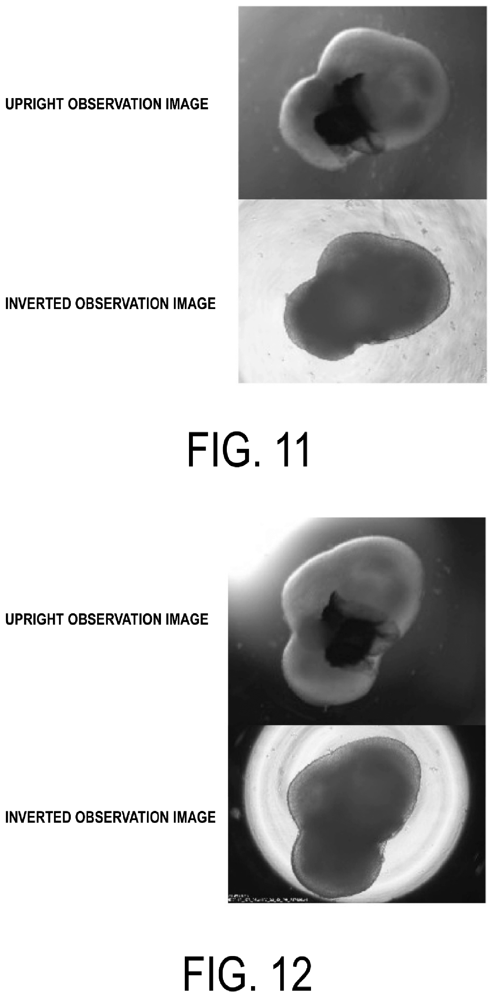

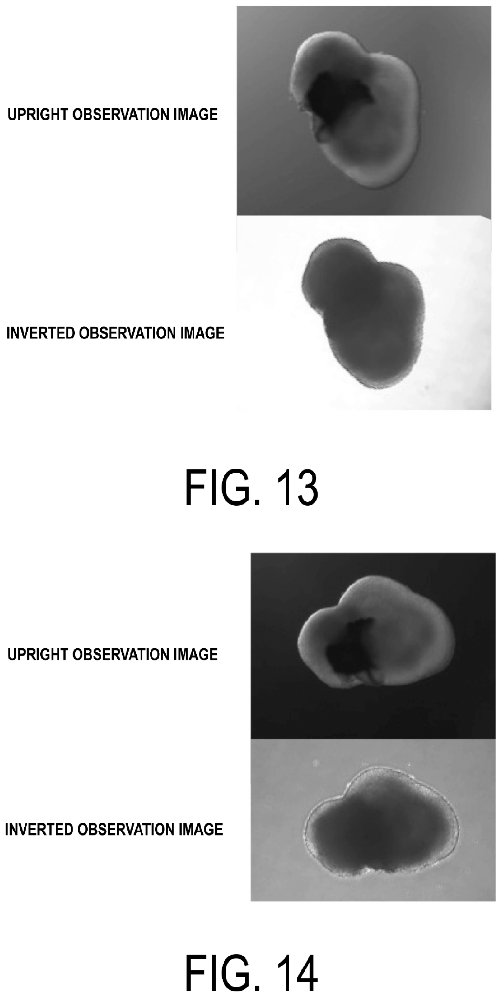

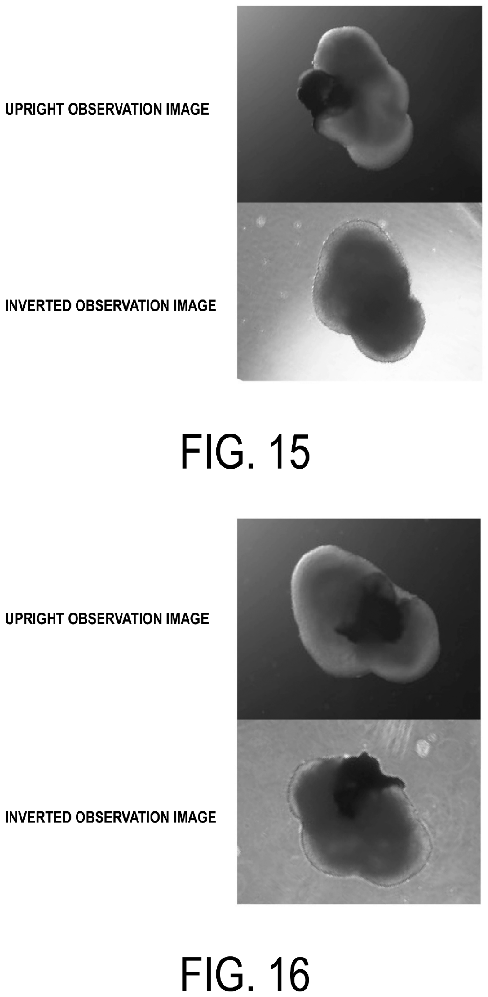

One of the cell aggregates produced by the method described in Observation Method 1 was selected to acquire sample SPL8. Sample SPL8 was transferred to vessel PLT1: 96-well plate (U-bottom (slit well)), vessel PLT2: 96-well plate (V-bottom), vessel PLT3: 24-well plate, vessel PLT4: 6-well plate, vessel PLT5: 60 mm dish, and vessel PLT6: T25 flask, and observed with an inverted microscope (manufactured by Olympus Corporation, IX-83) in a bright-field image (Hoffman modulation contrast, objective lens: 10 times, tiling 2 × 2), and further observed with an upright microscope (manufactured by Carl Zeiss), whereby an inverted observation image and an upright observation image were acquired. The observation images obtained by retaining and photographing the obtained sample SPL8 in vessel PLT1: 96-well plate (U bottom (slit well)), vessel PLT2: 96-well plate (V bottom), vessel PLT3 : 24-well plate, vessel PLT4: 6-well plate, vessel PLT5: 60 mm dish, and vessel PLT6: T25 flask are shown in FIGS. 11 to 16, respectively. In each of the figures, the upper image is an upright observation image, and the lower image is an inverted observation image.

As a result, in any of the culture vessels of vessel PLT1: 96-well plate (U-bottom (slit well)), vessel PLT2: 96-well plate (V-bottom), vessel PLT3: 24-well plate, vessel PLT4: 6-well plate, vessel PLT5: 60 mm dish, and vessel PLT6: T25 flask, the features of the retinal tissue were able to be photographed, and the RPE cells were able to be confirmed in at least one of the upright observation image or the inverted observation image. From this, it was confirmed that the method of combining the upright observation and the inverted observation can be used regardless of the shape of the vessel.

Further, in vessel PLT1: 96-well plate (U bottom) and vessel PLT2: 96-well plate (V bottom), the observation angles of the cell aggregate (angles at which the cell aggregate was viewed) in the inverted observation image and the upright observation image matched with each other, but in the other culture vessels, a slight change was observed in the observation angles of the cell aggregate in the two images. This is considered to be because, when the vessel was moved between the photographing of the upright observation image and the photographing of the inverted observation image, the cell aggregate was slightly rotated during the movement because the cell aggregate was freely movable except for vessel PLT1: 96-well plate (U-bottom) in the case of the size of the cell aggregate used. Thus, it was confirmed that observation from both directions can be performed with higher accuracy by selecting, as the observation vessel, an observation vessel having both a feature of the size suitable for the organoid tissue of the observation target and a feature of the well plate having a bottom surface shape that is convex downward. In this manner, specifically, it has been confirmed that vessel PLT1 and vessel PLT2 are suitable for sample SPL8. In general, when the number of wells of a vessel is large, the size of the wells is small, and it is possible to process many cell aggregates at once. Thus, by selecting a well having an appropriate size that is not too large with respect to the size of the cell aggregate to be determined, the observation angles of the cell aggregate in the inverted observation image and the upright observation image can be matched as much as possible, and the throughput of the observation can be improved by increasing the number of wells.

The liquid volume in the culture vessel can be, for example, from 0.05 to 0. 8 mL/cm², preferably from 0.15 to 0.6 mL/cm². For example, when 100 µL is placed in a 96-well plate, the most suitable liquid amount is about 0.3125 mL/cm². For example, when 10 mL is placed in a 90 mm dish, the most suitable liquid amount is about 0.17 mL/cm².

As the material of the culture vessel, plastic or glass can be used, and transparent plastic is preferable. This is because plastic is easy to surface-process and can be coated or such to facilitate cell culture. The vessel needs to be colorless and transparent, and a plastic culture dish can be generally used.

### <Observation Method 4: Observation of Various Types of Organoid Tissue with Upright Microscope and Inverted Microscope>

It was studied whether the observation with an upright microscope and an inverted microscope can be applied to organoid tissue other than the retinal tissue obtained during culture. In addition, it was verified whether each organoid tissue was distinguishable through observation with an upright microscope and an inverted microscope.

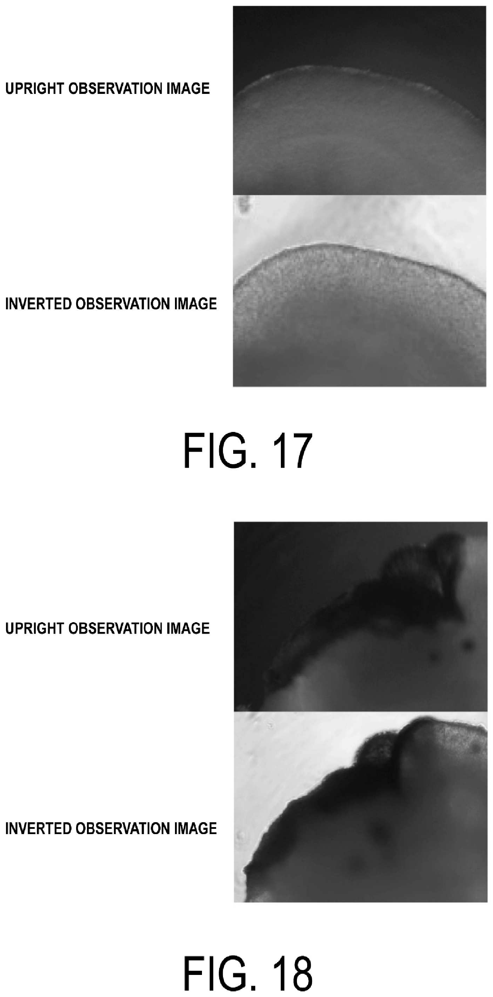

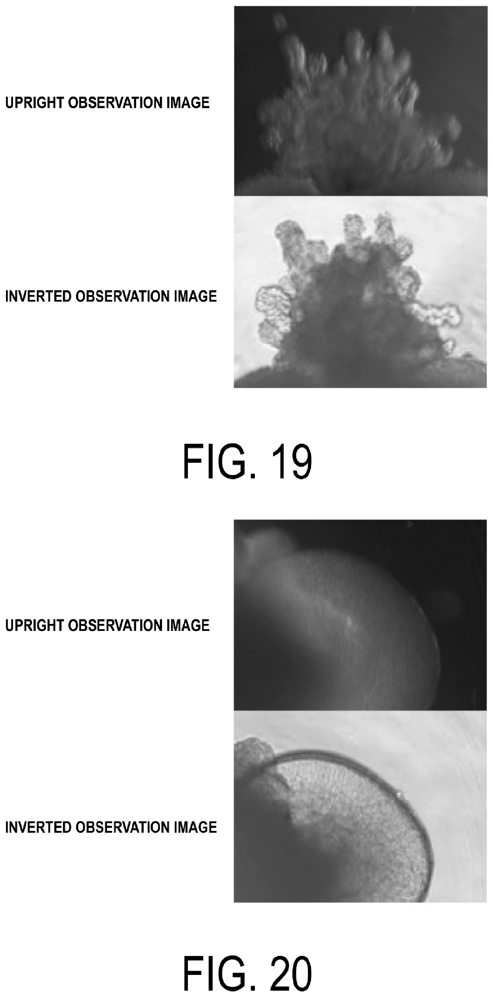

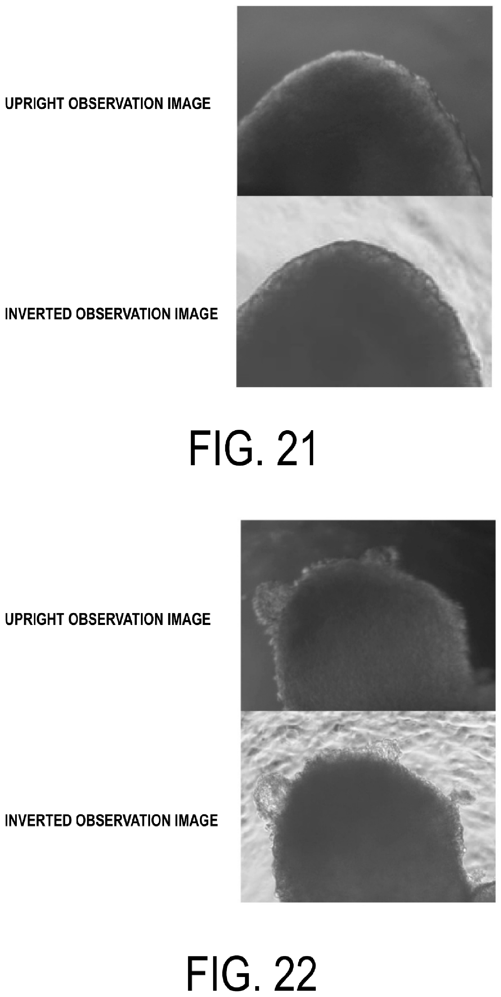

Cell aggregates were obtained by the method described in Observation Method 1. Cell aggregates respectively containing tissues having different features of morphology, a first tissue: retinal tissue, a second tissue: RPE, a third tissue: ciliary body, a fourth tissue: optic stalk, a fifth tissue: telencephalic tissue, and a sixth tissue: spinal cord tissue were selected and acquired as sample SPL9 to sample SPL14. The observation was performed by the method described in Observation Method 2, and an inverted observation image and an upright observation image were acquired according to the above-described procedure. The respective tissues in the obtained observation images from sample SPL9 to sample SPL14 are appropriately enlarged and shown in FIGS. 17 to 22, respectively. In each of the figures, the upper image is an upright observation image, and the lower image is an inverted observation image.

In the retinal tissue, in addition to the fact that the features in color tone were confirmed in the upright observation image and the inverted observation image, it was confirmed that the cells having granular shapes in which individual cells can be clearly distinguished were arranged in the inverted observation image. In addition, it was confirmed from the results of comparing the morphology and quality of many obtained cell aggregates that, to obtain a cell aggregate including a normal region that is qualitatively acceptable, it is preferable that an ONbL having a width of 0.03 mm or more has a length of such a degree that the ONbL is continuously present at 45 degrees or more when viewed in a cell aggregate having a diameter of 1 mm or more. The cell aggregate usually has a shape in which a plurality of ellipses are fused. The center of the cell aggregate is preferably the center of a shape obtained by approximating the shape of the cell aggregate by a predetermined method. Specifically, the center of the cell aggregate can be the center of a reference ellipse, which is the smallest ellipse that the cell aggregate touches at the largest number of points on its contour. The reference ellipse having such a shape is located immediately inside the contour of the cell aggregate and is in contact with the contour at as many points as possible (usually, four or more points), and therefore the shape of the cell aggregate can be approximated well. Thus, when an outer neuroblastic layer having a width of 0.03 mm or more is present continuously at 45 degrees or more from the center of the cell aggregate, it can be confirmed that the retinal tissue is a qualitatively acceptable retinal tissue as long as the off-target cells are not contained.

The RPE was observed as a tissue having a color tone close to black in the upright observation image and the inverted observation image. However, it was confirmed that the ability to distinguish the blackness of the pigment exhibited by the RPE from the blackness exhibited by the low light transmittance due to the discontinuity of the tissue structure such as the inside of the aggregate is more excellent in the upright observation image than in the inverted observation image.

The ciliary body was observed as a grape-like tissue having a wavy surface in the upright observation image and the inverted observation image. It was confirmed that the upright observation image was able to be observed more favorably because the ciliary body was in focus in a slightly wider range in the vertical direction.

It was confirmed that the optic stalk had a feature in a color tone similar to that of the retinal tissue in the upright observation image and the inverted observation image, and elongated cells were arranged in the inverted observation image, and it was confirmed that the optic stalk can be identified from the morphology of the retinal tissue.

In the telencephalic tissue, a bright layer was observed on the outermost side in the upright observation image and the inverted observation image, but it was confirmed that the width of the bright layer was small as compared with the retinal tissue. When the telencephalic tissue and the retinal tissue were compared, it was confirmed that the inverted observation image had a more remarkable difference in the width of the layer and higher distinguishability than the upright observation image.

In the spinal cord tissue, an orderly structure was not able to be confirmed as the entire tissue in the upright observation image or the inverted observation image, and the spinal cord tissue was observed as a tissue having a tissue shape with a non-smooth surface. The light transmittance of the spinal cord tissue was moderate, and the entire tissue was observed in a grayish color tone in the upright observation image, and it was confirmed that the distinguishability from the telencephalic tissue was higher than that in the inverted observation image.

From these results, it was confirmed that the inverted observation image can capture the structural information of the tissue and the shape of the cells constituting the tissue with high resolution by focusing on the epithelial tissue in the vertical direction, while the upright observation image is excellent in capturing organoid tissue by focusing on a wide range in the vertical direction, and can more accurately capture a tissue with low light transmittance by easily obtaining the contrast of brightness. Thus, it was confirmed that the observation using an inverted microscope and an upright microscope can observe the entire organoid tissue from both directions, and can enhance the detection accuracy of a plurality of types of tissues by utilizing the features of the observation images obtained by the respective observation methods.

### <Observation Method 5: Observation of Non-Neural Retinal Tissue with Upright Microscope and Inverted Microscope>

Whether upright microscope observation and inverted microscope observation can be applied to organoid tissue of a non-neural retinal tissue obtained by a culture method different from the method for producing a neural retina was studied.

Cell aggregates were produced by the method described in Observation Method 1, except that the medium added on day 3 after the start of suspension culture was changed to a serum-free medium containing no human recombinant BMP4.

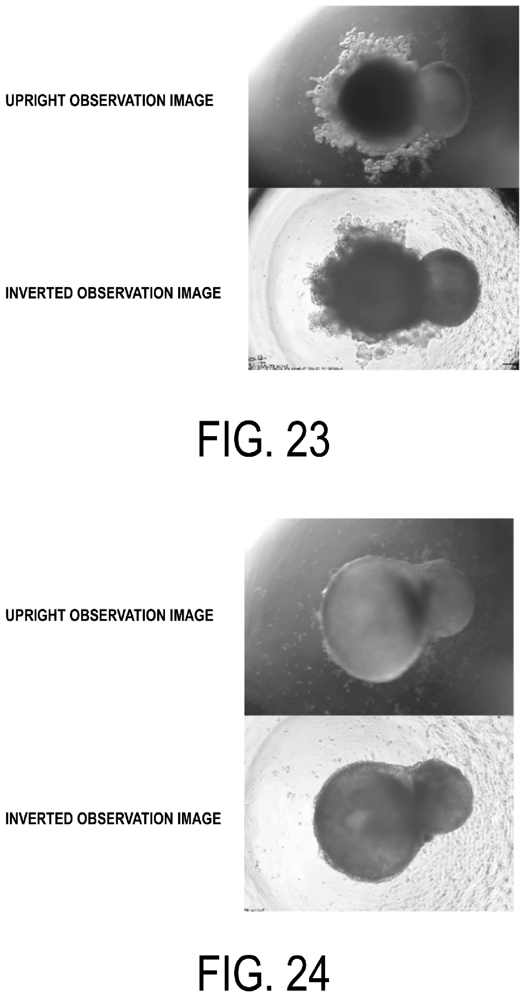

Three non-neural retinal tissues were selected from the produced cell aggregates and acquired as sample SPL15 to sample SPL17. The method described in Observation Method 3 was applied to the samples to acquire inverted observation images and upright observation images. The obtained images of sample SPL15 to sample SPL17 are shown in FIGS. 23 to 25, respectively. In each of the figures, the upper image is an upright observation image, and the lower image is an inverted observation image.

Based on FIGS. 23 to 25, the morphology and color of the cell aggregates were observed and evaluated. In the evaluation of the morphology, first, the entire cell aggregate is observed through upright observation in which a wider range in the vertical direction is in focus, and it is confirmed whether there is a feature of color tone that the outside is bright and the inside is dark. Next, the outer periphery is observed through inverted observation that enables more detailed observation of the structure, and whether tissues in which individual cells can be clearly distinguished as grains are continuously arranged in the tangential direction with regularity is checked. Next, whether the cell aggregate has a normal retinal tissue region is checked by the fact that the cell aggregate does not contain an off-target cell, the fact that there is no off-target cell on the upper side of the cell aggregate in upright observation, and the fact that there is no off-target cell on the lower side of the cell aggregate in inverted observation. When sample SPL15 to sample SPL17 were checked by the procedure as described above, the features of the neural retina (that there is a continuous epithelial structure and a two-layer structure of ONbL and INbL) were not confirmed from the upright observation image or the inverted observation image, and it was confirmed that they are non-neural retinas. In this manner, it was confirmed that the observation from both directions in the upright observation image and the inverted observation image can be applied to the non-neural retinal tissue obtained by a production method different from the method for producing neural retina, and that the non-neural retinal tissue can be clearly identified. That is, it was confirmed that the observation from both directions in the upright observation image and the inverted observation image can be applied to appropriately identify not only the retina but also the organoid tissue of various regions and organs in the body.

### <Observation Method 6: Study of Observation Method for Inverted Observation Image and Upright Observation Image>

Further studies were conducted on specific settings of the microscopes in the observation method. Hereinafter, general settings of an upright microscope and an inverted microscope and features thereof will be described.

The upright microscope (bright field observation) has a feature that a range in which the image is in focus is wide and information of a wide width in the Z direction can be acquired. In the case of a stereoscopic microscope, stereoscopic vision with both eyes is possible. In addition, when a zoom microscope is used, there is a feature that the magnification can be easily switched to an appropriate magnification. For example, an objective lens of from 0.5 to 2.0 times with a magnification of from 2 to 200 times can be used for the upright microscope observation. As the light source illumination method, either illumination with vertical incident light or oblique illumination can be used.

The inverted microscope (bright field observation, transmitted light) has a feature that a focused range is narrow but an object can be observed in detail. For example, the following combinations of magnifications and imaging methods can be used. That is, a four-fold phase contrast, a four-fold differential interference contrast, a four-fold polarization, a ten-fold Hoffman modulation contrast, a ten-fold phase contrast, a ten-fold differential interference contrast, a ten-fold polarization, a twenty-fold Hoffman modulation contrast, a twenty-fold phase contrast, a twenty-fold differential interference contrast, a twenty-fold polarization, and the like can be used. The features of the combination of the main magnification and imaging method will be described below.
· Four-fold phase contrast: Best used for observation of cells. The structure in the epithelial tissue can be observed well. Both retinal cells and off-target cells can be stably and well observed with moderate contrast. Due to the low magnification, it is easy to observe and evaluate the entire cell aggregate.
· Four-fold polarization: Shadows and array of cells in the epithelial tissue can be observed well. Due to the low magnification, it is easy to evaluate the entire cell aggregate.
· Ten-fold Hoffman modulation contrast: An image similar to differential interference contrast with a gradation of brightness and darkness applied to the entire sample is obtained, and is suitable for observation of thick organoid tissue. The structure in the epithelial tissue can be best observed. The gradation of brightness and darkness in the observation image is large, and thus, it is easier to find blackened RPE. However, due to the large gradation of brightness and darkness, there is a slight difference in the way of viewing (the difference in brightness and darkness between the outside and the inside, the ease of viewing the boundary, the ease of capturing the grainy texture of photoreceptor cells, and the like) even in the same type of cells depending on the difference in the vertical and horizontal positions of the observation range. In addition, the magnification of the objective lens is 10 times, which is large, and thus, there is a possibility that the entire cell aggregate cannot be observed, and the range to be focused is narrow, and it may be difficult to focus on the entire outer periphery of the cell aggregate. A similar image can be observed with differential interference contrast.
· Ten-fold phase contrast: The structure in epithelial tissue is easily observed. However, it is difficult to focus.
· Ten-fold polarization: The shadow of epithelial tissue and the array of cells are easily observed.

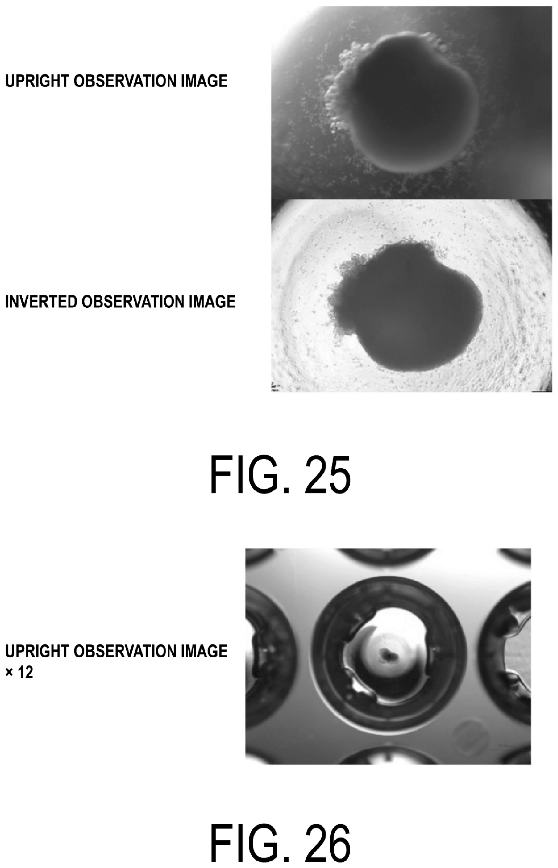

Cell aggregates were produced by the method described in Observation Method 1, and two of them were selected and acquired as sample SPL18 and sample SPL19. Sample SPL18 was observed under six conditions of a zoom magnification of 12, 20, 32, 50, 80, and 100 times with an upright microscope (manufactured by Carl Zeiss, STEREO DISCOVERY V12). Sample SPL18 was observed under three conditions of a bright-field image (phase-contrast image, objective lens: 4 times), a bright-field image (phase-contrast image, objective lens: 10 times), and a bright-field image (Hoffman modulation contrast, objective lens: 10 times, 2 × 2 tiling) with an inverted microscope (IX-83, manufactured by Olympus Corporation). The obtained observation images are shown in FIGS. 26 to 34, respectively. The correspondence between the observed images and the figures is shown in the following table. The scale is not constant between the figures.

| Sample SPL18 | | | | | | |
|---|---|---|---|---|---|---|
| Upright observation image | 12 times | 20 times | 32 times | 50 times | 80 times | 100 times |
| FIG. | FIG. 26 | FIG. 27 | FIG. 28 | FIG. 29 | FIG. 30 | FIG. 31 |

| Inverted observation image | Phase contrast 4 times | Phase contrast 10 times | Hoffman 10 times | | | |
|---|---|---|---|---|---|---|
| FIG. | FIG. 32 | FIG. 33 | FIG. 34 | | | |

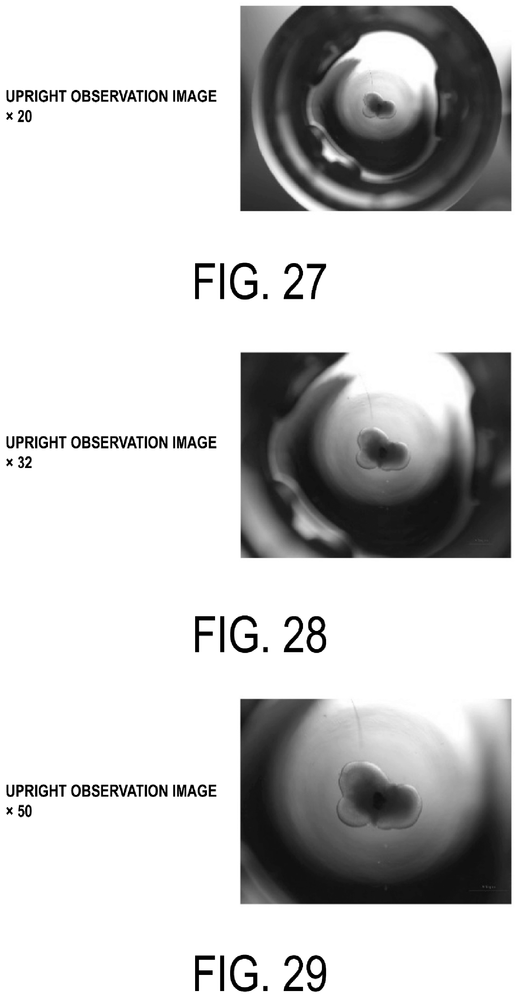

When the upright observation images at different magnifications in FIGS. 26 to 31 are checked, in all the samples, the feature of the color tone that the outside is bright and the inside is dark can be confirmed as a whole, and it can be confirmed that the neural retina is included. In addition, it can be confirmed that all the samples have a portion with a black color tone inside and that RPE is included. However, in the upright observation images with magnifications of 12 times and 20 times shown in FIGS. 26 and 27, the samples cannot be confirmed with a sufficient size, and thus, magnifications from 32 times to 100 times shown in FIGS. 28 to 31 are preferable.

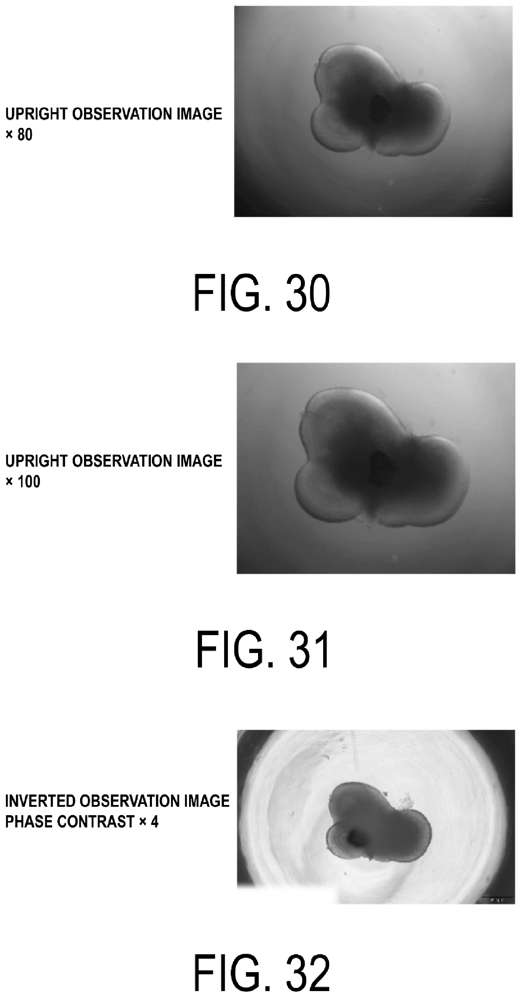

When the inverted observation images in FIGS. 32 to 34 are checked, the feature of the color tone of the neural retina that the outside is bright and the inside is dark can be confirmed, and it can also be confirmed that the black RPE in color tone is present inside, regardless of the magnification of any observation method.

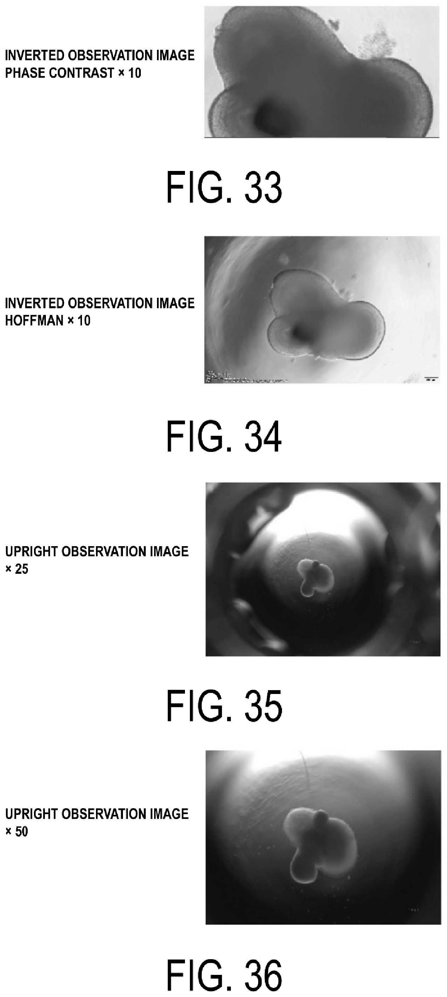

Sample SPL19 was observed under three conditions of a zoom magnification of 25, 50, and 80 times with an upright microscope (manufactured by Carl Zeiss, STEREO DISCOVERY V12). Sample SPL19 was observed under three conditions of a bright-field image (phase-contrast image, objective lens: 4 times), a bright-field image (phase-contrast image, objective lens: 10 times), and a bright-field image (Hoffman modulation contrast, objective lens: 10 times, 2 × 2 tiling) with an inverted microscope (IX-83, manufactured by Olympus Corporation). The obtained observation images are shown in FIGS. 35 to 40, respectively. The correspondence between the observed images and the figures is shown in the following table. The scale is not constant between the figures.

| Sample SPL19 | | | |
|---|---|---|---|
| Upright observation image | 25 times | 50 times | 80 times |
| FIG. | FIG. 35 | FIG. 36 | FIG. 37 |
| Inverted observation image | Phase contrast 4 times | Phase contrast 10 times | Hoffman 10 times |
| FIG. | FIG. 38 | FIG. 39 | FIG. 40 |

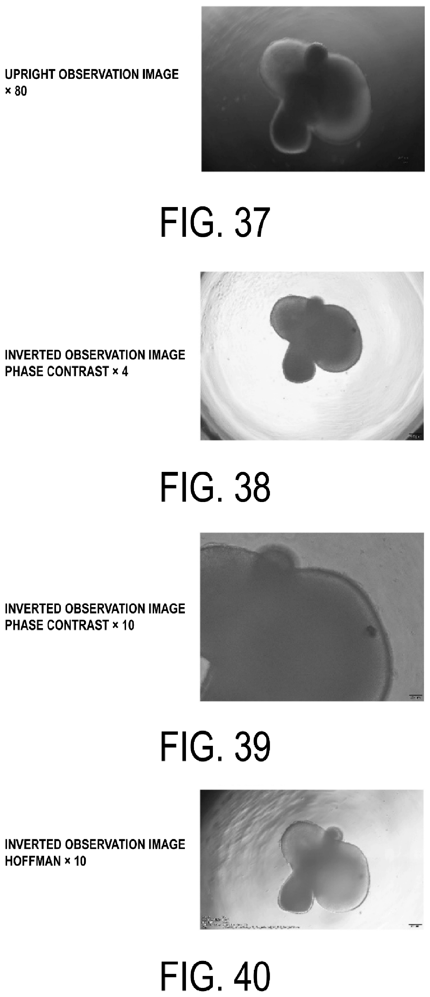

When the upright observation images at different magnifications in FIGS. 35 to 37 are checked, in all the samples, the feature of the color tone that the outside is bright and the inside is dark can be confirmed as a whole, and it can be confirmed that the neural retina is included. In addition, it can be confirmed that all the samples have a portion with a black color tone inside, and it can be confirmed that RPE is included. In the upright observation image at a magnification of 25 times shown in FIG. 35, the size of the sample is slightly small, but the feature of the color tone can be confirmed in detail to some extent. The magnifications of 50 to 80 times shown in FIGS. 36 and 37 are more preferable.

When the inverted observation images in FIGS. 38 to 40 are checked, the feature of the color tone of the neural retina that the outside is bright and the inside is dark can be confirmed, and it can be confirmed that the black RPE in color tone is present inside, regardless of the magnification of any observation method.

As shown in FIGS. 26 to 40, it was confirmed that it was possible to determine that the cell aggregate contained neural retina and RPE under any observation conditions. It was confirmed that, to observe the entire cell aggregate with higher accuracy, 2 × 2 tiling is preferably desirable at a zoom magnification of 32 to 100 times in upright observation and at an objective lens of 4 times or an objective lens of 10 times in the inverted observation image.

From the above results, it was confirmed that the entire cell aggregate can be efficiently and more accurately observed by observing the organoid tissue with an upright microscope and an inverted microscope and obtaining an upright observation image and an inverted observation image. It was confirmed that the features of various types of tissues contained in the cell aggregate were detected with higher accuracy by utilizing the respective features of the upright microscope and the inverted microscope.

### <Configuration of Normal Cell Aggregate Determination System 100>

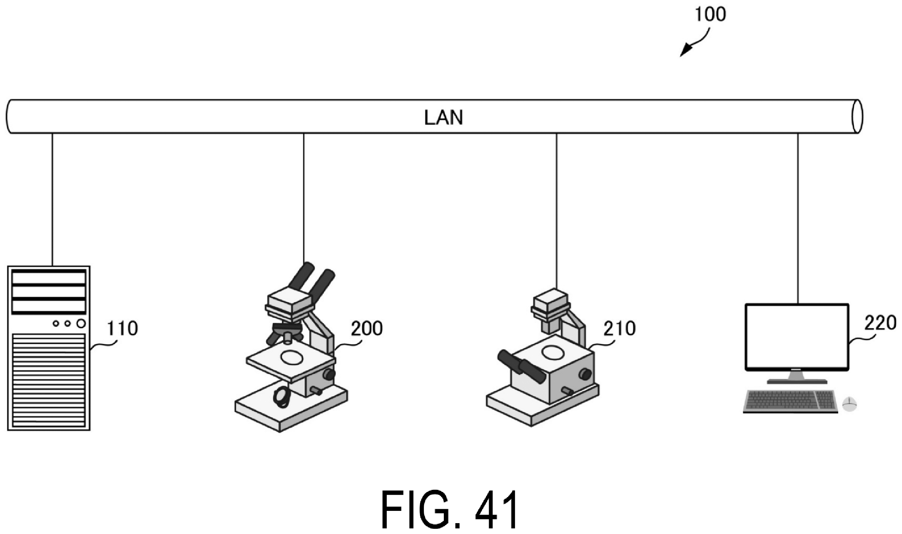

As described above, it was confirmed that the cell aggregate having a normal retinal tissue region can be discriminated with high accuracy by using both an upright observation image and an inverted observation image. As a result of further studying a method through automated determination using a computer as image evaluation of cell aggregates, it was confirmed that extraction of normal retinal tissue can be performed through automated determination by analyzing an upright observation image and an inverted observation image of cell aggregates including neural retina. In such a case, it was confirmed that the determination accuracy can be increased by performing determination by combining two types of images, that is, an upright observation image and an inverted observation image, and that more precise analysis and position-specific detection of a retinal tissue are possible by performing integrated analysis after associating the two types of images with positions. In addition, it was confirmed that the analysis combining the upright observation image and the inverted observation image is a highly versatile method that can be applied not only to organoid tissue including the retina but also to various types of organoid tissue such as non-retinal nerve tissue and the RPE. Hereinafter, a system for determining a normal retinal tissue region from an upright observation image and an inverted observation image of a cell aggregate through automated determination will be described. A normal cell aggregate determination system 100 according to an embodiment of the present invention will be described with reference to the figures. FIG. 41 is a schematic configuration diagram illustrating a configuration of the normal cell aggregate determination system 100 according to an embodiment of the present invention. The normal cell aggregate determination system 100 typically includes a normal cell aggregate determination server 110, an upright microscope 200, an inverted microscope 210, and an operation terminal 220 connected to each other via a local area network LAN. A digital camera 215 (not illustrated) including an optical system capable of performing enlarged photographing can be used instead of the upright microscope 200 or the inverted microscope 210.

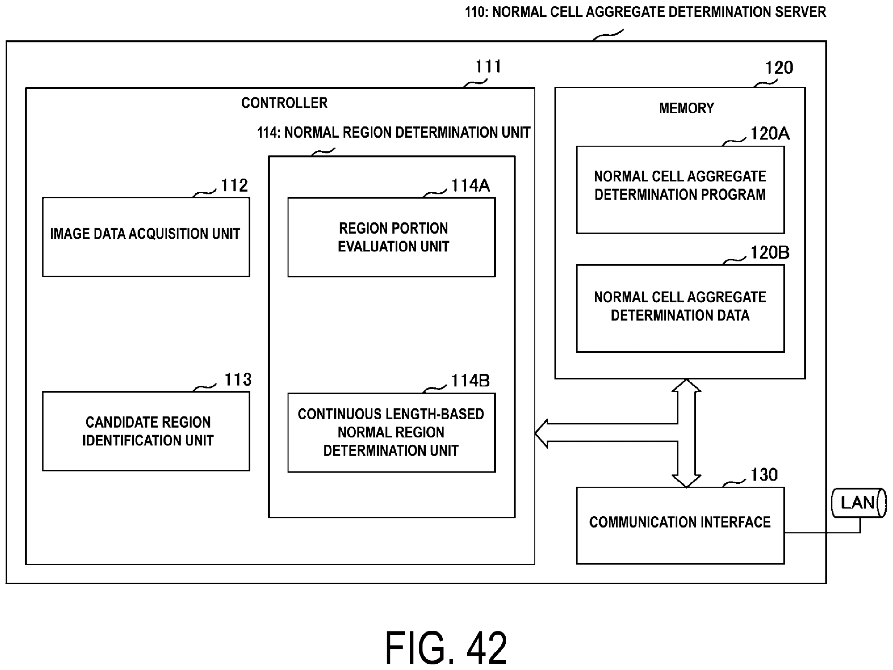

FIG. 42 shows a block diagram of a normal cell aggregate determination server 110. The normal cell aggregate determination server 110 is typically in the form of a server computer in which a computer program for realizing the function thereof is installed. The normal cell aggregate determination server 110 is mainly composed of a controller 111, a memory 120, and a communication interface 130. The controller 111 includes an image data acquisition unit 112, a candidate region identification unit 113, and a normal region determination unit 114 as components having more specific functions. The normal region determination unit 114 includes, as components having more specific functions, a region portion evaluation unit 114A and a continuous length-based normal region determination unit 114B. The memory 120 stores a normal cell aggregate determination program 120A and normal cell aggregate determination data 120B.

The controller 111 is a processing circuit for executing various functions for controlling the operation of the normal cell aggregate determination server 110, and is typically an information processing circuit including a processor (not illustrated) for causing the normal cell aggregate determination server 110 to execute a predetermined function by executing a computer program for realizing the predetermined function, a RAM (not illustrated) which is a temporary data storage area for the processor, and the like.

The memory 120 is typically a non-volatile storage unit such as a hard disk drive or a solid state drive (SSD), and stores a computer program and data referred to when the computer program is executed. When the computer program is executed, basic software such as an OS (operation system) is usually used, but the function of the OS is included in the function of the controller 111 executing the computer program, and thus the description thereof will be omitted here. The function of the normal cell aggregate determination server 110 is realized by configuring a specific information processing circuit according to the function by executing the computer program by the controller 111. The memory 120 stores the normal cell aggregate determination program 120A as a computer program, and stores the normal cell aggregate determination data 120B as data to be referred to when the normal cell aggregate determination program 120A is executed. The normal cell aggregate determination data 120B includes specific values of various determination criteria, and when determination based on the determination criteria is executed, the controller 111 reads the data from the memory 120 and refers to the data. The normal cell aggregate determination data 120B can be incorporated as part of the program logic in the normal cell aggregate determination program 120A, and in this case, the normal cell aggregate determination data 120B does not have to be stored in the memory 120 as individual data. By the controller 111 reading the normal cell aggregate determination program 120A from the memory 120 and executing the program, a specific information processing circuit that realizes various functions related to the normal cell aggregate determination is configured, and the information processing circuit executes an operation that realizes the functions. That is, a specific information processing circuit of the image data acquisition unit 112, the candidate region identification unit 113, and the normal region determination unit 114 including the region portion evaluation unit 114A and the continuous length-based normal region determination unit 114B is thereby configured. The memory 120 also has a data storage area (not illustrated) for storing any data such as image data and calculation results.

The image data acquisition unit 112 is an information processing circuit that realizes a function of acquiring image data of an observation image of a cell aggregate to be determined. More specifically, the image data acquisition unit 112 is an information processing circuit that realizes a function of acquiring image data of a cell aggregate to be determined as image data of an upright observation image and an inverted observation image photographed using the upright microscope 200 and/or the inverted microscope 210, respectively, via a LAN and storing the image data in a data storage area of the memory 120. When the cell aggregate to be determined is photographed with the digital camera 215, the image data acquisition unit 112 acquires image data of an observation image photographed using the digital camera 215.

The candidate region identification unit 113 is an information processing circuit that realizes a function of identifying a candidate region included in a region that is a candidate for the normal retinal tissue in the cell aggregate in the observation image represented by the image data. More specifically, the candidate region identification unit 113 is an information processing circuit that realizes a function of identifying a region determined to satisfy a candidate region determination criterion for the inverted observation image and/or the upright observation image as a candidate region included in a region to be a candidate for the normal retinal tissue region based on the candidate region determination criterion regarding the color corresponding to the observation method (inverted observation, upright observation, and the like) in the image region (region corresponding to portion where cell aggregate is present in inverted observation image and upright observation image) of the cell aggregate of the inverted observation image and/or the upright observation image.

The image of the cell aggregate in the inverted observation image and/or the upright observation image may have a chromatic color or an achromatic color such as gray scale. In the case of using an image represented by a chromatic color including blue, green, and red, brightness, hue, and saturation can be used as candidate region determination criteria regarding color. In the case of using gray scale, only brightness can be used as candidate region determination criteria regarding color.

The region that becomes the candidate of the normal retinal tissue region is a region that can be determined to be the normal retinal tissue region at a later stage by satisfying the candidate region determination criterion. The candidate region is a region included in a part or all of a candidate region of such a normal retinal tissue region, and when it satisfies a predetermined criterion at a later stage, it can be determined as a normal retinal tissue region, or both the normal retinal tissue region and a region adjacent thereto can be determined as a normal retinal tissue region. The candidate region is typically an ONbL, and when it satisfies a predetermined criterion at a later stage, the ONbL and an INbL adjacent to the inner side (the center side of the cell aggregate) thereof can be determined as a normal retinal tissue region.

The normal region determination unit 114 is an information processing circuit that realizes a function of determining whether the cell aggregate has a normal retinal tissue region. More specifically, the normal region determination unit 114 is an information processing circuit that realizes a function of determining a region portion of a candidate region determined to satisfy the normal region determination criterion corresponding to the observation method (inverted observation, upright observation, and the like) as a normal retinal cell region based on the normal region determination criterion regarding brightness and/or morphology corresponding to the observation method in the image region of the cell aggregate of the inverted observation image and/or the upright observation image. The normal region determination unit 114 includes a region portion evaluation unit 114A and a continuous length-based normal region determination unit 114B as information processing circuits that realize more specific functions.

The region portion evaluation unit 114A is an information processing circuit that realizes a function of obtaining an evaluation score of a region portion included in the candidate region of the epithelial tissue. More specifically, the region portion evaluation unit 114A is an information processing circuit that realizes a function of obtaining an evaluation score of each of region portions present in a range obtained by dividing a candidate region into a plurality of regions along a curve representing a direction in which the candidate region is present, based on evaluation criteria regarding color and/or morphology corresponding to the observation method, for a candidate region identified in an image region of a cell aggregate position-associated as necessary in each of the inverted observation image and/or the upright observation image. The evaluation criteria can be criteria regarding a width such as a width when the candidate region is viewed along the direction in which the shape extends, or a width in which a straight line passing through the center of the cell aggregate or the reference ellipse thereof overlaps the candidate region, or criteria regarding a length such as a continuous length of a region portion having a predetermined width or more. The candidate region is present at a position close to the contour (outer periphery) of the cell aggregate, and thus, the curve representing the direction in which the candidate region is present is usually along the contour or the outer periphery of the cell aggregate.

The position association is processing performed when both images of the inverted observation image and the upright observation image are used when the evaluation score of the region portion is obtained, and in the inverted observation image and the upright observation image obtained by photographing the same cell aggregate, any position in the image region of the cell aggregate in each image is associated so as to correspond to an identical position of the cell aggregate viewed from the vertical direction. For this purpose, first, by mirror-inverting one of the inverted observation image and the upright observation image, the positions of the cell aggregates on the image region corresponding to the cell aggregates in the upright observation image and the inverted observation image are matched. It is preferable that the inverted observation image and the upright observation image have the same magnification (the same object is represented by an image region having the same number of pixels). In this case, one of the images is enlarged/reduced as necessary to make the magnifications of the images the same.

As necessary, an image conversion operation such as parallel movement or rotation is performed on the image region of at least one cell aggregate so that the position of the same pixel in each image region corresponding to the cell aggregate corresponds to an identical position of the cell aggregate when viewed from the vertical direction. This operation may be performed by using a known feature point matching method including the stages of feature point detection, feature value description, and matching so that the contours of the image regions of the cell aggregates match each other. Alternatively, the operator may perform the operation using the eyes. In addition, by integrating an inverted microscope and an upright microscope so as to photograph the inverted observation image and the upright observation image without moving the vessel holding the cell aggregates, the value of parallel movement and rotation (and the enlargement ratio/reduction ratio) can be set as a constant, and the above-described image conversion operation can also be set as a predetermined conversion operation.

In addition, even when a conversion operation such as parallel movement or rotation is not performed on an image, a function (direction/distance of parallel movement, rotation angle) representing the conversion operation may be stored, and the function may be applied when the images are compared. When the inverted observation image and the upright observation image have different magnifications, it is preferable to store the enlargement ratio/reduction ratio for one image as a function of the conversion operation so that the images have the same magnification when the images are compared.

The continuous length-based normal region determination unit 114B is an information processing circuit that realizes a function of determining a region portion whose evaluation score has a value equal to or more than a reference value as a normal retinal tissue region when the region portion is continuously present by a length equal to or more than a predetermined reference length. More specifically, the continuous length-based normal region determination unit 114B is an information processing circuit that determines, as a normal retinal tissue region, a region portion determined to be present continuously by a length equal to or more than a predetermined normal region determination reference length in a direction along a curve representing the direction in which the region portion in which the evaluation score of the inverted observation image and/or the upright observation image has a value equal to or more than a normal region determination reference value corresponding to the observation method (inverted observation, upright observation) extends. The predetermined normal region determination reference length is not limited to the length of the curve representing the direction in which the shape of the region portion extends, but may be an angle of a range in which the region portion is present as viewed from the center of a reference ellipse which is the smallest ellipse that contacts the cell aggregate at the largest number of points (a central angle when the region portion is approximated to an arc). The predetermined reference length is to be determined by an actual physical length, but may be calculated by converting the actual physical length into another unit such as the number of continuous pixels in the image.

The communication interface 130 is an interface for connecting the normal cell aggregate determination server 110 to a local area network LAN to transmit and receive data. The normal cell aggregate determination server 110 is connected to the local area network LAN by the communication interface 130 in a wired or wireless manner, and can perform data communication with other components such as the upright microscope 200, the inverted microscope 210, and the operation terminal 220 connected to the local area network LAN.

The upright microscope 200 is a microscope that includes an optical system for performing upright magnifying observation and a camera for photographing an image magnified by the optical system. Image data photographed with the camera can be transmitted to the normal cell aggregate determination server 110 from a communication interface built in the upright microscope 200 (or a terminal such as a laptop PC connected to the upright microscope 200) via a LAN.

The inverted microscope 210 is a microscope that includes an optical system for performing inverted magnifying observation and a camera for photographing an image magnified by the optical system. The image data photographed with the camera can be transmitted from a communication interface built in the inverted microscope 210 (or a terminal such as a laptop PC connected to the inverted microscope 210) to the normal cell aggregate determination server 110 via a LAN.

The images are not limited to those obtained from the inverted microscope 210 and the upright microscope 200. Instead of such microscopes, a digital camera (a complementary metal oxide semiconductor (CMOS) camera, a charge-coupled device (CCD) camera, or the like) 215 having an optical system capable of performing magnifying photographing can be used. The digital camera 215 preferably has an autofocus function and a focus position adjustment function. In this case, the cell aggregate is photographed using a digital camera from both directions of the cell aggregate along the substantially vertical direction, and image data is acquired.

Accordingly, for example, a Z-stack image obtained by reconstructing a stack image photographed by gradually shifting the focal plane from the initially autofocused focal point can be used as an image including three-dimensional information. The normal cell aggregate determination server 110 may include an image selection unit (not illustrated) that selects an image to be used for determination from among a plurality of images photographed by slightly shifting the focal plane in the Z direction according to a predetermined criterion or selection by the user, and may be configured to select an appropriate image by the operation of the image selection unit. The selection criteria may be various viewpoints such as the clarity of the entire image or a specific region, the presence or absence of an object having a specific shape, the shape or color thereof, and the like.

The operation terminal 220 is a component that receives an operation input for an operator to operate the normal cell aggregate determination server 110, transmits the operation input to the normal cell aggregate determination server 110, and outputs a determination result or the like transmitted from the normal cell aggregate determination server 110. The operation terminal 220 may include an input unit such as a keyboard and a mouse and an output unit such as a display, and can display a graphical user interface on the display to allow an operator to input and output information to and from the normal cell aggregate determination server 110.

Further, the normal cell aggregate determination system 100 may be connected to an automatic cutout device as a part of a retinal tissue automatic cutout device. The position information of the normal retinal tissue region determined by the normal cell aggregate determination system 100 is transmitted to the automatic cutout device, and thus the automatic cutout device can automatically cut out the normal retinal tissue region from the cell aggregate. The automatic cutout device has a controller that controls various operations for cutout based on the position information obtained from the normal cell aggregate determination system 100, the controller is connected to at least a device that performs cutout such as a cutter whose operation can be controlled, a holding tool for holding a cell aggregate whose operation can be controlled, and the like, and the controller controls the holding tool and the cutter based on the position information of the normal retinal tissue region to hold the cell aggregate and cut out the retinal tissue. The automatic cutout device can also be configured to have a well position control device that moves a desired well to a work area for cutout, and the normal cell aggregate determination system 100 transmits information of a well in which a cell aggregate including a normal retinal tissue region is held to the automatic cutout device, so that the automatic cutout device can sequentially move only wells in which a cell aggregate including a normal retinal tissue region is held to the work area and cut out a normal retinal tissue region therefrom.

### Examples

Hereinafter, the present invention will be specifically described with reference to four examples, but the present invention is not limited to these examples. Cell aggregates were produced by the method described in Observation Method 1, and 12 cell aggregates were acquired as sample SPL20 to sample SPL31. The procedure of the example described below was performed on each of the samples. In all of the examples, automated determination is executed on the observation image of the cell aggregate, but different procedures of Example 1 to Example 4 were executed by combining the observation method and the processing method of the observation image. The following table lists the description of the four examples and the corresponding figures.

| Examples | Description of Examples | FIG. |
|---|---|---|
| Example 1 | Application of automated determination to inverted observation image of cell aggregate | 43, 44, 51, 56 |
| Example 2 | Application of automated determination to upright observation image of cell aggregate | 45 to 47, 52, 53, 57 |
| Example 3 | Determination through combination of individual determination results of upright observation image and inverted observation image of cell aggregate | 48, 58 |
| Example 4 | Simultaneous determination of combination of upright observation image and inverted observation image of cell aggregate | 49, 50, 54, 59 |

### <Example 1: Application of Machine Determination to Inverted Observation Image of Cell Aggregate>

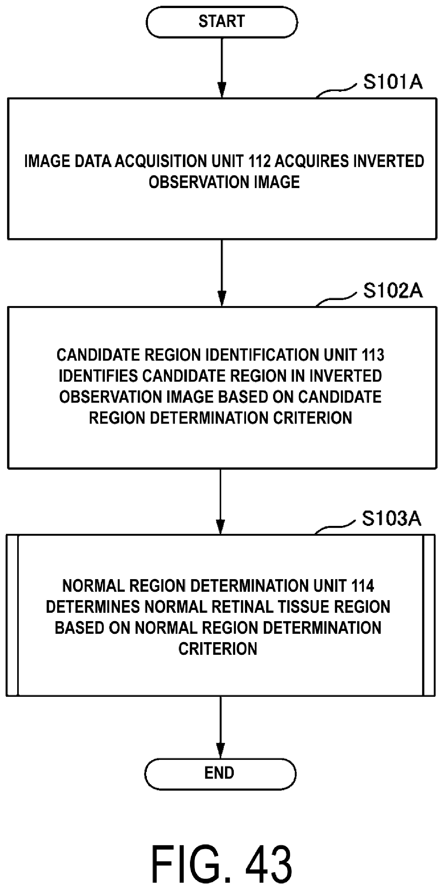

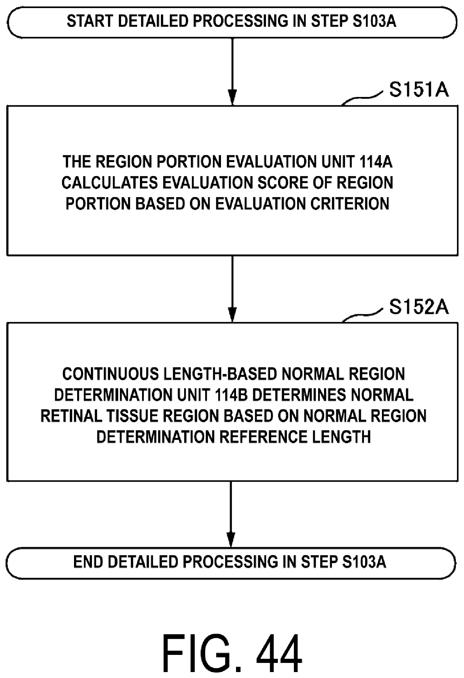

Whether automatic determination is possible by applying automated determination to an inverted observation image was studied. The overall operation flow is illustrated in FIG. 43, and the detailed operation flow relating to the normal region determination is illustrated in FIG. 44. The following stages were performed using sample SPL20 to sample SPL31 as cell aggregates to be determined.

### [1] Image Data Acquisition Stage

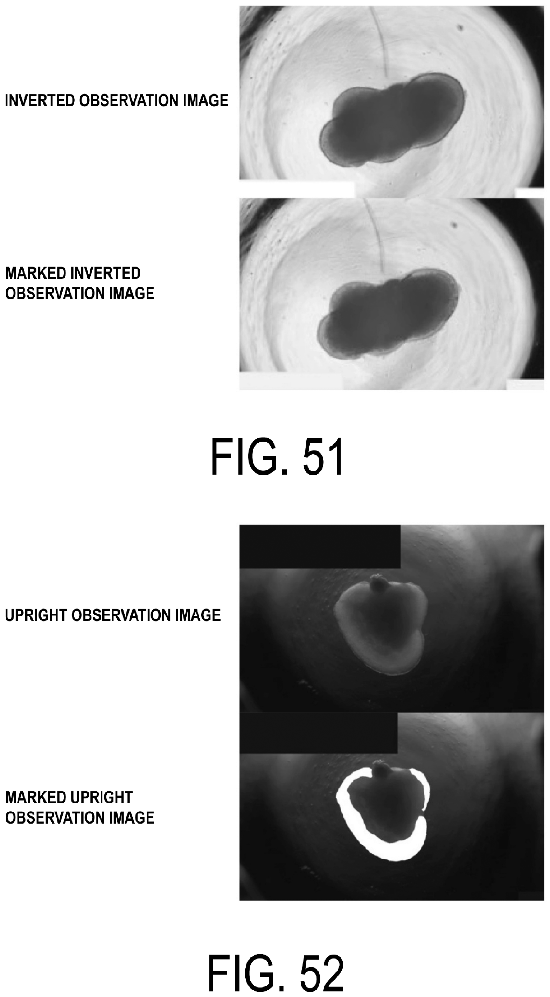

A vessel holding the cell aggregate to be determined is set in an inverted microscope 210, and the cell aggregate is photographed using the inverted microscope 210. Preferably, the cell aggregate is photographed in a state of being suspended in a liquid such as a culture solution in a plastic vessel having a downward convex shape or dent and/or having one or more isolated culture regions. The image data acquisition unit 112 acquires the image data of the inverted observation image thus acquired from the inverted microscope 210 via a LAN (step S101A). Specifically, the operator observes a bright-field image (phase-contrast image, objective lens: 4 times) of the cell aggregate to be determined held in the culture solution in an appropriate vessel using the inverted microscope 210, and photographs the image to acquire an inverted observation image. The inverted microscope 210 transmits image data representing the inverted observation image to the normal cell aggregate determination server 110 via a LAN, and the image data acquisition unit 112 acquires the image data and stores the image data in the data storage area in the memory 120. The upper side of FIG. 51 shows the acquired inverted observation image of the cell aggregate to be determined.

Next, analysis of the inverted observation image, which will be described in detail below, is performed on the acquired inverted observation image, and an analysis result of the inverted observation image is acquired. In the procedures (1) to (6) described later, image processing and image analysis processing were performed using a software construction tool Pipeline Pilot Version 22.1.0 of Dassault System as a part of the normal cell aggregate determination program 120A.

### [2] Candidate Region Identification Stage

The candidate region identification unit 113 identifies a region determined to satisfy the candidate region determination criterion as a candidate region included in a region that is a candidate of a normal retinal tissue region, based on the candidate region determination criterion regarding color, in the image region of the cell aggregate in the inverted observation image (step S102A). In this stage, an image region corresponding to the cell aggregate (that is, an image of the cell aggregate itself) is specified, and the candidate region is specified from among the specified image regions based on the candidate region determination criterion regarding color. The image region corresponding to the cell aggregate can be specified through image processing based on features such as brightness and morphology, for example. In addition, the region outside the cell aggregate can be clearly excluded by excluding the periphery (near the four corners) of the observation image, and thus, the image region corresponding to the cell aggregate can be specified. Here, the candidate region is typically a region of an ONbL. This is because the epithelial structure of the neural retina has a structure having a bright arc-shaped ONbL on the outside and a dark INbL on the inside thereof, and therefore, the ONbL is included in a part of the normal retinal tissue region. By providing the candidate region determination criterion based on a feature regarding color such as a bright color tone, it is possible to quickly identify an ONbL as a candidate region from an image region of a cell aggregate. As the feature regarding color, it is preferable to use only brightness. As a specific reference of the brightness, the brightness can be higher than the maximum value or the average value of the brightness of the central region or the entire region of the image region of the cell aggregate by a predetermined degree (relative value, absolute value). It is also possible to identify the candidate region by specifying an image region of a cell aggregate and excluding other image regions after performing identification processing based on the candidate region determination criterion regarding color first. Specifically, the candidate region identification stage can be performed by executing the following procedures (1) to (3).
(1) The candidate region identification unit 113 converts the image data of the inverted observation image represented by the RGB image into a monochrome image of 256 gradations. Typically, information of brightness in color is used as a candidate region determination criterion, and thus, information of hue and saturation is removed from the image data through this procedure.
(2) The candidate region identification unit 113 performs noise reduction processing in the inverted observation image using a known image processing technique, specifies a region satisfying a certain brightness standard, and combines sites having an adjacent relationship. The color tone is dark and the change in the gray scale is poor in the region near the center of the cell aggregate because of the presence of the INbL, and the color tone is bright and the change in the gray scale is poor in the region near the outer periphery of the cell aggregate because of the presence of the ONbL. Through this procedure, the candidate region identification unit 113 extracts a region having a large change in gradation as a circumferential contour of the cell aggregate, identifies a relatively bright region inside the contour as a region that can be a candidate region, and identifies and marks the region as a continuous region. Here, when the sites are combined, it is also possible to use, as a reference, a feature of morphology from a predetermined viewpoint such as a certain number of continuous pixels, in addition to the brightness. For example, it is possible to exclude a bright portion that does not continue for two or more pixels in any of the vertical, horizontal, and oblique directions from the target of the combination of the sites. This makes it possible to remove extremely small noise and the like.

In addition, the candidate region is usually present along the outer periphery of the cell aggregate, and thus, it is also possible to use, as the candidate region determination criterion, a feature of morphology in which the contour of the outermost portion of the region to be identified when viewed from the central portion of the image region of the cell aggregate substantially coincides with the contour of the image region of the cell aggregate. In addition, the candidate region is usually present along the outer periphery of the cell aggregate without a large angle change, and thus, it is also possible to use feature of morphology in which the change in the angle of the tangential direction of the contour of the region to be identified is continuous as the candidate region determination criterion. In addition, the candidate region usually has a width of a certain degree or more, it is also possible to use, as the candidate region determination criterion, a feature of morphology in which the width of the region (the width between the outermost portion and the innermost portion) of the cell aggregate in the region to be identified as viewed from the central portion of the image region has a predetermined reference width or more.

Off-target cells cannot be present in the candidate region. Thus, it is also possible to execute an off-target cell identification stage of identifying whether an off-target cell is present based on the off-target cell determination criterion for determining that an off-target cell is present in the region to be identified, and to use the absence of an off-target cell as the candidate region determination criterion as a feature of morphology. As the off-target cell determination criteria, it is possible to use that the orientation of the cells is disordered, that the cells are similar to features of typical off-target cells such as optic stalk, RPE, telencephalic tissue, and spinal cord tissue, that the color tone is darker than the average of the entire cell aggregate, and the like.

(3) The candidate region identification unit 113 removes, as noise, a region marked as a region that can be a candidate region in contact with the outermost side, that is, four sides of the entire image through a known image processing technique, and removes the marking. This is to eliminate adverse effects caused by a case where a non-cell aggregate is photographed on the outer side of the image and may become noise. In the procedure described above, such noise having a large size may remain adjacent to the four sides of the image, and thus, the region that is in contact with the four sides and may be noise is excluded from the region that may be the candidate region through this procedure.

These procedures identify candidate regions within the image region of the cell aggregate. On the lower side of FIG. 51, an image in which the candidate region is marked slightly brightly with respect to the inverted observation image of the cell aggregate to be determined is shown. Here, it is preferable to create an image in which the candidate region is overlaid on the original inverted observation image, and store the image data in the data storage area in the memory 120 as an output image for visual confirmation.

### [3] Normal Region Determination Stage

Next, the normal region determination unit 114 determines, in the candidate region of the inverted observation image, the candidate region of the identification target determined to satisfy the normal region determination criterion of the inverted observation image as a normal retinal tissue region based on the normal region determination criterion regarding the brightness and/or the morphology of each of the inverted observation images (step S103A). A cell aggregate having such a normal retinal tissue region is determined as qualitatively acceptable. This normal region determination stage is executed by stages from [3A] to [3B], which will be described in detail below. The normal region determination will be described with reference to the detailed operation flow illustrated in FIG. 44.

### Region Portion Evaluation Stage

The region portion evaluation unit 114A obtains an evaluation score of each of region portions present in a range obtained by dividing a candidate region into a plurality of regions along a curve representing an extending direction of the candidate region based on evaluation criteria regarding color and/or morphology corresponding to an observation method called inverted observation with respect to the candidate region identified in the image region of the cell aggregate of the inverted observation image (step S151A). The evaluation criteria may relate to the morphology such as a width and a length, but it is preferable to use the width as the evaluation criteria at this stage.

In the region portion evaluation stage, to further narrow down the candidate region to a region having a higher possibility of including normal retinal tissue, each evaluation score is obtained in each region portion present in a range obtained by dividing the candidate region into a plurality of regions. Specifically, this stage is performed by executing procedures (4) to (5) described later.

Here, the concept of the candidate region of the cell aggregate and the length and width of the region portion will be described. The actual physical width represented by µm or the like determines the characteristics of the cell aggregate, and thus, the length and the width are used as criteria for determination. However, in the image analysis, the calculation amount can be reduced by performing the calculation by converting the actual physical length or width into the number of pixels. When analysis is performed based on the number of pixels, and the direction in which the width extends is other than the direction in which the pixels are linearly arranged, such as the X axis and the Y axis, the length in any direction can be represented by the number of pixels by counting the number of pixels after rotating the direction in which the width extends so as to be parallel to the X axis or the Y axis. Hereinafter, even in a case where some determination is performed using the number of pixels, similarly, an actual physical length or the like is a criterion of the determination, but when analysis is performed using the image data of the observation image, analysis is performed using the number of pixels corresponding to the physical length or the like serving as the criterion to reduce the calculation amount. The analysis may be performed by converting the number of pixels into an actual length or width represented by µm or the like.

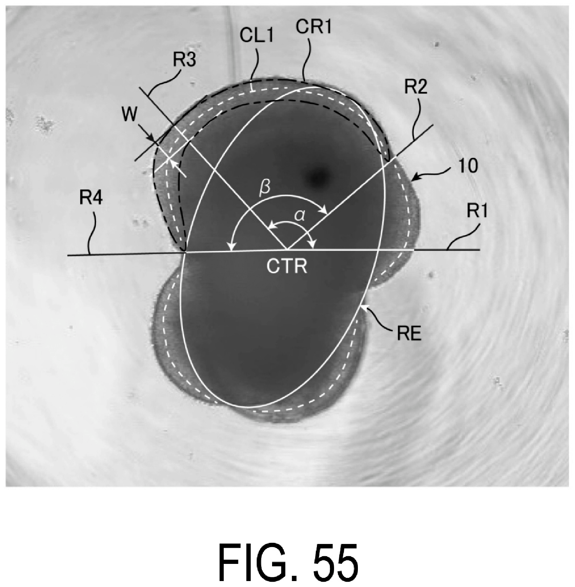

See the conceptual diagram for measuring a width W of a candidate region of a cell aggregate 10 shown in FIG 55. The candidate region of the cell aggregate 10 usually has an elongated shape, and is typically a shape approximating an arc of an ellipse along a contour of the cell aggregate 10. In FIG. 55, candidate regions which are bright arc-shaped regions can be confirmed at four positions near the upper left, upper right, lower right, and lower left, and curves representing the directions in which the shapes of the respective candidate regions extend are indicated by broken lines. The length of a candidate region CR1 indicated by an alternate long and short dash line at the upper left in FIG. 55 corresponds to the length of a curved line CL1 representing the direction in which the elongated shape extends. The curved line CL1 is a curved line connecting the widthwise central portions of the candidate region CR1. The curve representing the direction in which the elongated shape extends like this may be a curve passing through the central portion in the width direction in the direction in which the shape extends.

FIG. 55 shows a reference ellipse RE which is the smallest ellipse inside the contour of the cell aggregate 10 and in contact with the contour at the largest number of points. The reference ellipse RE is usually an ellipse whose major axis direction substantially coincides with the longitudinal direction of the entire cell aggregate, and it is preferable that the reference ellipse RE is in contact with the cell aggregate at least near four points, that is, two intersection points between the major axis and the ellipse and two intersection points between the minor axis and the ellipse. Such a reference ellipse RE is a favorable approximation of the overall shape of the corresponding cell aggregate. Here, as described above, the length of the candidate region may be based on a unit representing the length such as the number of pixels along the length of the curve representing the direction in which the shape of the candidate region extends (the curve CL1 for the candidate region CR1), or may be represented by an angle of a range in which the candidate region is present when viewed from the center of the cell aggregate (a central angle of an arc when the shape of the candidate region is approximated by an arc). It is assumed that a straight line R2 and a straight line R4 passing through a center CTR of the reference ellipse RE indicate a range where the candidate region CR1 is present. In this case, the length of the candidate region CR1 may be represented by an angle β between the straight line R2 and a straight line R4, which is a range in which the candidate region is present when viewed from the center CTR of the reference ellipse RE. The angle β corresponds to a central angle when the shape of the candidate region CR1 (or the curved line CL1) is considered as an elliptical arc.

The width of the candidate region at a specific position is the width of the curve (the curve CL1 for the candidate region CR1) approximating the candidate region at the specific position (width of the candidate region at which the straight line orthogonal to the tangent of the curve at the position overlaps the candidate region). Here, the specific position of the candidate region can be represented by an angle α as the position of the intersection of the candidate region and the straight line R1 rotated, for example, leftward by a predetermined angle α from the straight line R3 extending horizontally rightward from the center of the cell aggregate, that is, the center of the reference ellipse RE, as a reference of an angle of 0 degrees. In FIG. 55, the candidate region CR1 has a width W at the position of the candidate region of the angle α.

(4) The region portion evaluation unit 114A expresses a specific position of the candidate region along the direction in which the candidate region extends by a counterclockwise angle based on the center of the cell aggregate, that is, a straight line extending to the right in the horizontal direction from the center of the reference ellipse approximating the shape of the cell aggregate as a reference of an angle of 0 degrees, and obtains the width of the candidate region at the specific position from 0 degrees to 360 degrees in the counterclockwise direction by incrementing the angle α by a predetermined angle such as 1 degree. When the width is measured by incrementing the angle by 1 degree, the number of measurement positions is 360 degrees/1 degree = 360.

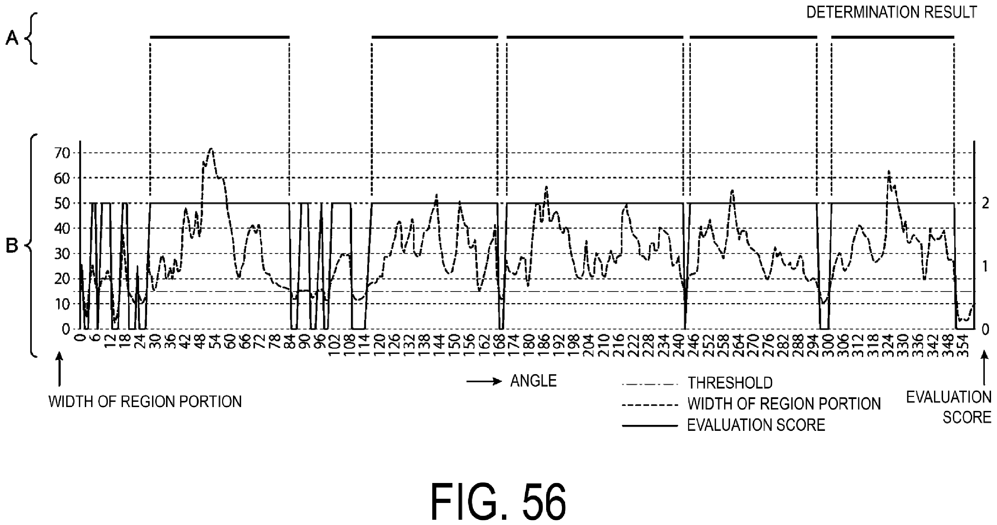

(5) The region portion evaluation unit 114A evaluates a region portion in which an angle of a region having a width of 15 pixels or more of the candidate region is continuous once as an evaluation score of level 1 and a region portion in which an angle of a region having a width of 15 pixels or more of the candidate region is continuous by 2 degrees or more as an evaluation score of level 2. This is because the width of the candidate region needs to be wide to some extent in order for the candidate region to be included in the normal retinal tissue region, and thus, the width of 15 pixels or more in the inverted observation image is a necessary condition, and therefore, an evaluation score of level 1 is given to such a region portion. In addition, as the length of the region portion satisfying such a width condition is longer, the region portion of the region candidate is more likely to be included in the normal retinal tissue region, and thus, a higher evaluation score is given to the case where the region portion satisfying the condition that the width is 15 pixels or more is continuously present by 2 degrees or more, and the evaluation score is set to level 2. As described above, the criterion for determining the width of the ONbL in the epithelial structure of the retinal tissue can be the width of 0.03 mm, and therefore, it was used as the criterion for evaluating the width. In the observation with an inverted microscope of the magnification in this example, the actual size of the target region corresponding to one pixel was 2.07 µm square. Thus, 0.03 mm as the determination criterion corresponds to approximately 15 pixels. Then, the region portion evaluation unit 114A measured the width of the candidate region along the outer periphery to obtain an evaluation score. The obtained evaluation scores are shown in a line graph in a region indicated by B in FIG. 56. In the graph of the region indicated by B in FIG. 56, the horizontal axis represents an angle of the measured region portion as viewed from the center of the cell aggregate, the angle being incremented by 1 degree in a range of 360 degrees counterclockwise with a straight line extending rightward in the horizontal direction from the center of the cell aggregate as a reference of an angle of 0 degree. The vertical axis of the region illustrated in B of FIG. 56 represents the width of the region portion and the evaluation score, the scale of the width of the region portion is illustrated on the left end, and the scale of the evaluation score is illustrated on the right end. The width of the region portion is the width of a portion where a straight line passing through the center of the cell aggregate and the region portion overlap each other, and is indicated by a broken line in the graph. The evaluation score is indicated by a solid line graph that takes any value of 0, 1, and 2 in the graph. The graph shown here may be displayed on a display of the operation terminal 220 so that the operator can confirm the graph.

### Continuous Length-Based Normal Region Determination Stage

When the region portion of the candidate region in which the evaluation score of the inverted observation image has a value equal to or more than each normal region determination reference value is continuously present by a length equal to or more than a predetermined normal region determination reference length along the curve representing the extending direction of the region portion, the continuous length-based normal region determination unit 114B determines the candidate region as the normal retinal tissue region (step S152A). That is, in this stage, to obtain a determination result by further narrowing down the candidate region determined to have an evaluation score equal to or more than the normal region determination reference value to a region having a higher possibility of including normal retinal tissue, whether such a candidate region is present by a length equal to or more than a predetermined normal region determination reference length is determined. This stage is specifically performed by executing the following procedure (6).

(6) When the candidate region inside the cell aggregate in the inverted observation image has a continuous region portion with a width of 15 pixels or more and an arc length corresponding to an angle of 45 degrees or more as viewed from the center of the reference ellipse approximating the cell aggregate (the central angle in the case of approximating the shape of the region portion with an arc is 45 degrees or more), the candidate region can be determined as a normal retinal tissue region having a sufficient size with a length (the length of the outer periphery of the candidate region along the contour of the cell aggregate) of about 0.4 mm or more in actual measurement in the cell aggregate having a diameter of 1 mm or more. Thus, the length was set as a normal region determination reference length, and the cell aggregate having a candidate region with a length equal to or more than the normal region determination reference length was determined to include neural retina (normal retinal tissue region). According to such criteria, the continuous length-based normal region determination unit 114B determines whether the region portion of the candidate region having an evaluation score of 2 or more has a length corresponding to an angle of 45 degrees, which is a predetermined normal region determination reference length, continuously along the curve representing the direction in which the region portion extends, and determines the candidate region satisfying the criteria as a normal retinal tissue region. In the region indicated by A in FIG. 56, as a determination result, the range of angles in which the region portion of the candidate region of the arc length corresponding to the width of 15 pixels or more and the continuous length of the central angle of 45 degrees or more is present is indicated by a solid line. A solid line of the determination result of the candidate region of the region indicated by A in FIG. 56 and a broken line of the corresponding position of the graph of the evaluation score in the region indicated by B in FIG. 56 are illustrated. It can be confirmed that the region portions where the determination results that the normal retinal tissue region satisfying the criteria are obtained are present at five positions corresponding to angles of about 28 degrees to 84 degrees, about 117 degrees to 167 degrees, about 172 degrees to 242 degrees, about 246 degrees to 296 degrees, and about 302 degrees to 351 degrees. A cell aggregate having one or more region portions of the candidate regions determined as the normal retinal tissue regions is determined as qualitatively acceptable. The determination result is stored in a data storage area in the memory 120 together with information for specifying the cell aggregate to be determined, so that the determination result can be referred to later. In this case, the angle at which the region portion for which the determination result that the criterion is satisfied is obtained is present can be stored as information for specifying the position of the normal retinal tissue region.

The results of applying the above stages to 12 cell aggregates from sample SPL20 to sample SPL31 and making a determination are shown in Table 1. In the determination result, eight of the twelve samples were determined to have one or more normal retinal tissue regions and the quality was determined to be "acceptable", and four of the twelve samples were determined not to have a normal retinal tissue region and the quality was determined to be "unacceptable".

**[Table 1]**

| Cell aggregate sample | Inverted observation image analysis | |
|---|---|---|
| | Width 15 pixels or more | Acceptable/Unacceptable |
| | Number of region portions having angle of 45 degrees or more | |
| SPL20 | 1 | Acceptable |
| SPL21 | 5 | Acceptable |
| SPL22 | 1 | Acceptable |
| SPL23 | 1 | Acceptable |
| SPL24 | 2 | Acceptable |
| SPL25 | 1 | Acceptable |
| SPL26 | 0 | Unacceptable |
| SPL27 | 0 | Unacceptable |
| SPL28 | 0 | Unacceptable |
| SPL29 | 2 | Acceptable |
| SPL30 | 1 | Acceptable |
| SPL31 | 0 | Unacceptable |

Here, to verify the validity of the determination result through the automated determination shown in Table 1, the determination result was compared with the result of determining whether the cell aggregate has a normal retinal tissue region through visual inverted observation. As a result, a sample having one or more candidate regions having an arc length corresponding to a central angle of 45 degrees or more with a width of 15 pixels was determined to have "acceptable" quality as in the automated determination, and when there were no such candidate regions, the quality was determined to be "unacceptable". In this manner, the results of the automated determination shown in Table 1 strongly correlated with the actual visual determination, and it was confirmed that the determination criterion of the candidate region having an arc length corresponding to a central angle of 45 degrees or more with a width of 15 pixels was appropriate as a determination criterion of the cell aggregate having a normal retinal tissue region. It was confirmed that the suitability of the cell aggregate can be determined from the inverted observation image by applying the automated determination in this manner. Thus, it was confirmed that the normal retinal tissue region can be precisely determined through the automated determination. However, the upright observation image is not taken into consideration in Example 1, and thus, there is a possibility that the state of the surface on the back side of the surface of the cell aggregate observed in the inverted observation is not reflected in the determination result.

### <Example 2: Application of Machine Determination to Upright Observation Image of Cell Aggregate>

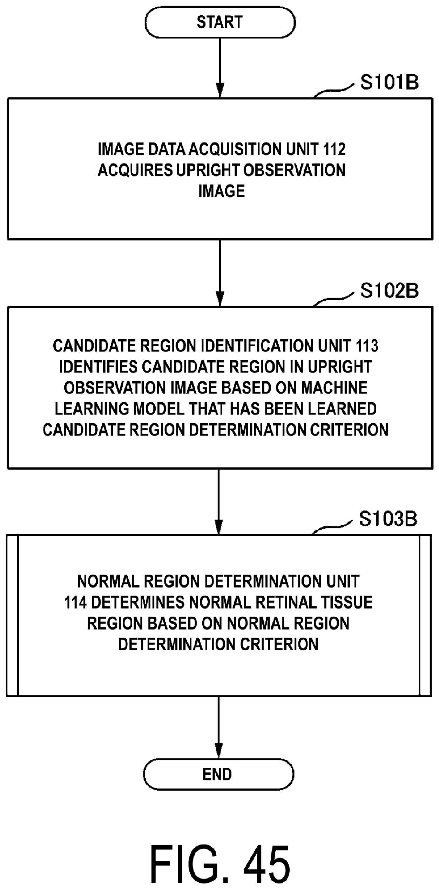

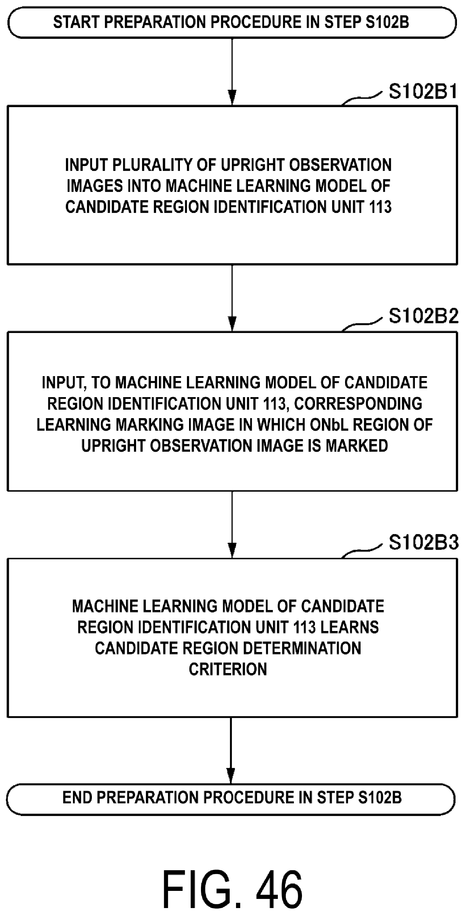

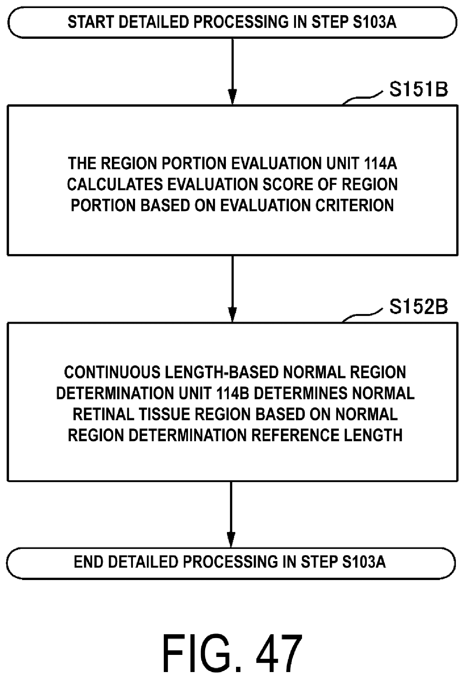

Next, whether the automated determination is applicable to an upright observation image was studied. An overall operation flow is shown in FIG. 45, an operation flow for constructing a machine learning model is shown in FIG. 46, and a detailed operation flow related to normal region determination is shown in FIG. 47. The following stages were performed using sample SPL20 to sample SPL31 as cell aggregates to be determined.

### [1] Image Data Acquisition Stage

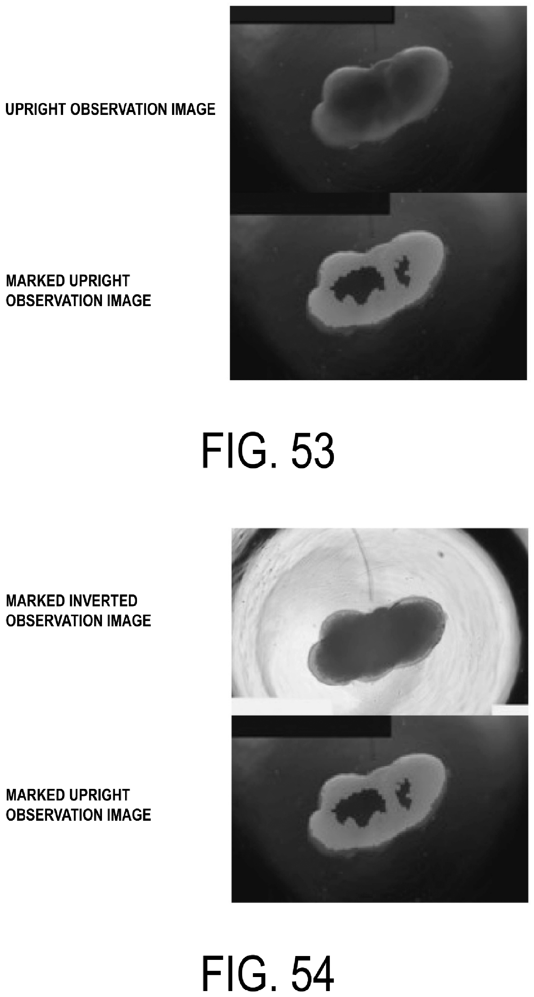

First, a vessel holding cell aggregates to be determined is set in the upright microscope 200, and the cell aggregates are photographed using the upright microscope 200. As the upright microscope, AxioZoom V16 manufactured by Carl Zeiss was used. The image data acquisition unit 112 acquires the image data of the upright observation image acquired thereby from the upright microscope 200 via the LAN (step S101B). Specifically, as in step S101A of Example 1, the operator observes the bright-field image (zoom magnification: 33.3 times) of the cell aggregate to be determined held in the culture solution in an appropriate vessel by using the upright microscope 200, photographs an image, and acquires an upright observation image. The upright microscope 200 transmits image data representing the upright observation image to the normal cell aggregate determination server 110 via a LAN, and the image data acquisition unit 112 acquires the image data and stores the image data in the data storage area in the memory 120. The upper side of FIG. 53 shows the acquired upright observation image of the cell aggregate to be determined.

Next, analysis of the upright observation image, which will be described in detail below, is performed on the acquired upright observation image, and an analysis result of the upright observation image is acquired. In the procedures (1) to (6) described later, image processing and image analysis processing were performed using a software construction tool Pipeline Pilot Version 22.1.0 of Dassault System as a part of the normal cell aggregate determination program 120A.

### [2] Candidate Region Identification Stage

The candidate region identification unit 113 identifies a region determined to satisfy the candidate region determination criterion as a candidate region included in a region that is a candidate of a normal retinal tissue region, based on the candidate region determination criterion regarding color, in the image region of the cell aggregate in the upright and inverted observation images (step S102B). The purpose of this stage is the same as that of step S102A of Example 1, but in the upright observation image, the state indicated by the image such as brightness and contrast is greatly different because of the difference in photographing conditions as compared with the inverted observation image, and a favorable result cannot be obtained in the procedure using the same determination criterion as that of Example 1. However, it was confirmed that a favorable result was obtained by performing discrimination through machine learning in the upright observation image. Thus, to automatically extract the ONbL region, which is a bright arc-shaped region, in the cell aggregate, machine learning is used so that analysis and image processing can be performed even on an upright observation image in which the color tone is not clear. Through this stage, an image region corresponding to the cell aggregate is specified, and a candidate region is specified therein based on a machine learning model that has learned a candidate region determination criterion regarding color. Here, brightness is a target of learning of the machine learning model. As in Example 1, the candidate region may be a region of the ONbL. It is also possible to identify the candidate region by specifying an image region of a cell aggregate and excluding other image regions after performing identification processing based on the machine learning model that has learned the candidate region determination criterion regarding color first. Specifically, this candidate region identification stage can be performed by executing a preparation procedure shown in (1) below and procedures of (2) to (3) shown below.
(1) As the preparation processing of step S102B, a machine learning model for the image identification processing is constructed. Prior to this, learning data for causing the machine learning model to learn was prepared. The learning data is a plurality of upright observation images and a learning marking image corresponding to each of the plurality of upright observation images. Here, three images were prepared for each. One upright observation image prepared as learning data is illustrated on the upper side of FIG. 52. A learning marking image corresponding to the upright observation image is illustrated on the lower side of FIG. 52. The marking image for learning is marked by visually checking the upright observation image and painting out the ONbL region, which is a bright arc-shaped portion, with white to maximize the brightness so that the region can be clearly distinguished. That is, the region of the ONbL is identified and marked by brightness. Here, when the bright arc-shaped portion does not have a certain width, not only the brightness but also information based on the morphology can be included in the learning marking image by not marking the portion. In the bright ONbL region in the upright observation image, the change in brightness in the width direction is ambiguous, and this can be approximated as following a Gaussian distribution. Thus, as the machine learning model, an image identification model based on Gaussian was used in consideration of the characteristics of the color distribution of the upright observation image. The image identification based on Gaussian is a method of learning a distribution of color tones for a region of a certain size included in an image and searching for a target region and a region in which a distribution of colors approximates a modeled Gaussian distribution from the image on the premise that a change in colors follows the Gaussian distribution. It is possible to select another machine learning model related to an appropriate image analysis method in accordance with the characteristics of the image.
   An operation flow for constructing the machine learning model is illustrated in FIG. 46. To cause the machine learning model to learn, a plurality of sets of the upright observation images and the learning marking images are sequentially input to the machine learning model. One upright observation image is input to the machine learning model of the candidate region identification unit 113 (step S102B1). Next, the corresponding learning marking image in which the ONbL region of the upright observation image is marked is input to the machine learning model of the candidate region identification unit 113 (step S102B2). As a result, the machine learning model of the candidate region identification unit 113 compares the characteristic based on the distribution of the brightness of the region to be determined as the candidate region corresponding to the ONbL region in the upright observation image with the characteristic of the distribution of the brightness of the region indicated as the ONbL region by the marking in the learning marking image, thereby constructing the machine learning model for identifying the candidate region from the upright observation image (step S102B3). The determination mechanism realized by the learned machine learning model corresponds to a candidate region determination criterion. Once the machine learning model is constructed, the procedure of constructing the machine learning model is not necessary thereafter.
(2) The candidate region identification unit 113 identifies a region that can be a candidate region that is likely to be determined as a region included in the ONbL using the machine learning model with respect to the upright observation image of the cell aggregate to be determined, and marks the region as a continuous region. Through this procedure, the candidate region identification unit 113 extracts a region with a large change in gradation as a circumferential contour of the cell aggregate, identifies a relatively bright region in which the brightness changes in the width direction like a Gaussian distribution inside the contour as a region that can be a candidate region, and identifies and marks the regions as continuous regions.
(3) The candidate region identification unit 113 removes, as noise, a region marked as a region that can be a candidate region in contact with the outermost side, that is, four sides of the entire image through a known image processing technique, and removes the marking. These procedures identify candidate regions within the image region of the cell aggregate. On the lower side of FIG. 53, an image in which the candidate region is marked slightly brightly with respect to the upright observation image of the cell aggregate to be determined is shown. Here, it is preferable to create an image in which the candidate region is overlaid on the original inverted observation image, and store the image data in the data storage area in the memory 120 as an output image for visual confirmation.

### [3] Normal Region Determination Stage

Next, the normal region determination unit 114 determines, in the candidate region of the upright observation image, the candidate region of the identification target determined to satisfy the normal region determination criterion of the upright observation image as a normal retinal tissue region based on the normal region determination criterion regarding the brightness and/or the morphology of each of the upright observation images (step S103B). A cell aggregate having such a normal retinal tissue region is determined as qualitatively acceptable. This normal region determination stage is executed by stages from [3A] to [3B], which will be described in detail below. The normal region determination will be described with reference to the detailed operation flow illustrated in FIG. 47.

### Region Portion Evaluation Stage

The region portion evaluation unit 114A obtains an evaluation score of each of region portions present in a range obtained by dividing a candidate region into a plurality of regions along a curve representing an extending direction of the candidate region based on evaluation criteria regarding color and/or morphology corresponding to an observation method called upright observation with respect to the candidate region identified in the image region of the cell aggregate of the upright observation image (step S151B). In the region portion evaluation stage, to further narrow down the candidate region to a region having a higher possibility of including normal retinal tissue, each evaluation score is obtained in each region portion present in a range obtained by dividing the candidate region into a plurality of regions. This stage is specifically performed by executing the following procedures (4) to (5).

(4) The region portion evaluation unit 114A expresses a specific position of the candidate region along the direction in which the candidate region extends by a counterclockwise angle based on the center of the cell aggregate, that is, a straight line extending to the right in the horizontal direction from the center of the reference ellipse approximating the shape of the cell aggregate as a reference of an angle of 0 degrees, and obtains the width of the candidate region at the specific position from 0 degrees to 360 degrees in the counterclockwise direction by incrementing the angle α by a predetermined angle such as 1 degree.

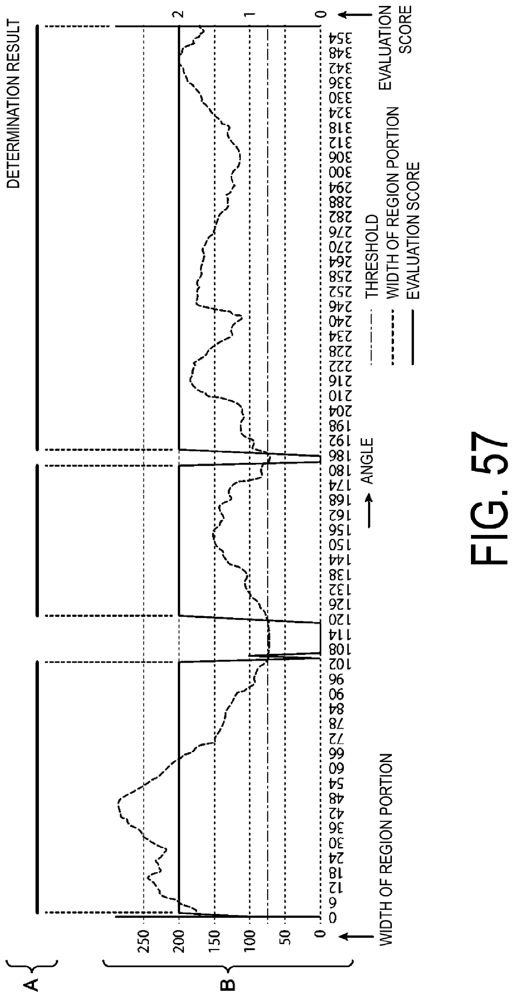

(5) The region portion evaluation unit 114A evaluates a region portion in which the region portion having a width of 75 pixels or more of the candidate region is continuous by 1 degree as an evaluation score of level 1 and a region portion in which an angle of a region having a width of 75 pixels or more is further continuous by 2 degrees or more as an evaluation score of level 2. This is because the width of the candidate region needs to be wide to some extent in order for the candidate region to be included in the normal retinal tissue region. It is confirmed by actual upright observation that the width is about 0.15 mm, which corresponds to 75 pixels in an upright observation image. Thus, the necessary condition is that the width is 75 pixels or more, which is the reference width corresponding to the observation method of the upright observation, and therefore, an evaluation score of level 1 is given to such a region portion. The determination criterion of 75 pixels is a value larger than the determination criterion of 15 pixels in the inverted observation image, but such a difference is caused by a low contrast in the upright observation image, a bright ONbL region being seen to be widened, and the like. In addition, as the length of the region portion satisfying such a width condition is longer, the region portion of the region candidate is more likely to be included in the normal retinal tissue region, and thus, a higher evaluation score is given to the case where the region portion satisfying the condition that the width is 75 pixels or more is continuously present by 2 degrees or more, and the evaluation score is set to level 2. The reference width corresponding to the observation method (upright observation) in Example 2 was about 75 pixels. Then, the region portion evaluation unit 114A measured the width of the candidate region along the outer periphery to obtain an evaluation score. The obtained evaluation scores are shown in a line graph in a region indicated by B in FIG. 57. In the graph of the region indicated by B in FIG. 57, the horizontal axis represents an angle of the measured region portion as viewed from the center of the cell aggregate, the angle being incremented by 1 degree in a range of 360 degrees counterclockwise with a straight line extending rightward in the horizontal direction from the center of the cell aggregate as a reference of an angle of 0 degree. The vertical axis of the region illustrated in B of FIG. 57 represents the width of the region portion and the evaluation score, the scale of the width of the region portion is illustrated on the left end, and the scale of the evaluation score is illustrated on the right end. The width of the region portion is the width of a portion where a straight line passing through the center of the cell aggregate and the region portion overlap each other, and is indicated by a broken line in the graph. The evaluation score is indicated by a solid line graph that takes any value of 0, 1, and 2 in the graph. The graph shown here may be displayed on a display of the operation terminal 220 so that the operator can confirm the graph.

### Continuous Length-Based Normal Region Determination Stage

When the region portion of the candidate region in which the evaluation score of the upright observation image has a value equal to or more than each normal region determination reference value is continuously present by a length equal to or more than a predetermined normal region determination reference length along the curve representing the extending direction of the region portion, the continuous length-based normal region determination unit 114B determines the candidate region as the normal retinal tissue region (step S152B). That is, in this stage, to obtain a determination result by further narrowing down the candidate region determined to have an evaluation score equal to or more than the normal region determination reference value to a region having a higher possibility of including normal retinal tissue, whether such a candidate region is present by a length equal to or more than a predetermined normal region determination reference length is determined. This stage is specifically performed by executing the following procedure (6).

(6) When the candidate region inside the cell aggregate in the upright observation image has, in the cell aggregate having a diameter of about 1 mm or more, a continuous region portion with a width of 75 pixels or more and an arc length corresponding to an angle of 45 degrees or more as viewed from the center of the reference ellipse approximating the cell aggregate (the central angle in the case of approximating the shape of the region portion with an arc is 45 degrees or more), the candidate region can be determined to be a normal retinal tissue region having a sufficient size with a length of 0.4 mm or more in actual measurement. Thus, the length was set as a normal region determination reference length, and the cell aggregate having a candidate region with a length equal to or more than the normal region determination reference length was determined to include neural retina (normal retinal tissue region). According to such criteria, the continuous length-based normal region determination unit 114B determines whether the region portion of the candidate region having an evaluation score of 2 or more has a length corresponding to an angle of 45 degrees, which is a predetermined normal region determination reference length, continuously along the curve representing the direction in which the region portion extends, and determines the candidate region satisfying the criteria as a normal retinal tissue region. In the region indicated by A in FIG. 57, as a determination result, the range of angles in which the region portion of the candidate region of the arc length corresponding to the width of 75 pixels or more and the continuous length of the central angle of 45 degrees or more is present is indicated by a solid line. A solid line of the determination result of the candidate region of the region indicated by A in FIG. 57 and a broken line of the corresponding position of the graph of the evaluation score in the region indicated by B in FIG. 57 are illustrated. It can be confirmed that the region portions for which the determination result indicating that the region is a normal retinal tissue region satisfying the criteria are present at three positions corresponding to angles of about 2 degrees to 102 degrees, about 121 degrees to 182 degrees, and about 189 degrees to 356 degrees. A cell aggregate having a region portion of the candidate region determined as these normal retinal tissue regions is determined as having a normal retinal tissue region and as qualitatively acceptable. The determination result is stored in a data storage area in the memory 120 together with information for specifying the cell aggregate to be determined, so that the determination result can be referred to later. In this case, the angle at which the region portion for which the determination result that the criterion is satisfied is obtained is present can be stored as information for specifying the position of the normal retinal tissue region.

The results of applying the above stages to 12 cell aggregates from sample SPL20 to sample SPL31 and making a determination are shown in Table 2. In the determination result, twelve of the twelve samples were determined to have one or more normal retinal tissue regions and the quality was determined to be "acceptable", and there were no samples determined not to have a normal retinal tissue region and the quality was determined to be "unacceptable".

**[Table 2]**

| Cell aggregate sample | Upright observation image analysis | |
|---|---|---|
| | Width 75 pixels or more | Acceptable/Unacceptable |
| | Number of region portions having angle of 45 degrees or more | |
| SPL20 | 2 | Acceptable |
| SPL21 | 3 | Acceptable |
| SPL22 | 3 | Acceptable |
| SPL23 | 1 | Acceptable |
| SPL24 | 2 | Acceptable |
| SPL25 | 2 | Acceptable |
| SPL26 | 1 | Acceptable |
| SPL27 | 2 | Acceptable |
| SPL28 | 2 | Acceptable |
| SPL29 | 2 | Acceptable |
| SPL30 | 3 | Acceptable |
| SPL31 | 1 | Acceptable |

It was confirmed that the tendency of the number of samples whose quality was determined as "acceptable" in Table 2 correlated with the determination result of the inverted observation image of Example 1 shown in Table 1. That is, the samples whose quality was determined as "acceptable" in the determination result of the inverted observation image of Example 1 shown in Table 1 were all determined as "acceptable" in the quality in Example 2 shown in Table 2. It was confirmed that the suitability of the cell aggregate can be determined from the upright observation image by applying the automated determination in this manner. However, the inverted observation image is not taken into consideration in Example 2, and thus, there is a possibility that the state of the surface on the back side of the surface of the cell aggregate observed in the upright observation is not reflected in the determination result.

### <Example 3: Determination through Combination of Individual Determination Results of Upright Observation Image and Inverted Observation Image of Cell Aggregate>

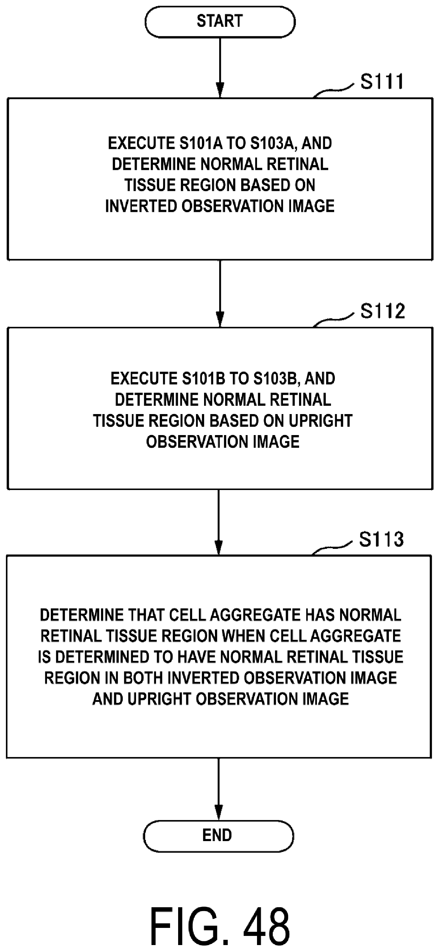

It is considered that more accurate determination can be made by combining the determination result based on the inverted observation image according to Example 1 and the determination result based on the upright observation image according to Example 2. In Example 3, when it is determined that the cell aggregate to be determined has a normal retinal tissue region in both the determination result based on the inverted observation image according to Example 1 and the determination result based on the upright observation image according to Example 2, it is finally determined that the cell aggregate to be determined has a normal retinal tissue region. The overall operation flow of Example 3 is shown in FIG. 48. The following stages were performed using sample SPL20 to sample SPL31 as cell aggregates to be determined.

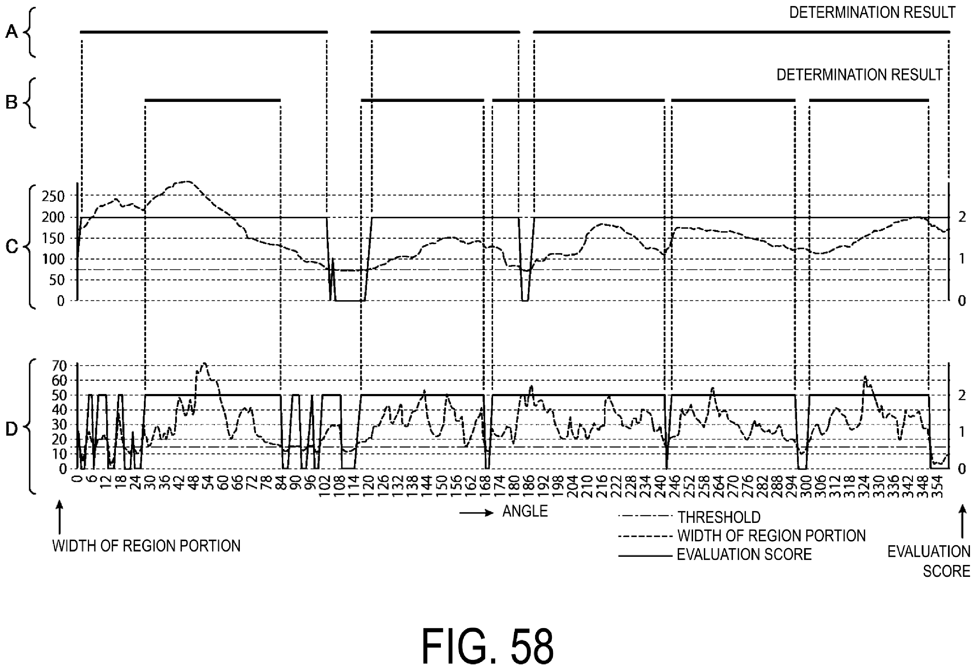

Steps S101A to S103A of Example 1 were executed, and whether the sample of cell aggregate had a normal retinal tissue region was determined based on the inverted observation image (step S111). A line graph of the evaluation scores obtained in step S103A is illustrated in a region indicated by D in FIG. 58, and the determination results of the candidate regions obtained in step S103A are illustrated in a region indicated by B in FIG. 58. A solid line of the determination result of the candidate region of the region indicated by B in FIG. 58 and a broken line of the corresponding position of the graph of the evaluation score in the region indicated by D in FIG. 58 are illustrated.

Steps S101B to S103B of Example 2 were executed, and whether the sample of cell aggregate had a normal retinal tissue region was determined based on the upright observation image (step S112). A line graph of the evaluation scores obtained in step S103B is illustrated in a region indicated by C in FIG. 58, and the determination results of the candidate regions obtained in step S103B are illustrated in a region indicated by A in FIG. 58. A solid line of the determination result of the candidate region of the region indicated by A in FIG. 58 and a broken line of the corresponding position of the graph of the evaluation score in the region indicated by C in FIG. 58 are illustrated.

When it is determined that the cell aggregate to be determined has a normal retinal tissue region in both the determination result with the inverted observation image obtained in steps S101A to S103A of Example 1 and the determination result with the upright observation image obtained in steps S101B to S103B of Example 2, it is finally determined that the cell aggregate to be determined has a normal retinal tissue region (step S113). The cell aggregate having the region portion of the candidate region determined as the normal retinal tissue region is determined as qualitatively acceptable. The determination result is stored in a data storage area in the memory 120 together with information for specifying the cell aggregate to be determined, so that the determination result can be referred to later. In this case, the angle at which the region portion for which the determination result that the criterion is satisfied is obtained is present can be stored as information for specifying the position of the normal retinal tissue region.

The results of applying the above stages to 12 cell aggregates from sample SPL20 to sample SPL31 and making a determination are shown in Table 3. In the determination result, eight of the twelve samples were determined to have one or more normal retinal tissue regions and the quality was determined to be "acceptable", and four of the twelve samples were determined not to have a normal retinal tissue region and the quality was determined to be "unacceptable".

**[Table 3]**

| Cell aggregate sample | Upright observation image | Inverted observation image | Determination through combination of upright and inverted observation images |
|---|---|---|---|
| SPL20 | Acceptable | Acceptable | Acceptable |
| SPL21 | Acceptable | Acceptable | Acceptable |
| SPL22 | Acceptable | Acceptable | Acceptable |
| SPL23 | Acceptable | Acceptable | Acceptable |
| SPL24 | Acceptable | Acceptable | Acceptable |
| SPL25 | Acceptable | Acceptable | Acceptable |
| SPL26 | Acceptable | Unacceptable | Unacceptable |
| SPL27 | Acceptable | Unacceptable | Unacceptable |
| SPL28 | Acceptable | Unacceptable | Unacceptable |
| SPL29 | Acceptable | Acceptable | Acceptable |
| SPL30 | Acceptable | Acceptable | Acceptable |
| SPL31 | Acceptable | Unacceptable | Unacceptable |

The determination result of the combination of the upright observation image and the inverted observation image in Example 3 shown in Table 3 was the same as the determination result of the inverted observation image in Example 1. However, in Example 3, when the quality is not "acceptable" in the determination results of both the upright observation image and the inverted observation image, the quality is not finally determined to be "acceptable". Thus, it is considered that the determination can be made more severe than the determination with any single observation image. That is, in Example 3, it is possible to more strictly determine that the quality is "unacceptable". In this manner, by combining individual determination results from the upright observation image and the inverted observation image, it is possible to determine whether the cell aggregate has a normal retinal tissue region with higher accuracy than the determination with any one of the observation images alone.

### <Example 4: Simultaneous Determination of Upright Observation Image And Inverted Observation Image of Cell Aggregate>

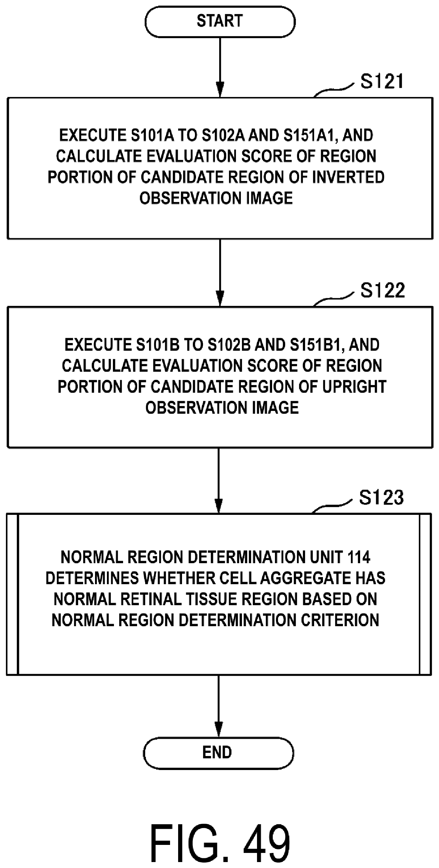

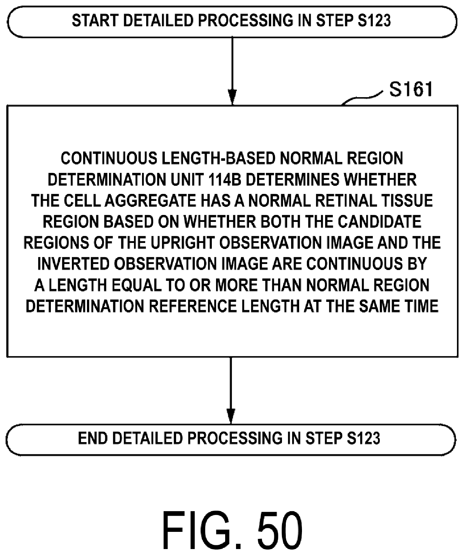

In Example 3, the determination is performed by combining the determination results obtained by individually analyzing the upright observation image and the inverted observation image. However, it is considered that the determination can be made with higher accuracy by simultaneously performing the determination of both the upright observation image and the inverted observation image as much as possible. In Example 4, after the analysis with the inverted observation image according to Example 1 and the analysis of the upright observation image according to Example 2 are individually performed up to the stage of obtaining the evaluation score of the candidate region, in the determination of whether the cell aggregate has a normal retinal tissue region, whether the cell aggregate to be determined has a normal retinal tissue region is determined by simultaneously considering the evaluation scores of the region portions of the respective candidate regions from the inverted observation image and the upright observation image. FIG. 49 shows an overall operation flow of Example 3, and FIG. 50 shows a detailed operation flow relating to normal region determination. The following stages were performed using sample SPL20 to sample SPL31 as cell aggregates to be determined.

Steps S101A to S102A of Example 1 are executed to acquire an image in which the candidate region is marked on the inverted observation image of the cell aggregate to be determined, and step S151A1 of Example 1 is executed to calculate the evaluation score of the region portion of the candidate region based on the evaluation criteria corresponding to the observation method of the inverted observation (step S121). The upper side of FIG. 54 shows an image in which the candidate region of the inverted observation image acquired in step S102A is marked.

Steps S101B to S102B of Example 2 are executed to acquire an image in which the candidate region is marked on the upright observation image of the cell aggregate to be determined, and step S151B1 of Example 2 is executed to calculate the evaluation score of the region portion of the candidate region based on the evaluation criteria corresponding to the observation method of the upright observation (step S122). The lower side of FIG. 54 shows an image in which the candidate region of the upright observation image acquired in step S102B is marked.

When the region portions of the candidate region in which both the evaluation score of the region portion of the candidate region of the inverted observation image and the evaluation score of the region portion of the candidate region of the upright observation image have values equal to or more than the normal region determination reference value at the same time are continuously present along the curve representing the direction in which the region portions extend by a length equal to or more than a predetermined normal region determination reference length, the continuous length-based normal region determination unit 114B determines the candidate region as a normal retinal tissue region (step S161). This stage is specifically performed by executing the following procedure (1).
(1) The continuous length-based normal region determination unit 114B determines whether both of the region portion of the candidate region having the evaluation score of 2 in the inverted observation image (that is, the region portion of the candidate region having the width of 15 pixels or more and being continuously present with the arc length corresponding to the central angle of 2 degrees or more) and the region portion of the candidate region having the evaluation score of 2 in the upright observation image (that is, the region portion of the candidate region having the width of 75 pixels or more and being continuously present with the arc length corresponding to the central angle of 2 degrees or more) have the arc length corresponding to the angle of 45 degrees which is the predetermined normal region determination reference length continuously along the curve representing the extending direction, and determines the candidate region satisfying the reference as the normal retinal tissue region. The cell aggregate having the region portion of the candidate region determined as the normal retinal tissue region is determined as qualitatively acceptable.

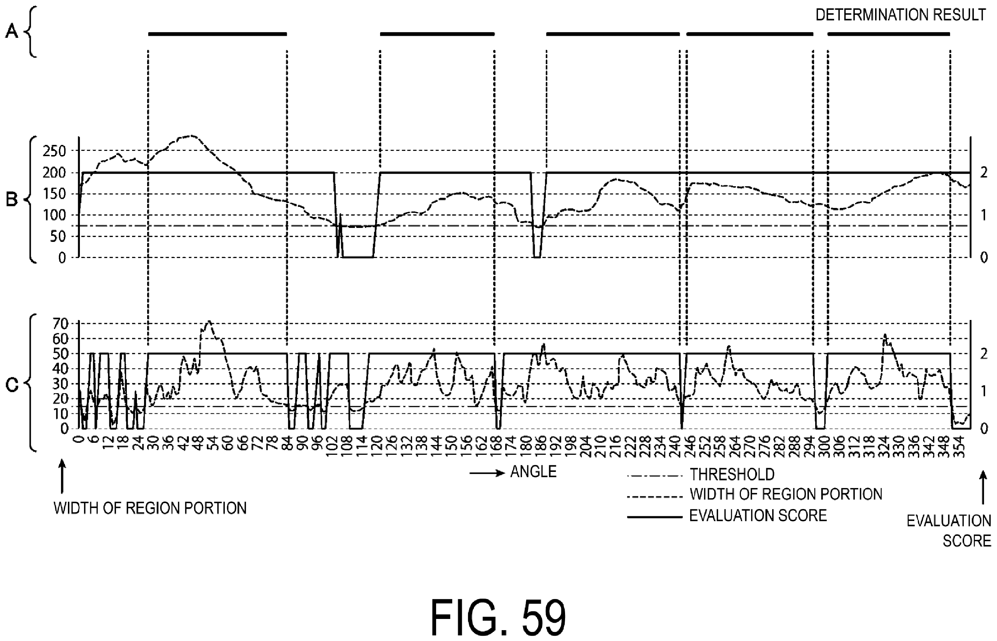

In the graph of the region indicated by C in FIG. 59, the horizontal axis is an angle obtained by incrementing the straight line extending horizontally rightward from the center of the cell aggregate (the center of the reference ellipse approximating the shape thereof) in the region portion of the cell aggregate of the measured inverted observation image by 1 degree in the range of 360 degrees in the counterclockwise direction as a reference of the angle of 0 degrees. The vertical axis of the region illustrated in C of FIG. 59 represents the width of the region portion and the evaluation score, the scale of the width of the region portion is illustrated on the left end, and the scale of the evaluation score is illustrated on the right end. The width of the region portion is the width of a portion where a straight line passing through the center of the cell aggregate and the region portion overlap each other, and is indicated by a broken line in the graph. The evaluation score based on the continuous length represented by the angle of the region portion having a width of 15 pixels or more (the evaluation score is 1 when the angle is 1 degree, and the evaluation score is 2 when the angle is 2 degrees or more) is indicated by a solid line.

In the graph of the region indicated by B in FIG. 59, the horizontal axis is an angle obtained by incrementing the straight line extending horizontally rightward from the center of the cell aggregate (the center of the reference ellipse approximating the shape thereof) in the region portion of the cell aggregate of the measured upright observation image by 1 degree in the range of 360 degrees in the counterclockwise direction as a reference of the angle of 0 degrees. The vertical axis of the region illustrated in B of FIG. 59 represents the width of the region portion and the evaluation score, the scale of the width of the region portion is illustrated on the left end, and the scale of the evaluation score is illustrated on the right end. The width of the region portion is the width of a portion where a straight line passing through the center of the cell aggregate and the region portion overlap each other, and is indicated by a broken line in the graph. The evaluation score based on the continuous length represented by the angle of the region portion having a width of 75 pixels or more (the evaluation score is 1 when the angle is 1 degree, and the evaluation score is 2 when the angle is 2 degrees or more) is indicated by a solid line.

In the region indicated by A in FIG. 59, as a determination result, such an angle range is indicated by a solid line, in which there is a region portion of an arc length candidate region corresponding to a width of 15 pixels or more and a continuous length of 45 degrees or more at the central angle in the inverted observation image, and there is a region portion of an arc length candidate region corresponding to a width of 75 pixels or more and a continuous length of 45 degrees or more at the central angle in the upright observation image. A solid line of the determination result of the candidate region of the region indicated by A in FIG. 59 and a broken line of the corresponding position of the graph of the evaluation score in the region indicated by B in FIG. 59 and the region indicated by C in FIG. 59 are illustrated. It can be confirmed that the region portions where the determination results that the normal retinal tissue region satisfying the criteria are obtained are present at five positions corresponding to angles of about 28 degrees to 84 degrees, about 121 degrees to 167 degrees, about 189 degrees to 242 degrees, about 246 degrees to 296 degrees, and about 302 degrees to 351 degrees. Thus, the cell aggregate having the region portions of the candidate regions determined as these normal retinal tissue regions are determined as having normal retinal tissue regions and qualitatively acceptable. The determination result is stored in a data storage area in the memory 120 together with information for specifying the cell aggregate to be determined, so that the determination result can be referred to later. In this case, the angle at which the region portion for which the determination result that the criterion is satisfied is obtained is present can be stored as information for specifying the position of the normal retinal tissue region.

The results of applying the above stages to 12 cell aggregates from sample SPL20 to sample SPL31 and making a determination are shown in Table 4. In the determination result, seven of the twelve samples were determined to have one or more normal retinal tissue regions and the quality was determined to be "acceptable", and five of the twelve samples were determined not to have a normal retinal tissue region and the quality was determined to be "unacceptable".

**[Table 4]**

| Cell aggregate sample | Width 75 pixels (upright) width 15 pixels (inverted) or more Number of region portions having angle of 45 degrees or more | | | Acceptable/ Unacceptable |
|---|---|---|---|---|
| | Upright | Inverted | Simultaneous determination of upright and inverted observation images | |
| SPL20 | 2 | 1 | 1 | Acceptable |
| SPL21 | 3 | 5 | 5 | Acceptable |
| SPL22 | 3 | 1 | 1 | Acceptable |
| SPL23 | 1 | 1 | 1 | Acceptable |
| SPL24 | 2 | 2 | 2 | Acceptable |
| SPL25 | 2 | 1 | 0 | Unacceptable |
| SPL26 | 1 | 0 | 0 | Unacceptable |
| SPL27 | 2 | 0 | 0 | Unacceptable |
| SPL28 | 2 | 0 | 0 | Unacceptable |
| SPL29 | 2 | 2 | 1 | Acceptable |
| SPL30 | 3 | 1 | 2 | Acceptable |
| SPL31 | 1 | 0 | 0 | Unacceptable |

In the determination result through the simultaneous determination of the upright observation image and the inverted observation image in Example 4 shown in Table 4, the quality is determined to be "unacceptable" in five samples, which is a stricter determination result than the determination result of the inverted observation image in Example 1 and the determination result of the combination of the upright observation image and the inverted observation image in Example 3 in which the quality is determined to be "unacceptable" in four samples. In the simultaneous determination of the upright observation image and the inverted observation image, the number of normal retinal tissue regions in sample SPL21 is five, which is larger than three in the upright observation image, but this is because a plurality of smaller regions in the region determined as the normal retinal tissue region in the upright observation image are determined as the normal retinal tissue region in the inverted observation image or the simultaneous determination of the upright observation image and the inverted observation image, and a more precise result is obtained in that the non-retinal tissue included in the region determined as the normal retinal tissue region in the upright observation image is excluded. Although there is a possibility that the angle at which the evaluation score is 2 is different between the upright observation image and the inverted observation image, it was confirmed that a stricter determination result can be obtained without any problem by using these evaluation scores for the determination at the same time. In the cell aggregate, the correlation coefficient of the width of the candidate region in the case of being determined as the neural retina and the significance test result p value are calculated as reference values for quality measurement between the upright observation image and the inverted observation image, and the difference between the upright observation image and the inverted observation image is evaluated, whereby it is confirmed that there is a correlation therebetween. Thus, an appropriate determination result should be obtained by simultaneously using the evaluation scores of the upright observation image and the inverted observation image for the same angle. Then, it was confirmed that, by determining that the region portions of the angles having different evaluation scores do not satisfy the criteria, it is possible to determine whether the cell aggregate has a normal retinal tissue region with higher accuracy than any of Examples 1 to 3.

### <Production of Neural Retinal Sheet for Transplantation>

When the cell aggregate determined to have a normal retinal tissue region in any of Examples 1 to 4 is composed of a plurality of lobes, a neural retinal sheet for transplantation can be produced by cutting out the lobes including the normal retinal tissue region. In particular, the cell aggregate determined to have a normal retinal tissue region in Example 4 has been subjected to the most precise analysis, and thus is suitable for the production of a neural retinal sheet for transplantation. When a lobe including a normal retinal tissue region is cut out, an appropriate lobe can be selected by referring to data of the angle at which a region portion for which a determination result that satisfies the criterion is obtained is present, the data being stored in the data storage area in the memory 120. When the cell aggregate is composed of a single lobe, the lobe can be used as a neural retinal sheet for transplantation.

### <Parameter Value as Reference>

In the above example, the fact that an ONbL having a width of 0.03 mm or more is continuously present at 45 degrees or more as viewed from the center of the cell aggregate in the inverted observation image of the cell aggregate is the condition of a normal retinal tissue region. In this case, the length of the cell aggregate having a diameter of about 1 mm along the outer periphery of the ONbL is about 0.4 mm or more. However, the quality standard for visual confirmation may be slightly changed, and the parameter values may be changed accordingly. Whereby, the condition of the parameter value can be set within a certain range. For example, regarding the criterion for the width, when the quality standard is more relaxed, the widths of the regions that need to be present continuously at 45 degrees or more may be set to 0.02 mm, 0.025 mm, and the like. In addition, when the quality standard is made stricter, the widths of the regions that need to be present continuously at 45 degrees or more may be set to 0.06 mm, 0.07 mm, and the like. Regarding the reference value of the angle, when the quality standard is more relaxed, the central angle of the range in which the ONbL having a width equal to or larger than the predetermined reference value should be present can be set to 30 degrees, 35 degrees, 40 degrees, and the like. When the quality standard is made stricter, the central angle of the range in which the ONbL having a width of the predetermined reference value or more should be present may be set to 50 degrees, 60 degrees, 75 degrees, and the like. In addition, when the diameter of the cell aggregate is increased, the length along the outer periphery of the ONbL is also increased in proportion thereto, provided that the continuous angles at which the ONbL portions are present as viewed from the center are the same. Thus, when the diameter of the cell aggregate is increased, the criterion of the continuous angle at which the ONbL portion viewed from the center is present can be reduced. On the other hand, by fixing the reference of the continuous angle at which the ONbL is present as viewed from the center to 45 degrees and setting the diameter of the cell aggregate to be determined to about 1 mm or more, it is also possible to determine the presence of an ONbL whose length along the outer periphery is about 0.4 mm or more regardless of the size of the cell aggregate.

In addition, the center point serving as a reference of the central angle can also be appropriately changed. That is, instead of the angle from the center point of the smallest ellipse in contact with the contour of the image of the portion of the cell aggregate, for example, an ellipse approximating the center point for each lobe can be set as, for example, an ellipse including the contour, and the center point can be set as the angle from the center point of the ellipse. In this case, it is preferable to correct the parameter value of the condition of the central angle based on the size (for example, the length of the major axis) of the ellipse approximating each lobe. That is, for example, when the size of the ellipse approximating each lobe becomes small, it is preferable to increase the condition of the central angle in inverse proportion thereto.

As described above, it is a basic technical idea of the present invention to execute the determination based on some criteria regarding the appropriate width and length of the ONbL in a manner that the region where the ONbL and the INbL are adjacent in the cell aggregate can be determined as the normal retinal tissue region.

### <Other Evaluation Parameters>

In the evaluation of the retinal tissue, evaluation parameters from the following viewpoints can be further used. That is, the continuity of the epithelial structure, the normality of the shape of the cells in the epithelial structure, the normality of the color tone of the epithelial structure, the normality of the texture of the cells inside the epithelial structure, the appropriateness of the width of the epithelial structure, and the smoothness of the outer periphery of the epithelial structure can be used as evaluation parameters for the normal retinal tissue region. The evaluation parameters of the morphology and color for the normal retinal tissue region can be obtained by calculating the similarity between the image of the evaluation target region of the cell aggregate to be evaluated and the image of the region having a typical normal morphology of the cell aggregate by a known method. In addition, as the evaluation parameter regarding the off-target cells, the color tone of the epithelial structure, the texture inside the epithelial structure, the thickness of the epithelial structure, the smoothness of the outer periphery of the epithelial structure, the presence or absence of the pigmented cell, the presence or absence of the adherent matter, and the like can be used. The evaluation parameter regarding the off-target cells can be obtained by calculating the similarity between the image of the evaluation target region of the cell aggregate to be evaluated and the image of a typical predetermined region of the off-target cells in the cell aggregate by a known method.

As a specific example of the evaluation of the evaluation parameter, the following criteria can be used. That is, the evaluation parameters regarding the retinal tissue and the off-target cells each have criteria for evaluating the score, and the criteria can be as follows.

Regarding the continuity of the epithelial structure, a higher evaluation score can be given as the degree of continuity of the epithelial structure is higher. The normality of the shape of the cells in the epithelial structure may be in a manner that fewer cells of abnormal shape are given a higher score. The normality of the color tone of the epithelial structure can be given a higher evaluation score as the color tone is closer to a standard state in which the color tone changes stepwise from bright white, light brown, and black in order from the outside to the inside in the normal direction of the epithelial structure. Regarding the normality of the texture of the cells inside the epithelial structure, a higher evaluation score can be given as the cells having a similar size and being rounded inside the epithelial structure are uniformly arranged and the grainy texture is higher. The appropriateness of the width of the epithelial structure can be determined in a manner that the width of the bright ring-shaped region on the outer side as viewed from the normal direction of the epithelial structure is 30 µm or more, and the larger the width, the higher the evaluation score is given. The smoothness of the outer periphery of the epithelial structure can be evaluated in a manner that a higher evaluation score is given as the number of inflection points at which the angle changes rapidly in the contour of the cell aggregate is smaller and the continuity of the change in the angle is higher. The presence or absence of pigmented cells can be scored in a manner that fewer colored cells are given a higher evaluation score. The presence or absence of an adherent matter can be given a higher evaluation score as the number of dead cells or non-cellular components derived from equipment or the like is smaller.

### <Method for Producing Retinal Tissue That Can be Transplanted Into Animal>

Although a retinal tissue that can be transplanted into an animal can be produced using a cell aggregate determined to have a normal retinal tissue region by any of the directions of Examples 1 to 4, a method for producing a retinal tissue that can be transplanted into an animal using a cell aggregate determined to have a normal retinal tissue region in Example 4 having particularly high determination accuracy will be described below. First, a cell aggregate determined to have a normal retinal tissue region in Example 4 is prepared. From this, a region portion of the candidate region satisfying the normal region determination criterion is cut out as a normal retinal tissue region, whereby a retinal tissue that can be transplanted into an animal can be produced.

Further, by confirming the expression of the neural retina-related cell-associated gene and the non-neural retina-related cell-associated gene, it is possible to determine a normal retinal tissue region that can be transplanted into an animal with higher accuracy. For this purpose, the following procedures are preferably further performed on a normal retinal tissue region that is a region determined to simultaneously satisfy each of the normal region determination criteria by any of the methods of Examples 1 to 4:
detecting expression of a neural retina-related cell-associated gene and a non-neural retina-related cell-associated gene in a normal retinal tissue region;
determining that the normal retinal tissue region can be transplanted into an animal when expression of the neural retina-related cell-associated gene is observed and expression of the non-neural retina-related cell-associated gene is not observed. Here, the non-neural retina-related cell-associated gene preferably includes one or more genes selected from the group consisting of a spinal cord tissue marker gene and an eyeball-associated tissue marker gene. The presence of neural retina-related cells can be confirmed by the presence or absence of expression of a neural retina-related cell-associated gene (also referred to as a "neural retina-related cell marker" or a "neural retina marker"), and therefore, by confirming the presence or absence of expression of the gene, a more precise determination of the normal retinal tissue region can be made. The presence or absence of expression of a neural retina-related cell marker, or the proportion of neural retina-related cell marker-positive cells in a cell population or tissue can be confirmed by using a known method such as a method using an antibody, a method using a nucleic acid primer, or a method using a sequencing reaction.

Further, by confirming the expression of the neural retina-related cell-associated gene and the non-neural retina-related cell-associated gene, it is possible to prepare a retinal tissue that includes a more favorable normal retinal tissue region and can be transplanted into an animal. For this purpose, first, a cell aggregate determined to have a normal retinal tissue region in any of Examples 1 to 4 is prepared. The following procedures [1] to [4] are executed on the region portion of the candidate region satisfying the normal region determination criterion in the cell aggregate.
[1] A part or all of the region portion of the candidate region satisfying the normal region determination criterion is extracted as a sample for quality evaluation.
[2] Expression of a neural retina-related cell-associated gene and a non-neural retina-related cell-associated gene in the sample for quality evaluation is detected.
[3] When expression of the neural retina-related cell-associated gene is observed and expression of the non-neural retina-related cell-associated gene is not observed, the following (1), (2) or (3) is determined to be usable as a neural retina for transplantation:
   (1) neural retina (neural retina for transplantation) in the same cell aggregate as the cell aggregate containing the sample for quality evaluation in a case where the sample is a part of the region portion;
   (2) neural retina (neural retina for transplantation) in a cell aggregate of the same lot as that of the cell aggregate containing the sample for quality evaluation in a case where the sample is a part of the region portion;
   (3) neural retina (neural retina for transplantation) in a cell aggregate of the same lot as that of the cell aggregate of the sample for quality evaluation in a case where the sample is all of the region portion.
   Here, the non-neural retina-related cell-associated gene preferably includes one or more genes selected from the group consisting of a spinal cord tissue marker gene and an eyeball-associated tissue marker gene.
[4] The normal retinal tissue region determined to be usable as the neural retina for transplantation is cut out. This makes it possible to produce a neural retina for transplantation from a cell aggregate in which a normal retinal tissue region has been determined more precisely.

As described above, the neural retina for transplantation produced by cutting out the cell aggregate using tweezers, scissors, a knife, or the like can be used for treating a disease based on retinal cell disorder or a damaged state of retinal cells as a pharmaceutical composition containing a cell population for transplantation in the form of a cell sheet as an active ingredient or a therapeutic agent containing a cell population for transplantation in the form of a cell sheet. When a cell aggregate of a tissue other than the retina is used for the cell sheet, the cell sheet can be used as a therapeutic agent for the tissue.

The neural retina-related cell-associated gene (target cell-associated gene) means a gene expressed by a neural retina cell. As the neural retina-related cell-associated gene, genes that are expressed at a higher level in photoreceptor cells (rod photoreceptor cells, cone photoreceptor cells), horizontal cells, amacrine cells, interneurons, retinal ganglion cells (ganglionic cells), bipolar cells (rod bipolar cells, cone bipolar cells), Müller glial cells, or precursor cells of these cells, neural retinal precursor cells, and the like, as compared with off-target cells, are preferable. Examples of the neural retina-related cell-associated genes include the neural retina cell markers described above, and RAX, Chx10, SIX3, SIX6, RCVRN, CRX, NRL, and NES are preferable.

In one aspect, the cerebrospinal tissue marker gene may be one or more genes selected from the group consisting of a telencephalon marker gene, a diencephalon/mesencephalon marker gene, and a spinal cord marker gene. The diencephalon/mesencephalon marker gene may be one or more genes selected from the group consisting of a diencephalon marker gene, a mesencephalon marker gene, and a hypothalamus marker gene relating to hypothalamus which is a part of the diencephalon. In one aspect, the eyeball-associated tissue marker gene may be one or more genes selected from the group consisting of an optic stalk marker gene, a ciliary body marker gene, a lens marker gene, and a retinal pigment epithelium marker gene.

The telencephalon marker gene means a gene expressed in the telencephalon. The telencephalon marker genes may include one or more genes selected from the group consisting of FoxG1 (alias Bf1), Emx2, Dlx2, DIx1, and Dlx5. Other examples of telencephalon marker genes include Emx1, LHX2, LHX6, LHX7, and Gsh2. The diencephalon/mesencephalon marker gene means a gene expressed in the diencephalon and/or the mesencephalon. The diencephalon/mesencephalon marker genes may include one or more genes selected from the group consisting of OTX1, OTX2, and DMBX1. The hypothalamus marker gene means a gene expressed in the hypothalamus. The hypothalamus marker gene may include one or more genes selected from the group consisting of Rx, Nkx2.1, Dmbx1, OTP, gad1, FGFR2, and EFNA5. The spinal cord marker gene means a gene expressed in the spinal cord. The spinal cord marker genes may include one or more genes selected from the group consisting of HoxB2, HoxA5, HOXC5, HOXD1, HOXD3, and HOXD4.

The optic stalk marker gene means a gene expressed in the optic stalk. The optic stalk marker gene may include one or more genes selected from the group consisting of GREM1, GPR17, ACVR1C, CDH6, Pax2, Pax8, GAD2, and SEMA5A. The lens marker gene means a gene expressed in the lens. The lens marker gene may include one or more genes selected from the group consisting of CRYAA and CRYBAl. The ciliary body marker gene means a gene expressed in the ciliary body, the ciliary marginal zone, and/or the ciliary body. The ciliary body marker genes may include one or more genes selected from the group consisting of ZC1, MAL, HNF1beta, FoxQ1, CLDN2, CLDN1, GPR177, AQP1, and AQP4. The retinal pigment epithelial marker gene means a gene expressed in retinal pigment epithelium cells. Examples of the retinal pigment epithelium marker gene include the retinal pigment epithelium markers described above, and the retinal pigment epithelium marker genes may include one or more genes selected from the group consisting of MITF, TTR, and BEST1.

### <Determination Using Artificial Intelligence Using Deep Learning>

In Examples 1 to 4, automated determination was performed based on determination criteria based on features of specific morphology and color such as the width and length of the bright region of the cell aggregate to be determined. This method is very effective when the determination criterion can be clearly defined as being based on a predetermined viewpoint. By constructing a machine learning model using deep learning in which an inverted observation image or an upright observation image of a cell aggregate is used as an input and normal/abnormal for each cell aggregate is used as an output, it is also possible to perform automated determination as to whether the cell aggregate has a normal retinal tissue region with artificial intelligence using deep learning. In the case of automated determination using deep learning, it is possible to perform determination in consideration of properties that cannot be formulated as clearly defined determination criteria based on a specific morphology and/or color as a result of observation using the eyes. Hereinafter, automated determination with artificial intelligence using deep learning will be described.

First, a method for generating a machine learning model will be described. A method for generating a machine learning model to be applied to a discrimination apparatus for discriminating a cell aggregate having a normal retinal tissue region that can be transplanted into an animal in a cell aggregate that may contain a retinal tissue derived from such pluripotent stem cells includes:
a training data acquisition stage of acquiring, for each of a plurality of cell aggregates, a plurality of pieces of training data including image data to which a teaching signal (label) indicating whether an image region of the cell aggregates included in image data acquired by photographing the cell aggregates using an inverted microscope and an upright microscope has a normal retinal tissue region is attached; and
a model learning step of learning, using the training data, a machine learning model in which image data of the cell aggregate to be determined is used as an input and information indicating whether the cell aggregate to be determined has a normal retinal tissue region is used as an output.

It is preferable to execute appropriate preprocessing such as contrast enhancement, contour coordination, deletion of unnecessary image regions, and unification of the positions and sizes of the image regions of the cell aggregate on the image data included in the training data.

Here, the inverted observation image and the upright observation image of the cell aggregate can be simultaneously input to the machine learning model. That is, the image data of the inverted observation image and the image data of the upright observation image to which the teaching signal indicating whether the image region of the cell aggregate has a normal retinal tissue region is added can be used as the training data. Accordingly, it is possible to generate a machine learning model that performs determination in consideration of both the inverted observation image and the upright observation image.

A method for generating a machine learning model to be applied to a discrimination apparatus for discriminating a cell aggregate having a normal retinal tissue region that can be transplanted to an animal in a cell aggregate that may contain a retinal tissue derived from pluripotent stem cells, the method including:
a training data acquisition stage of acquiring, for each of a plurality of cell aggregates, a plurality of pieces of training data including a set of image data of an inverted observation image and an upright observation image of the same cell aggregate to which a teaching signal indicating whether an image region of the cell aggregate having a contour of a region of a predetermined length or more included in the image data of the inverted observation image and the upright observation image, wherein positions in respective images of the inverted observation image and the upright observation image are position-associated so that an identical position of the cell aggregate as viewed from the vertical direction corresponds, is a normal retinal tissue region, acquired by photographing the cell aggregate using the inverted microscope and the upright microscope, respectively; and
a model learning stage of learning a machine learning model in which image data of the inverted observation image and the upright observation image of the cell aggregate to be determined are input using a plurality of training data and information indicating whether the cell aggregate to be determined has a normal retinal tissue region is output.

In addition, it is possible to configure a discrimination apparatus in which an inverted observation image and an upright observation image are individually determined and then the determination results are combined.

A discrimination apparatus for a cell aggregate having a normal retinal tissue region that can be transplanted includes:
a machine learning model generated by the machine learning model generation method described above;
an image data input unit that inputs image data of an inverted observation image and an upright observation image of the same cell aggregate to be determined to a machine learning model;
an output unit that receives an output of information indicating whether the cell aggregate to be determined in each of the inverted observation image and the upright observation image has a normal retinal tissue region from the machine learning model; and
a determination unit that determines that the cell aggregate to be determined has a normal retinal tissue region when both the inverted observation image and the upright observation image of the cell aggregate to be determined indicate that the cell aggregate to be determined has a normal retinal tissue region.

Further, as a method for discriminating a normal retinal tissue region that can be transplanted using this discrimination apparatus,
an acquisition stage of acquiring image data of an inverted observation image and an upright observation image of the same cell aggregate to be determined;
an image data input stage of inputting the acquired image data of the inverted observation image and the upright observation image of the same cell aggregate to be determined to the discrimination apparatus; and
a determination result reception stage of receiving a determination result that the cell aggregate to be determined has a normal retinal tissue region from the discrimination apparatus.

Further, a discrimination apparatus for simultaneously discriminating an inverted observation image and an upright observation image can be configured.

A discrimination apparatus for a cell aggregate having a normal retinal tissue region that can be transplanted includes:
a machine learning model generated by the machine learning model generation method described above;
an image data input unit that inputs a set of image data of an inverted observation image and an upright observation image of the same cell aggregate to be determined to a machine learning model; and
an output unit that receives an output of information indicating whether the cell aggregate to be determined has a normal retinal tissue region from the machine learning model.

Further, as a method for discriminating a normal retinal tissue region that can be transplanted using this discrimination apparatus,
an acquisition stage of acquiring a set of image data of an inverted observation image and an upright observation image of the same cell aggregate to be determined;
an image data input stage of inputting the acquired set of image data of the inverted observation image and the upright observation image of the same cell aggregate to be determined to the discrimination apparatus; and
a determination result reception stage of receiving a determination result that the cell aggregate to be determined has a normal retinal tissue region from the discrimination apparatus.

Further, as a method for producing a retinal tissue for transplantation, a method including a stage of cutting out a normal retinal tissue region from a cell aggregate including a normal retinal tissue region discriminated based on any of the above-described methods for discriminating a transplantable normal retinal tissue region can be performed.

Although the retinal tissue has been described as an example of the cell aggregate, the above-described method can be used to observe human or animal tissues other than the retinal tissue. That is, the present invention enables more effective observation synergistically by performing observation with both an inverted observation image and an upright observation image of a cell aggregate containing a tissue other than a retinal tissue derived from a pluripotent stem cell, thereby compensating for the shortcomings of the observation methods of each other. In addition, the present invention can also be configured in a manner that, in an image region of a cell aggregate other than retinal tissue, a region determined to satisfy a candidate region determination criterion regarding color or the like corresponding to each of an inverted observation image and an upright observation image is identified as a candidate region included in a region to be a candidate of normal tissue region based on the candidate region determination criterion regarding color or the like corresponding to each of an inverted observation image and an upright observation image, and in each of the candidate regions of the inverted observation image and the upright observation image, a cell aggregate having a candidate region determined to satisfy a normal region determination criterion regarding brightness and/or morphology corresponding to each of the inverted observation image and the upright observation image is determined to have a normal tissue region based on the normal region determination criterion regarding brightness and/or morphology or the like corresponding to each of the inverted observation image and the upright observation image. With such a configuration, it is also possible to automatically extract a cell aggregate including a region other than a retinal tissue including a three-dimensionally favorable normal region through information processing using image data observed from two directions of upper and lower directions using an upright microscope and an inverted microscope.

### Industrial Applicability

The present invention can be widely used for producing a cell aggregate containing various tissues, in particular, neural retinal tissues in the field of transplantation therapy of humans and animals using regenerative medicine.

### Reference Signs List

10 Cell aggregate
21 Upright microscope
22 Inverted microscope
23 Vessel
24 Culture solution
31 Upright observation
32 Inverted observation
100 Normal cell aggregate determination system
110 Normal cell aggregate determination server
111 Controller
112 Image data acquisition unit
113 Candidate region identification unit
114 Normal region determination unit
114A Region portion evaluation unit
114B Continuous length-based normal region determination unit
120 Memory
120A Normal cell aggregate determination program
120B Normal cell aggregate determination data
130 Communication interface
200 Upright microscope
210 Inverted microscope
215 Digital camera
220 Operation terminal
CL1 Curve
CR1 Candidate region
CTR Center point
ET1 to ET3 Epithelial tissue
LAN Local area network
PLT1 to PLT6 Vessel
R1 to R4 Straight line
RE Reference ellipse
SPL1 to SPL31 Sample
W Width

## Claims

1. A method for determining a normal retinal tissue region that can be transplanted into an animal in a cell aggregate that may contain retinal tissue derived from pluripotent stem cells, the method comprising:
an image data acquisition stage of acquiring image data of an inverted observation image and an upright observation image of the cell aggregate by photographing the cell aggregate using an inverted microscope and an upright microscope;
a candidate region identification stage of identifying, in the image region of the cell aggregate of each of the inverted observation image and the upright observation image, a region determined to satisfy a candidate region determination criterion regarding color corresponding to each of the inverted observation image and the upright observation image as a candidate region included in a region to be a candidate of the normal retinal tissue region based on the candidate region determination criterion regarding color corresponding to each of the inverted observation image and the upright observation image; and
a normal region determination stage of determining, in the candidate region of each of the inverted observation image and the upright observation image, a candidate region determined to satisfy a normal region determination criterion regarding brightness and/or morphology corresponding to each of the inverted observation image and the upright observation image as the normal retinal tissue region based on the normal region determination criterion regarding brightness and/or morphology corresponding to each of the inverted observation image and the upright observation image.

2. The method according to claim 1, wherein
the inverted observation image and the upright observation image are position-associated in a manner that a position in the inverted observation image and a position in the upright observation image correspond to an identical position of the cell aggregate viewed from a vertical direction, and
in the normal region determination stage, in the candidate region of each of the inverted observation image and the upright observation image, the candidate region determined to simultaneously satisfy a normal region determination criterion regarding brightness and morphology corresponding to each of the inverted observation image and the upright observation image is determined as the normal retinal tissue region based on the normal region determination criterion corresponding to each of the inverted observation image and the upright observation image.

3. The method according to claim 2, wherein
the normal region determination stage includes:
a region portion evaluation stage of dividing the candidate region into a plurality of ranges in which region portions are present, along a curve representing a direction in which the candidate region extends, and obtaining an evaluation score of each of the region portions, for the candidate region identified in the image region of the cell aggregate with which the position of each of the inverted observation image and the upright observation image is associated, based on an evaluation criterion regarding color and/or morphology corresponding to each of the inverted observation image and the upright observation image; and
a continuous length-based normal region determination stage of determining the region portion as the normal retinal tissue region when it is determined that the region portion of the candidate region in which both the evaluation scores of the inverted observation image and the upright observation image have a value equal to or more than a corresponding normal region determination reference value is continuously present along a curve representing an extending direction of the candidate region by a length equal to or more than a predetermined normal region determination reference length.

4. The method according to claim 3, wherein the candidate region determination criterion in the candidate region identification stage further includes a requirement that brightness of the region to be identified is higher than a maximum value or an average value of brightness of a central region of an image region of the cell aggregate by a predetermined degree or more.

5. The method according to claim 4, wherein the candidate region determination criterion in the candidate region identification stage further includes a requirement that, in the region to be identified, an outermost contour viewed from a central portion of an image region of the cell aggregate in a region portion included in the region to be identified substantially matches a contour of the image region of the cell aggregate.

6. The method according to claim 5, wherein the candidate region determination criterion in the candidate region identification stage further includes a requirement that a change in an angle in a tangential direction of the outermost contour of the region to be identified is continuous.

7. The method according to claim 6, wherein the candidate region determination criterion in the candidate region identification stage further includes a requirement that a width between an outermost portion and an innermost portion of the region to be identified as viewed from a central portion of an image region of the cell aggregate is equal to or more than a predetermined reference width.

8. The method according to claim 7, wherein
the candidate region identification stage further includes an off-target cell identification stage of identifying whether an off-target cell is present based on a predetermined off-target cell determination criterion based on color and morphology in the image region of the cell aggregate of each of the inverted observation image and the upright observation image, and
the candidate region determination criterion further includes a requirement that the off-target cell is not present in the region to be identified.

9. The method according to claim 3, wherein
the region portion evaluation stage divides the candidate region identified in the image region of the cell aggregate to which the position of each of the inverted observation image and the upright observation image is associated into a plurality of ranges in which region portions are present at every predetermined angle as viewed from a central portion of the image region of the cell aggregate, and obtains an evaluation score of each of the region portions based on an evaluation criterion regarding morphology corresponding to each of the inverted observation image and the upright observation image, and
the continuous length-based normal region determination stage determines that the cell aggregate in which the region portion in which both the evaluation scores of the inverted observation image and the upright observation image are determined to have values equal to or more than the corresponding normal region determination reference value is continuously present at a predetermined normal region determination reference angle or more has the normal retinal tissue region.

10. The method according to claim 7, wherein the predetermined reference width in the candidate region identification stage is 0.03 mm for the inverted observation image and 0.15 mm for the upright observation image.

11. The method according to claim 3, wherein the predetermined normal region determination reference length in the continuous length-based normal region determination stage is 0.4 mm.

12. The method according to claim 9, wherein the predetermined normal region determination reference angle in the continuous length-based normal region determination stage is 45 degrees.

13. The method according to claim 3, wherein the image data of the inverted observation image and the upright observation image of the cell aggregate that may include the retinal tissue is obtained by photographing the cell aggregate suspended in a liquid.

14. The method according to claim 13, wherein the image data of the inverted observation image and the upright observation image of the cell aggregate that may include the retinal tissue is obtained by photographing the cell aggregate in a plastic vessel.

15. The method according to claim 14, wherein the plastic vessel is a vessel having one or more isolated culture regions.

16. The method according to claim 15, wherein the plastic vessel is a spherical or conical vessel having a downward convex shape or a recess.

17. A method for producing a retinal tissue that can be transplanted into an animal, the method comprising cutting out the normal retinal tissue region determined by the method described in any one of claims 3 to 16.

18. A method for determining a normal retinal tissue region that can be transplanted into an animal, the method comprising, with respect to the normal retinal tissue region determined by the method described in any one of claims 3 to 16, further:
detecting expression of a neural retina-related cell-associated gene and a non-neural retina-related cell-associated gene within the normal retinal tissue region; and
determining that the normal retinal tissue region can be transplanted into an animal when expression of the neural retina-related cell-associated gene is observed and expression of the non-neural retina-related cell-associated gene is not observed.

19. A method for producing a retinal tissue that can be transplanted into an animal by executing, with respect to the normal retinal tissue region determined by the method described in any one of claims 3 to 16, further:
extracting a part or all of the normal retinal tissue region as a sample for quality evaluation;
detecting expression of a neural retina-related cell-associated gene and a non-neural retina-related cell-associated gene in the sample for quality evaluation;
when expression of the neural retina-related cell-associated gene is observed and expression of the non-neural retina-related cell-associated gene is not observed, determining that the following (1), (2), or (3) is usable as a neural retina for transplantation:
(1) the neural retina (neural retina for transplantation) in the same cell aggregate as the cell aggregate containing the sample for quality evaluation in a case where the sample is a part of the normal retinal tissue region,
(2) the neural retina (neural retina for transplantation) in a cell aggregate of the same lot as that of the cell aggregate containing the sample for quality evaluation in a case where the sample is a part of the normal retinal tissue region,
(3) the neural retina (neural retina for transplantation) in a cell aggregate of the same lot as that of the cell aggregate of the sample for quality evaluation in a case where the sample is all of the normal retinal tissue region;
and
cutting out the normal retinal tissue region determined to be usable as the neural retina for transplantation.
